(19) Europäisches Patentamt
European Patent Office
Office européen des brevets

(11) EP 1 417 967 A1

(12) **EUROPEAN PATENT APPLICATION**
published in accordance with Art. 158(3) EPC

(43) Date of publication:
**12.05.2004 Bulletin 2004/20**

(21) Application number: **02760622.7**

(22) Date of filing: **13.08.2002**

(51) Int Cl.[7]: **A61K 31/706**, A61K 45/00, A61P 31/12, C07H 19/04, C12N 9/99

(86) International application number:
**PCT/JP2002/008250**

(87) International publication number:
**WO 2003/015798 (27.02.2003 Gazette 2003/09)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **14.08.2001 JP 2001245896**

(71) Applicant: **TOYAMA CHEMICAL CO., LTD.**
**Tokyo 160-0023 (JP)**

(72) Inventors:
- **FURUTA, Yousuke**
  **Toyama-shi, Toyama 939-8045 (JP)**
- **EGAWA, Hiroyuki**
  **Toyama-shi, Toyama 930-2205 (JP)**
- **TAKAHASHI, Kazumi**
  **Nakaniikawa-Gun, Toyama 930-0237 (JP)**
- **TSUTSUI, Yasuhiro**
  **Toyama-shi, Toyama 930-0817 (JP)**
- **UEHARA, Sayuri**
  **Toyama-shi, Toyama 939-8081 (JP)**
- **MURAKAMI, Makoto**
  **Toyama-shi, Toyama 930-0817 (JP)**

(74) Representative: **Hartz, Nikolai F., Dr. et al**
**Wächtershäuser & Hartz**
**Patentanwälte**
**Weinstrasse 8**
**80333 München (DE)**

(54) **NOVEL VIRUS PROLIFERATION INHIBITION/VIRUCIDAL METHOD AND NOVEL PYRADINE NUCLEOTIDE/PYRADINE NUCLEOSIDE ANALOGUE**

(57)



Printed by Jouve, 75001 PARIS (FR)

(Cont. next page)

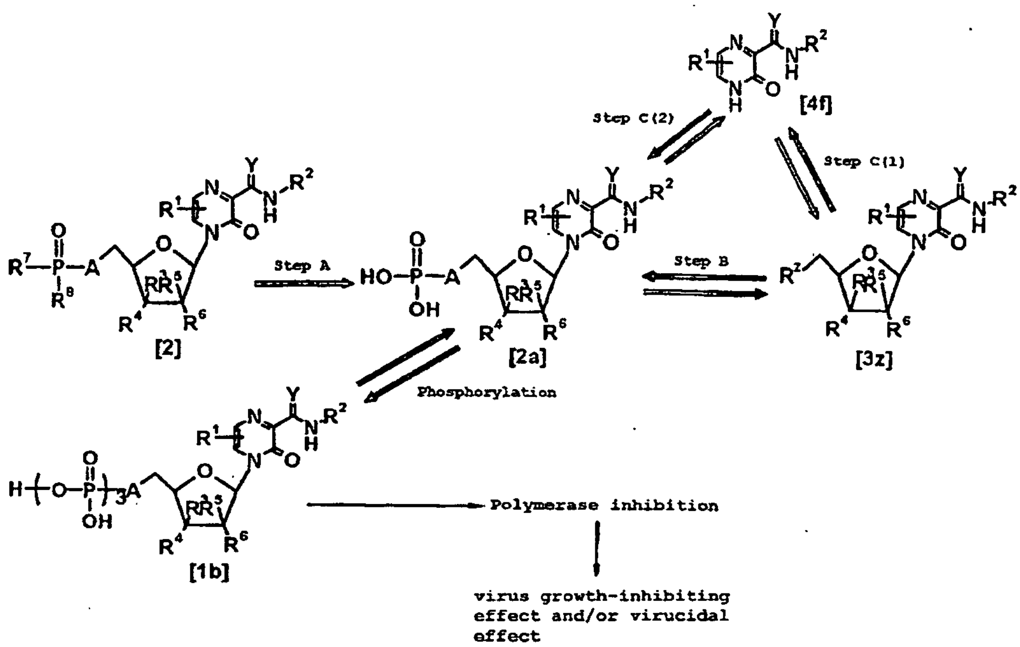


wherein each of $R^1$, $R^2$, $R^3$, $R^4$, $R^5$, $R^6$, $R^7$, $R^8$, $R^9$ and A has the same meaning as given in the specification.

The present invention relates to a method for exhibiting a virus growth-inhibiting effect and/or a virucidal effect, in which pyrazine nucleotide analog [2] and pyrazine nucleoside analog [3z] are subjected to biotransformation, decomposed and then phosphorylated, so that they become a pyrazine nucleotide analog [1b] exhibiting the aforementioned effect. This method is useful as a method for treating virus infections. Moreover, the pyrazine carboxamide analog or a salt thereof of the present invention is useful as an agent for preventing or treating virus infections.

## Description

TECHNICAL FIELD

The present invention relates to a virus growth inhibition and/or virucidal method characterized in that it uses a pyrazine nucleotide or pyrazine nucleoside analog generated by kinase or a salt thereof, a novel pyrazine nucleotide or pyrazine nucleoside analog or a salt thereof, and a method for treating virus infection using them.

BACKGROUND ART

Infectious virus diseases (e.g., influenza infection, herpesvirus infection, acquired immunodeficiency syndrome (AIDS), viral hepatitis, viral hemorrhagic fever, etc.) have been recognized to be medically important problems, and a wide range of treatments such as prevention of the diseases with vaccines or therapeutic methods of using drugs have been studied. A large number of nucleic acids having purine bases and pyrimidine bases and derivatives thereof have been developed to date as agents used for drug treatment of virus infections. These agents have an action mechanism such that they are triphosphorylated in cells and inhibit virus polymerase. Examples of such agents may include azidothymidine and acyclovir [Proceedings of the National Academy of Science of the United States of America (Proc. Natl. Acad. Sci. USA), Vol. 83, pp. 8333 to 8337 (1986); the same publication, Vol. 74, pp. 5716 to 5720 (1977)].

Moreover, it has been reported that the active form of a compound whose antiviral effect is exhibited when its portion corresponding to bases of nucleic acid is converted into an unnatural chemical structure is a monophosphorylated form obtained by converting the compound in a cell, and that it inhibits inosine monophosphate dehydrogenase (IMPDH) in the cell, thereby exhibiting effects. Examples of such a compound may include ribavirin and EICAR [Proceedings of the National Academy of Science of the United States of America (Proc. Natl. Acad. Sci. USA), Vol. 70, pp. 1174 to 1178 (1973); The Journal of Biological Chemistry (J. Biol. Chem.), Vol. 268, pp. 24591 to 24598 (1993)].

Moreover, as an example of nucleoside and nucleotide analogs having a pyrazine ring as a base, the following general formula has been known:

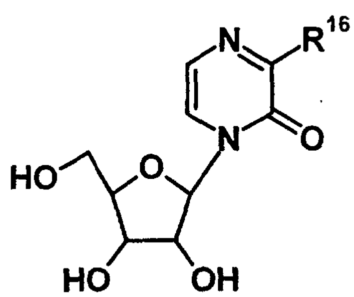

wherein $R^{16}$ represents a hydrogen atom, a methyl group or decyl group. However, this compound exhibits no antiviral activity (no anti-Visna virus activity) [Nucleosides & Nucleotides, Vol. 15, Nos. 11 and 12, pp. 1849 to 1861 (1996)].

On the other hand, nucleoside and nucleotide analogs having a pyrazine ring that is substituted with a carbamoyl group have not been known.

DISCLOSURE OF THE INVENTION

It is an object of the present invention to provide a highly safe antiviral agent with low toxicity, which has an unnatural chemical structure in a portion corresponding to bases of nucleic acid, and a novel virus growth inhibition and/or virucidal method using the antiviral agent.

The present inventors have found that a pyrazine nucleotide or pyrazine nucleoside analog represented by the following general formula [1] or a salt thereof:

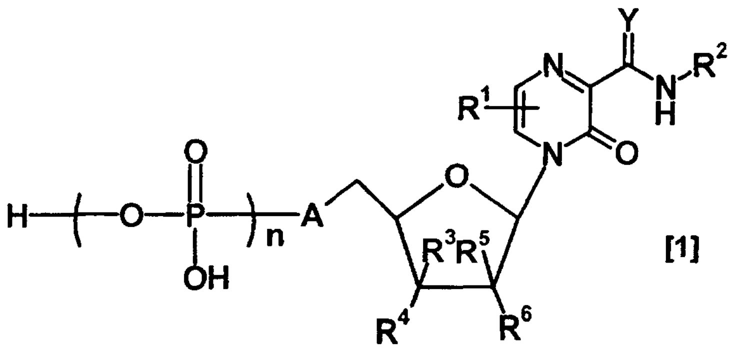

[1]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^4$, $R^5$ and $R^6$ identically or differently represents a hydrogen atom, or a hydroxyl group that may be substituted or protected; A represents an oxygen atom or a methylene group; Y represents an oxygen atom or an imino group; and n represents an integer of 0 to 3, especially, a triphosphorylated pyrazine nucleotide analog or a salt thereof exhibits a highly safe, excellent virus growth-inhibiting and/or virucidal effect with low toxicity, which inhibits virus polymerase, especially RNA polymerase directly or in the form of a substance converted therefrom in vivo.

Moreover, the present inventors have found a novel method of hydrolyzing or decomposing in vivo or in a cell a pyrazine nucleotide analog represented by the following general formula [2] or a salt thereof:

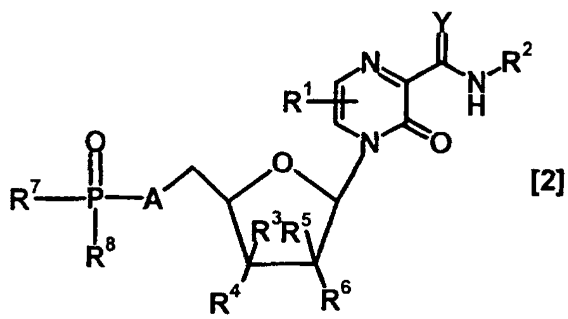

[2]

wherein each of $R^1$, $R^2$, $R^3$, $R^4$, $R^5$, $R^6$, A and Y has the same meaning as given above; and each of $R^7$ and $R^8$ in phosphoric acid or phosphonic acid independently represents a protected or unprotected, substituted or unsubstituted hydroxyl group to be decomposed under physiological conditions, and then inducing the product thus obtained into the pyrazine nucleotide or pyrazine nucleoside analog represented by general formula [1] or a salt thereof by the effect of kinase such as nucleotide kinase, so as to make it exhibit a virus growth-inhibiting effect and/or a virucidal effect.

Furthermore, the present inventors have found that a pyrazine nucleotide analog represented by the following general formula [1z] or a salt thereof:

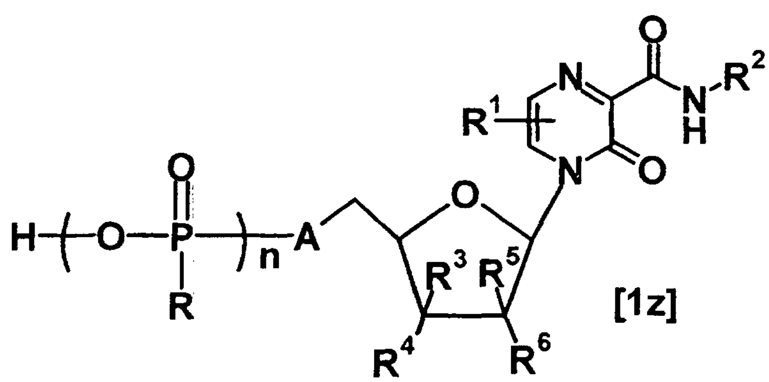

[1z]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^4$, $R^5$ and $R^6$ identically or differently represents a hydrogen atom, or a hydroxyl group that may be substituted or protected; R represents a hydroxyl group that may be protected or substituted with a group decomposed under physiological conditions; A represents an oxygen atom or a methylene group; and n represents an integer of 1 to 3, is a compound, which is converted under physiological conditions and inhibits virus RNA polymerase in the same manner as the general formula [1], thus exhibiting a virus growth-inhibiting effect and/or a virucidal effect.

Any of the aforementioned compounds is converted in vivo or in a cell into a pyrazine triphosphate nucleotide analog represented by the following general formula [1y]:

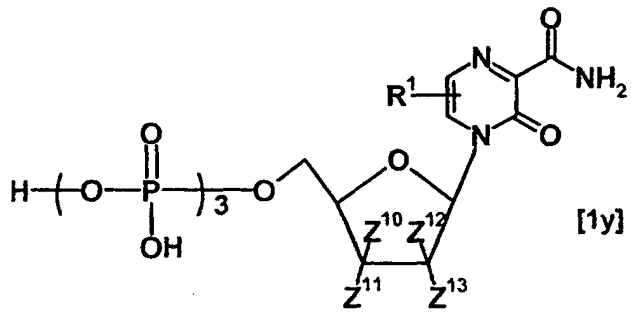

[1y]

wherein $R^1$ represents a hydrogen atom, or a substituent of a pyrazine ring; and each of $Z^{10}$, $Z^{11}$, $Z^{12}$ and $Z^{13}$ identically or differently represents a hydrogen atom or hydroxyl group, and it exhibits an RNA polymerase inhibitory effect. In addition, a compound represented by general formula [1x] indicated below is an RNA polymerase inhibitor precursor that is converted into the compound represented by general formula [1y] in vivo or in a cell:

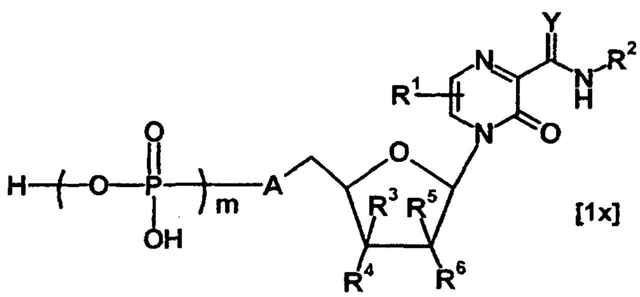

[1x]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^4$, $R^5$ and $R^6$ identically or differently represents a hydrogen atom, or a hydroxyl group that may be substituted or protected; A represents an oxygen atom or a methylene group; Y represents an oxygen atom or an imino group; and m represents an integer of 0 to 2.

The RNA polymerase inhibitor precursor of the present invention inhibits virus-derived RNA polymerase with selectivity much more higher than host-derived RNA polymerase. The present RNA polymerase inhibitor precursor can inhibit virus-derived RNA polymerase with selectivity preferably 200 times or more, more preferably 1,000 times or more, and further more preferably 2,000 times or more, higher than that for host-derived RNA polymerase. Moreover, the RNA polymerase inhibitor precursor of the present invention hardly inhibits inosine monophosphate dehydrogenase, and after it is converted in vivo into a triphosphorylated form, it inhibits virus polymerase. Accordingly, the present RNA polymerase inhibitor precursor is characterized in that it has an extremely strong virus polymerase inhibitory effect after it is converted in vivo, and it also has high selectivity, while cytotoxicity caused by inhibition of inosine monophosphate dehydrogenase is extremely reduced. Using this high selectivity, a highly safe agent can be obtained.

The RNA polymerase inhibitor precursor of the present invention has extremely high selectivity to RNA polymerase and inosine monophosphate dehydrogenase. In a pyrazine nucleoside or pyrazine mononucleotide analog structure represented by the following general formula [1w] :

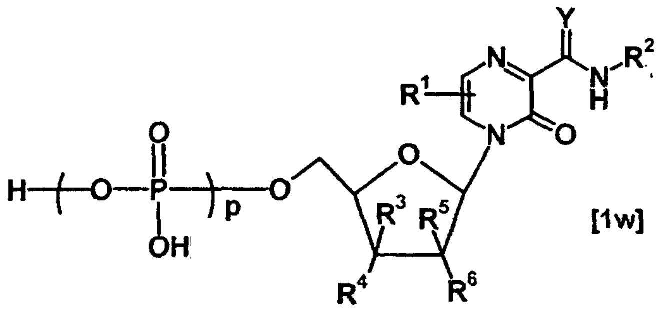

[1w]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^4$, $R^5$ and $R^6$ identically or differently represents a hydrogen atom, or a hydroxyl group that may be substituted or protected; Y represents an oxygen atom or an imino group; and p represents 0 or 1, the ratio of the inhibitory effect on virus-derived RNA polymerase of the precursor after it is converted in vivo that on the host cell-derived inosine monophosphate dehydrogenase of the precursor is preferably 900 : 1 or more, more preferably 5,000 : 1 or more , and further more preferably 10,000 : 1 or more.

The present inventors have confirmed that when each of $R^1$, $R^3$ and $R^5$ is a hydrogen atom and each of $R^{4Z}$, $R^{6Z}$ and $R^Z$ is a hydroxyl group, for example, in a pyrazine nucleotide derivative represented by the following general formula [3z]:

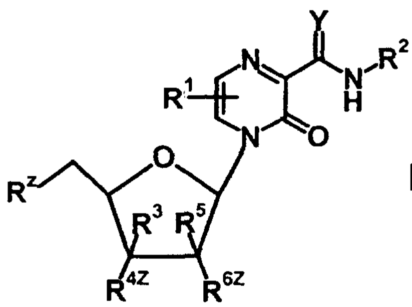

[3z]

wherein each of $R^1$, $R^2$, $R^3$, $R^5$ and Y has the same meaning as given above; $R^Z$ represents a protected or unprotected, substituted or unsubstituted hydroxyl group to be decomposed under physiological conditions; each of $R^{4Z}$ and $R^{6Z}$ identically or differently represents a hydrogen atom or a hydroxyl group that may be substituted or protected, or $R^{4Z}$ and $R^{6Z}$ together represent a group represented as -O-alkylene-O- that may be substituted, and when such a compound is administered to an animal, there is generated 4-[(2R, 3R, 4S, 5R)-3,4-dihydroxy-5-(hydroxymethyl)tetrahydro-2-furanyl]-3-oxo-3,4-dihydro-2-pyrazinecarboxamide (substitution nomenclature: 3,4-dihydro-3-oxo-4-β-D-ribofuranosyl-2-pyrazinecarboxamide) in the blood plasma of the animal.

What is more, the present inventors have confirmed that when the pyrazine nucleoside analog represented by general formula [3z] or a salt thereof such as 4-[(2R, 3R, 4S, 5R)-3,4-dihydroxy-5-(hydroxymethyl)tetrahydro-2-furanyl]-3-oxo-3,4-dihydro-2-pyrazinecarboxamide is administered to an animal, there is generated {(2R, 3S, 4R, 5R)-5-[3-(aminocarbonyl)-2-oxo-1(2H)-pyrazinyl]-3,4-dihydroxytetrahydro-2-furanyl} methyl triphosphate in the organ of the animal.

Thus, the present inventors have found that a method of administering the compound represented by general formula [3z] or a salt thereof to mammals, or administering the compound represented by general formula [2] or a salt thereof to mammals, so as to induce the pyrazine nucleotide or pyrazine nucleoside analog represented by general formula [1] or a salt thereof in vivo, exhibits a novel virus growth-inhibiting effect and/or a novel virucidal effect, thereby completing the present invention.

The method of the present invention is useful as a method for treating patients infected with virus, which comprises a step of administering to a patient infected with virus the aforementioned pyrazine nucleotide or pyrazine nucleoside analog or a salt thereof such as the compound represented by general formula [3z] or a salt thereof. More preferably, the method of the present invention further comprises a step of converting the above compound or a salt

thereof into the pyrazine triphosphate nucleotide analog represented by general formula [1y].

Moreover, it is preferably that, in the body of a patient infected with virus, the general formula [3z] is converted into the pyrazine triphosphate nucleotide analog represented by general formula [1y] through a pyrazine nucleotide analog represented by the following general formula [1v]:

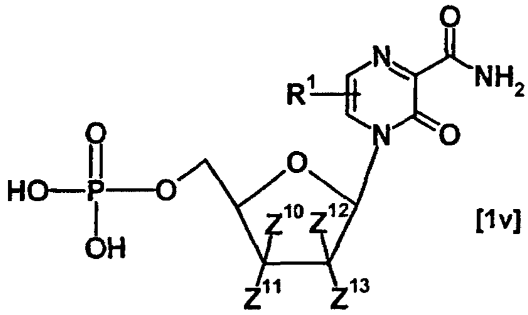

[1v]

wherein $R^1$ represents a hydrogen atom, or a substituent of a pyrazine ring; and each of $Z^{10}$, $Z^{11}$, $Z^{12}$ and $Z^{13}$ identically or differently represents a hydrogen atom or hydroxyl group. The pyrazine nucleotide analog represented by general formula [1v] is characterized in that it does not substantially inhibit inosine monophosphate dehydrogenase derived from host cells. Moreover, the pyrazine triphosphate nucleotide analog represented by general formula [1y] is characterized in that it inhibits virus-derived RNA polymerase more selectively than host-derived RNA polymerase.

Furthermore, as a result of intensive studies of the present inventors regarding anhydrides of 4-[(2R, 3R, 4S, 5R)-3,4-dihydroxy-5-(hydroxymethyl)tetrahydro-2-furanyl]-3-oxo-3,4-dihydro-2-pyrazinecarboxamide that is a representative compound of the present invention, they have found 4-[(2R, 3R, 4S, 5R)-3,4-dihydroxy-5-(hydroxymethyl) tetrahydro-2-furanyl]-3-oxo-3,4-dihydro-2-pyrazinecarboxamide monohydrate, which has excellent stability during the process of pharmaceutical preparation. This hydrate is a crystal that is stable during the process of pharmaceutical preparation by common methods, and during the process of pharmaceutical preparation, and it has less dustability and does not adhere to instruments when compared with anhydrides. Accordingly, it enables good mixing and granulation.

Wet granulation is generally used as granulation during the process of pharmaceutical preparation. During such wet granulation, water and an aqueous solution containing a binder are generally used. However, it is known that when an anhydride is used, a part of the anhydride is converted into a hydrate depending on conditions. Moreover, an amorphous substance generated in this process causes a problem from the viewpoint of pharmaceutical preparation or stability. Accordingly, when a hydrate exists as a crystal polymorph, strict conditions are required for preparation of a pharmaceutical from an anhydride. However, a monohydrate is a stable crystal during the common pharmaceutical preparation process, and therefore, it is an excellent compound that does not cause the aforementioned problem.

In addition, such a monohydrate does not need an organic solvent in the final preparation process, but it can be crystallized from water. Accordingly, the risk that organic solvents are remained in the finally obtained crystal is low. Moreover, since the monohydrate does not need an organic solvent, it does not need explosion-proof equipment. Thus, it can be said that this compound has great advantages on the production process.

The compound of the present invention will be described in detail below.

BEST MODE FOR CARRYING OUT THE INVENTION

In the specification, unless otherwise mentioned, a halogen atom means a fluorine atom, a chlorine atom, and an iodine atom; an alkyl group means a lower alkyl group, for example, a $C_{1-5}$ alkyl group such as methyl, ethyl, n-propyl, isopropyl, n-butyl, isobutyl, sec-butyl, tert-butyl, and pentyl; an alkoxy group means a lower alkoxy group, for example, a $C_{1-5}$ alkoxy group such as methoxy, ethoxy, n-propoxy, isopropoxy, n-butoxy, isobutoxy, sec-butoxy, tert-butoxy, and pentyloxy; an alkoxycarbonyl group means a lower alkoxycarbonyl group, for example, a $C_{1-5}$ alkoxycarbonyl group such as methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl, 4-hydroxybutoxycarbonyl, and pentyloxycarbonyl; an alkylamino group means, for example, a mono- or di-$C_{1-5}$ alkylamino group such as methylamino, ethylamino, propylamino, dimethylamino, diethylamino, and methylethylamino; a halogenoalkyl group means, for example, a halogeno-$C_{1-5}$ alkyl group such as fluoromethyl, chloromethyl, bromomethyl, dichloromethyl, trifluoromethyl, trichloromethyl, chloroethyl, dichloroethyl, trichloroethyl, and chloropropyl; a carbamoylalkyl group means, for example, a $C_{1-5}$ carbamoylalkyl group, such as carbamoylmethyl, carbamoylethyl, carbamoyl-n-propyl, carbamoylisopropyl, carbamoyl-n-butyl, carbamoylisobutyl, and carbamoylpentyl; a carboxyalkyl group means, for example, a $C_{1-5}$ carboxyalkyl group

such as carboxymethyl, carboxyethyl, carboxy-n-propyl, carboxyisopropyl, carboxy-n-butyl, carboxyisobutyl, and carboxypentyl; an alkenyl group means, for example, a $C_{2-5}$ alkenyl group such as vinyl and allyl; a cycloalkyl group means, for example, a $C_{3-6}$ cycloalkyl group such as cyclopropyl, cyclobutyl, cyclopentyl, and cyclohexyl; a cycloalkyloxy group means, for example, a $C_{3-6}$ cycloalkyloxy group such as cyclopropyloxy, cyclobutyloxy, cyclopentyloxy, and cyclohexyloxy; an aryl group means, for example, a group such as phenyl and naphthyl; a heterocyclic group means, for example, a 4- to 6-membered or condensed heterocyclic group containing at least one heteroatom selected from an oxygen atom, a nitrogen atom, and a sulfur atom such as azetidinyl, thienyl, furyl, pyrrolyl, imidazolyl, pyrazolyl, thiazolyl, isothiazolyl, oxazolyl, isoxazolyl, furazanyl, pyrrolidinyl, pyrrolynyl, imidazolydinyl, imidazolynyl, pyrazolidinyl, pyrazolinyl, 1,3,4-oxadiazolyl, 1,2,3-thiadiazolyl, 1,2,4-thiadiazolyl, 1,3,4-thiadiazolyl, 1,2,3-triazolyl, 1,2,4-triazolyl, thiatriazolyl, pyridyl, pyrazinyl, pyrimidinyl, pyridazinyl, pyranyl, morpholinyl, 1,2,4-triazinyl, benzothienyl, naphthothienyl, benzofuryl, isobenzofuryl, chromenyl, indolizinyl, isoindolyl, indolyl, indazolyl, purinyl, quinolyl, isoquinolyl, phthalazinyl, naphthylidinyl, quinoxalinyl, quinazolinyl, cinnolinyl, pteridinyl, isochromanyl, chromanyl, indolinyl, isoindolinyl, benzoxazolyl, triazolopyridyl, tetrazolopyridazinyl, tetrazolopyrimidinyl, thiazolopyridazinyl, thiadiazolopyridazinyl, triazolopyridazinyl, benzimidazolyl, benzothiazolyl, 1,2,3,4-tetrahydroquinolyl, imidazo[1,2-b][1,2,4]triazinyl, and quinuclidinyl; an alkylene group means, for example, a straight or branched $C_{1-5}$ alkylene group such as methylene, ethylene, and propylene; an alkylthio group means, for example, a straight or branched $C_{1-5}$ alkylthio group such as methylthio, ethylthio, n-propylthio, isopropylthio, n-butylthio, isobutylthio, sec-butylthio, tert-butylthio, and pentylthio; an aryloxy group means, for example, a group represented by aryl-O- such as phenoxy and naphthoxy; an arylthio group means, for example, a group represented by aryl-S- such as phenylthio and naphthylthio; an arylamino group means, for example, phenylamino and naphthylamino; a cycloalkylamino group means, for example, a $C_{3-6}$ cycloalkylamino group such as cyclopropylamino, cyclobutylamino, cyclopentylamino, and cyclohexylamino; an acyl group means, for example, $C_{2-6}$ alkanoyl group such as formyl, acetyl, or propionyl, an aroyl group such as benzoyl or naphthoyl, and a heterocyclic carbonyl group such as nicotinoyl, thenoyl, pyrrolidinocarbonyl, or furoyl; an acyloxy group means, for example, a $C_{2-6}$ alkanoyloxy group such as acetyloxy or propionyloxy, an aroyloxy group such as benzoyloxy or naphthoyloxy, and a heterocyclic carbonyloxy group such as nicotinoyloxy, thenoyloxy, pyrrolidinocarbonyloxy or furoyloxy; an arylsulfonyloxy group means, for example, a group such as phenylsulfonyloxy and p-toluenesulfonyloxy; an alkylsulfonyloxy group means, for example, a straight or branched $C_{1-5}$ alkylsulfonyloxy group such as methylsulfonyloxy, ethylsulfonyloxy, n-propylsulfonyloxy, isopropylsulfonyloxy, n-butylsulfonyloxy, isobutylsulfonyloxy, sec-butylsulfonyloxy, tert-butylsulfonyloxy, and pentylsulfonyloxy, respectively.

In a general formula described in the specification, substituents of a pyrazine ring of $R^1$ include a group selected from a halogen atom; an alkyl group that may be substituted by hydroxyl, alkoxy, alkylthio, aryl, amino, or alkylamino group; an alkyl or alkenyl group that may be substituted by a halogen atom; a cycloalkyl group; a hydroxyl group; an alkoxy group; a cycloalkyloxy group; an alkoxycarbonyl group; a mercapto group; an alkylthio group that may be substituted by an aryl group; an aryl group; an aryloxy group; an arylthio group; an arylamino group; a cyano group; a nitro group; an amino group that may be substituted by an acyl group; an alkylamino group; a cycloalkylamino group; an acyl group; a carboxyl group; a carbamoyl group; a thiocarbamoyl group; an alkylcarbamoyl group; and a heterocyclic group, and one or more such groups may be substituted.

Protecting groups and substituents of a hydroxyl group of $R^Z$ include, for example, an acyl group that may be substituted, a lower alkoxycarbonyl group, and an acyloxyalkyl group, more specifically, an acyl group that may be substituted, such as acetyl, propionyl, valeryl, benzoyl, pivaloyl, 2-aminoacetyl, 2-aminopropionyl, 2-aminovaleryl, and 2-aminocaprolyl; a lower alkoxycarbonyl group such as methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl, and 4-hydroxybutoxycarbonyl; and an acyloxyalkyl group such as acetyloxymethyl, propionyloxymethyl, isopropionyloxymethyl, butyryloxymethyl, isobutyryloxymethyl, valeryloxymethyl, isovaleryloxymethyl, pivaloyloxymethyl, and 1-pivaloyloxyethyl.

$R^7$ and $R^8$, each independently a protecting group or substituent of a hydroxyl group to be decomposed under the physiological condition in the phosphate or phosphonate, and the R group decomposable under such condition are, for example, a protecting group or substituent of the phosphate or phosphonate described in Progress in Medicinal Chemistry, Vol. 34, pp. 111-147 (1997), Elsevier Science B.V., and Current Medicinal Chemistry, Vol. 7, pp. 995-1039 (2000). The specific examples include an aryl group such as phenyl, chlorophenyl, nitrophenyl, cyanophenyl, naphthyl; a cyclosaligenyl group such as cyclosaligenyl, 5-methylcyclosaligenyl; an amidate group such as methoxyalaninyl and phenoxyalaninyl; a haloethyl group such as trichloroethyl; an acyloxyalkyl group such as acetyloxymethyl, propionyloxymethyl, isopropionyloxymethyl, butyryloxymethyl, isobutyryloxymethyl, valeryloxymethyl, isovaleryloxymethyl, pivaloyloxymethyl, and 1-pivaloyloxyethyl; an acyloxybenzyl group such as acetyloxybenzyl, propionyloxybenzyl, isopropionyloxybenzyl, butyryloxybenzyl, isobutyryloxybenzyl, valeryloxybenzyl, isovaleryloxybenzyl, pivaloyloxybenzyl; an s-lower acylthioalkyl group such as acetylthioethyl, propionylthioethyl, isopropionylthioethyl, butyrylthioethyl, isobutyrylthioethyl, valerylthioethyl, isovalerylthioethyl, pivaloylthioethyl, and pivaloylthiobutyl; an s-higher acylthioalkyl group such as lauroylthioethyl; an s-aroylthioalkyl group such as benzoylthioethyl and naphthoylthioethyl; and dithiodiethyl group.

The expression "decomposed under the physiological condition" means to be decomposed by an enzyme such as esterase, phosphodiesterase, phosphonamidase, hydrolase, aminohydrolase, transaminase, or reductase, as well as a physiological oxidation, hydrolysis, and/or reduction reaction.

Examples of a kinase include a nucleotide kinase, nucleoside kinase, nucleoside phosphotransferase, and 5'-nucleotidase.

A precursor means a substance that produces a pharmacologically active substance itself by conversion/ decomposition in vivo.

Examples of a protecting group of a carboxyl group include all groups that can be used as a usual protecting group of a carboxyl group, for example, a lower alkyl group such as methyl, ethyl, n-propyl, isopropyl, 1,1-dimethylpropyl, n-butyl, and tert-butyl; an aryl group such as phenyl and naphthyl; an aryl-lower alkyl group such as benzyl, diphenylmethyl, trityl, p-nitrobenzyl, p-methoxybenzyl, and bis(p-methoxyphenyl)methyl; an acyl-lower alkyl group such as acetylmethyl, benzoylmethyl, p-nitrobenzoylmethyl, p-bromobenzoylmethyl, and p-methanesulfonylbenzoylmethyl; an oxygen-containing heterocyclic group such as 2-tetrahydropyranyl and 2-tetrahydrofuranyl; a halogeno-lower alkyl group such as 2,2,2-trichloroethyl; a lower alkylsilylalkyl group such as 2-(trimethylsilyl)ethyl; an acyloxyalkyl group such as acetoxymethyl, propionyloxymethyl, and pivaloyloxymethyl; a nitrogen-containing heterocyclic-lower alkyl group such as phthalimidemethyl and succinimidemethyl; a cycloalkyl group such as cyclohexyl; a lower alkoxy-lower alkyl group such as methoxymethyl, methoxyethoxymethyl, and 2-(trimethylsilyl)ethoxymethyl; an aryl-lower alkoxy-lower alkyl group such as benzyloxymethyl; a lower alkylthio-lower alkyl group such as methylthiomethyl and 2-methylthioethyl; an arylthio-lower alkyl group such as phenylthiomethyl; a lower alkenyl group such as 1,1-dimethyl-2-propenyl, 3-methyl-3-butynyl, and aryl; and a substituted silyl group such as trimethylsilyl, triethylsilyl, triisopropylsilyl, diethylisopropylsilyl, tert-butyldimethylsilyl, tert-butyldiphenylsilyl, diphenylmethylsilyl, and tert-butylmethoxyphenylsilyl.

Examples of a protecting group of an amino group and an imino group include all groups that can be used as a usual amino protecting group, for example, an acyl group such as trichloroethoxycarbonyl, tribromoethoxycarbonyl, benzyloxycarbonyl, p-nitrobenzyloxycarbonyl, o-bromobenzyloxycarbonyl, (mono-, di-, tri-)chloroacetyl, trifluoroacetyl, phenylacetyl, formyl, acetyl, benzoyl, tert-amyloxycarbonyl, tert-butoxycarbonyl, p-methoxybenzyloxycarbonyl, 3,4-dimethoxybenzyloxycarbonyl, 4-(phenylazo)benzyloxycarbonyl, 2-furfuryloxycarbonyl, diphenylmethoxycarbonyl, 1,1-dimethylpropoxycarbonyl, isopropoxycarbonyl, phthaloyl, succinyl, alanyl, leucyl, 1-adamantyloxycarbonyl, and 8-quinolyloxycarbonyl; an aryl-lower alkyl group such as benzyl, diphenylmethyl, and trityl; an arylthio group such as 2-nitrophenylthio and 2,4-dinitrophenylthio; an alkane- or arene-sulfonyl group such as methanesulfonyl and p-toluenesulfonyl; a di-lower alkylamino-lower alkylidene group such as N,N-dimethylaminomethylene; an aryl-lower alkylidene group such as benzylidene, 2-hydroxybenzylidene, 2-hydroxy-5-chlorobenzylidene, and 2-hydroxy-1-naphthylmethylene; a nitrogen-containing heterocyclic alkylidene group such as 3-hydroxy-4-pyridylmethylene; a cycloalkylidene group such as cyclohexylidene, 2-ethoxycarbonylcyclohexylidene, 2-ethoxycarbonylcyclopentylidene, 2-acetylcyclohexylidene, and 3,3-dimethyl-5-oxycyclohexylidene; a diaryl- or diaryl-lower alkylphosphoryl group such as diphenylphosphoryl and dibenzylphosphoryl; an oxygen-containing heterocyclic alkyl group such as 5-methyl-2-oxo-2H-1,3-dioxol-4-yl-methyl; and a lower alkyl substituted silyl group such as trimethylsilyl.

Examples of a protecting group of a hydroxyl group include all groups that can be used as a usual hydroxyl protecting group, for example, an acyl group such as benzyloxycarbonyl, 4-nitrobenzyloxycarbonyl, 4-bromobenzyloxycarbonyl, 4-methoxybenzyloxycarbonyl, 3,4-dimethoxybenzyloxycarbonyl, methoxycarbonyl, ethoxycarbonyl, tert-butoxycarbonyl, 1,1-dimethylpropoxycarbonyl, isopropoxycarbonyl, isobutyloxycarbonyl, diphenylmethoxycarbonyl, 2,2,2-trichloroethoxycarbonyl, 2,2,2-tribromoethoxycarbonyl, 2-(trimethylsilyl)ethoxycarbonyl, 2-(phenylsulfonyl) ethoxycarbonyl, 2-(triphenylphosphonio)ethoxycarbonyl, 2-furfuryloxycarbonyl, 1-adamantyloxycarbonyl, vinyloxycarbonyl, allyloxycarbonyl, S-benzylthiocarbonyl, 4-ethoxy-1-naphthyloxycarbonyl, 8-quinolyloxycarbonyl, acetyl, formyl, chloroacetyl, dichloroacetyl, trichloroacetyl, trifluoroacetyl, methoxyacetyl, phenoxyacetyl, pivaloyl, and benzoyl; a lower alkyl group such as methyl, tert-butyl, 2,2,2-trichloroethyl, and 2-trimethylsilylethyl; a lower alkenyl group such as allyl; an aryl-lower alkyl group such as benzyl, p-methoxybenzyl, 3,4-dimethoxybenzyl, diphenylmethyl, and trityl; an oxygen- and sulfur-containing heterocyclic group such as tetrahydrofuryl, tetrahydropyranyl, and tetrahydrothiopyranyl; a lower alkoxy- and lower alkylthio-lower alkyl group such as methoxymethyl, methylthiomethyl, benzyloxymethyl, 2-methoxyethoxymethyl, 2,2,2-trichloroethoxymethyl, 2-(trimethylsilyl)ethoxymethyl, and 1-ethoxyethyl; an alkyl- and aryl-sulfonyl group such as methanesulfonyl and p-toluenesulfonyl; and a substituted silyl group such as trimethylsilyl, triethylsilyl, triisopropylsilyl, diethylisopropylsilyl, tert-butyldimethylsilyl, tert-butyldiphenylsilyl, diphenylmethylsilyl, and tert-butylmethoxyphenylsilyl, and in the case of a dihydroxyl group, further include a lower alkylidene group such as methylene, benzylidene, and isopropylidene, a lower alkoxy-lower alkylidene group such as methoxymethylene, and a lower alkyl substituted silyl group such as 1,1,3,3-tetraisopropyldisiloxanylidene.

A carbamoylalkyl group and a carboxyalkyl group of $R^2$ may be substituted by one or more substituents selected from a halogen atom; an alkyl group that may be substituted by a hydroxyl, alkoxy, alkylthio, aryl, amino, or alkylamino group; a halogenoalkyl group; an alkenyl group; a cycloalkyl group; hydroxyl group; alkoxy group; a cycloalkyloxy group; an alkoxycarbonyl group; a mercapto group; an alkylthio group that may be substituted by an aryl group; an aryl group; an aryloxy group; an arylthio group; an arylamino group; cyano group; nitro group; an amino group that may be substituted by an acyl group; an alkylamino group; a cycloalkylamino group; an acyl group; a carboxyl group; a carbamoyl group; a thiocarbamoyl group; an alkylcarbamoyl group, and a heterocyclic group.

A hydroxyl group of $R^3$, $R^4$, $R^5$, and $R^6$ may be substituted by one or more substituents selected from a carboxyl group that may be protected, an alkyl group, an alkoxycarbonyl group, an aryl group, a cycloalkyl group, an alkenyl group, a halogenoalkyl group, and a heterocyclic group.

Examples of salts of general formula [1] and general formula [2] include salts of a usually known basic group such as an amino group, or an acidic group such as a hydroxyl, phosphoryl, phosphonyl, or carboxyl group. Salts of basic groups include, for example, a salt with a mineral acid such as hydrochloric acid, hydrobromic acid, and sulfuric acid; a salt with an organic carboxylic acid such as tartaric acid, formic acid, and citric acid; and a salt with a sulfonic acid such as methanesulfonic acid, benzenesulfonic acid, p-toluenesulfonic acid, mesitylenesulfonic acid, and naphthalenesulfonic acid. Salts of acidic groups include, for example, a salt with an alkali metal such as sodium and potassium; a salt with an alkali earth metal salt such as calcium and magnesium; an ammonium salt; as well as a salt with a nitrogen-containing organic base such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine.

Moreover, when the compounds represented by general formulas [1], [1v], [1w], [1x], [1y], [1z], [2] and [3z], and salts thereof have isomers (e.g., optical isomers, geometric isomers, tautomers, etc.), the present invention includes these isomers, solvates, hydrates, and various forms of crystals.

Examples of virus to which the virus growth inhibition and/or virucidal method of the present invention is applied may include influenza virus, RS virus, AIDS virus, papilloma virus, adenovirus, hepatitis A virus, hepatitis B virus, hepatitis C virus, hepatitis E virus, poliovirus, echovirus, Coxsackie virus, enterovirus, rhinovirus, rotavirus, Newcastle disease virus, mumps virus, vesicular stomatitis virus, rabies virus, Lassa fever virus, measles virus, Filovirus, Japanese encephalitis virus, yellow fever virus, dengue fever virus or West Nile virus. Preferably, such examples may include influenza virus, RS virus, hepatitis A virus, hepatitis C virus, hepatitis E virus, poliovirus, echovirus, Coxsackie virus, enterovirus, rhinovirus, rotavirus, Newcastle disease virus, mumps virus, vesicular stomatitis virus, rabies virus, Lassa fever virus, measles virus, Filovirus, Japanese encephalitis virus, yellow fever virus, dengue fever virus or West Nile virus. Particularly preferably, such examples may include influenza virus and hepatitis C virus.

Preferred compounds of the present invention may include compounds having the below mentioned substituents.

In each of the general formulas described in the present invention, examples of a preferred substituent of $R^1$ may include a hydrogen atom, a halogen atom, a lower alkyl group and a hydroxyl group, more preferably a hydrogen atom, a fluorine atom and a chlorine atom, and further more preferably a hydrogen atom.

Examples of a preferred substituent of $R^2$ may include a hydrogen atom, an acetyl group, a benzoyl group, a pivaloyl group, a carbamoylmethyl group and a carboxymethyl group, more preferably a hydrogen atom, an acetyl group and a carboxymethyl group, and further more preferably a hydrogen atom.

Examples of a preferred substituent of each of $R^3$, $R^4$, $R^5$ and $R^6$ may include a hydrogen atom and a hydroxyl group that may be substituted with a lower alkoxycarbonyl, acetyl, benzoyl or pivaloyloxymethyl group, more preferably a hydrogen atom and a hydroxyl group that may be substituted with an acetyl or benzoyl group, and further more preferably a hydrogen atom and a hydroxyl group.

Examples of a preferred substituent of each of $R^{4Z}$ and $R^{6Z}$ may include the same substituents described as for $R^4$ and $R^6$, and a methylene group in which both $R^{4Z}$ and $R^{6Z}$ may be substituted.

Examples of a preferred substituent of $R^Z$ may include a hydroxyl group that may be substituted with an acyl that may be substituted, lower alkoxycarbonyl, or acyloxyalkyl group, more preferably a hydroxyl group that may be substituted with an isovaleryl, acetyl or propionyl group that may be substituted with an amino group that may be protected, benzoyl group, pivaloyl group, ethoxycarbonyl group, isopropyloxycarbonyl group, or pivaloyloxymethyl group, and further more preferably a hydroxyl group that may be substituted with an isovaleryl, acetyl or benzoyl group that may be substituted with an amino group.

Examples of a preferred substituent of each of $R^7$ and $R^8$ may include a hydroxyl group that may be substituted with a cyclosaligenyl, pivaloyloxymethyl, 1-pivaloyloxyethyl or S-pivaloyl-2-thioethyl group.

An example of a preferred substituent of Y may be an oxygen atom. An example of a preferred substituent of A may be an oxygen atom.

In particular, a preferred example of the compound represented by general formula [3z] is a compound represented by general formula [3z']:

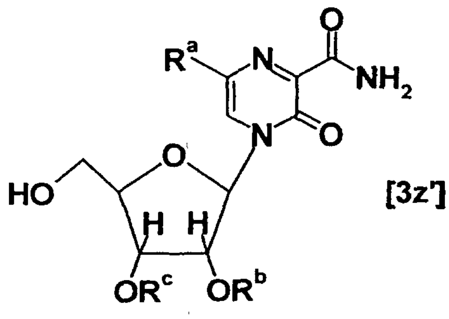

[3z']

wherein $R^a$ represents a hydrogen or halogen atom; and each of $R^b$ and $R^c$ identically or differently represents a hydrogen atom or hydroxyl protecting group, or $R^b$ and $R^c$ together represent an alkylene group that may be substituted. A compound wherein, in the general formula [3z'], each of $R^a$, $R^b$ and $R^c$ represents a hydrogen atom is more preferable.

The compounds indicated below are representative compounds of the present invention. Codes in the formulas have the following meanings.

Ac: acetyl, Bz: benzoyl, Me: methyl, and Et: ethyl

In addition, sugar chains in the following formulas of the representative compounds are described in commonly used expressions. For example, the configuration of a compound represented by the following formula:

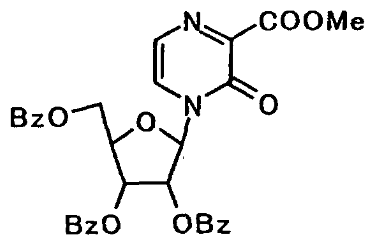

means each of the compounds represented by the following formulas:

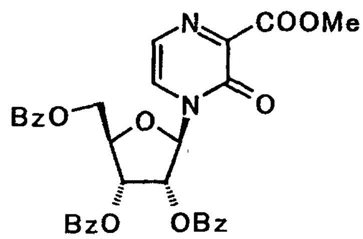

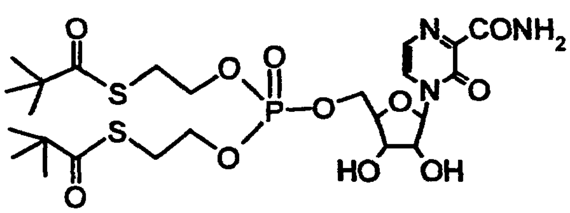

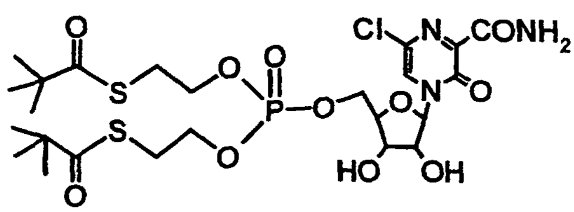

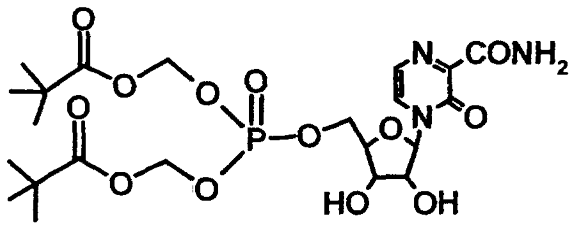

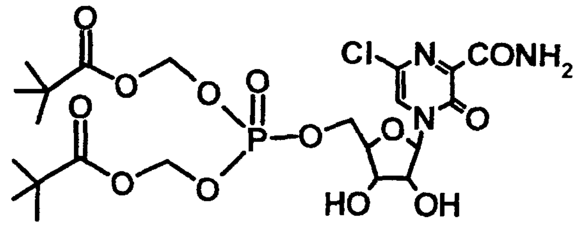

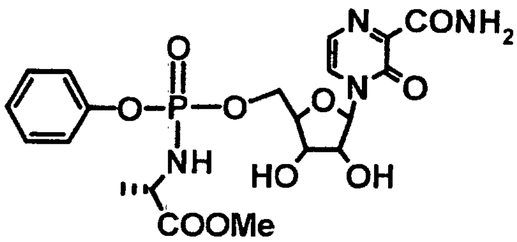

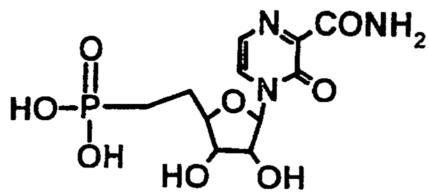

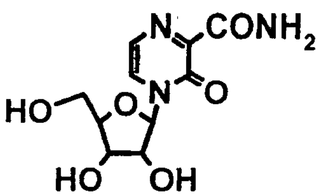

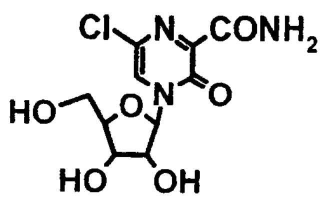

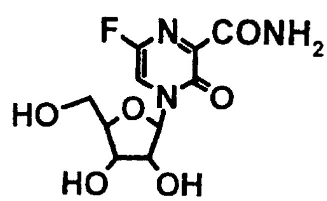

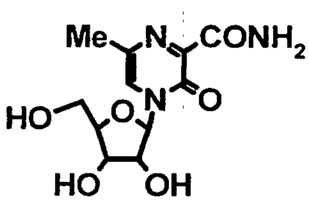

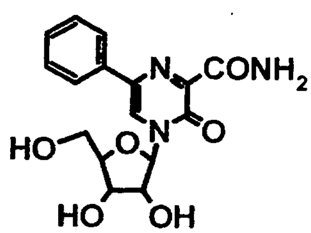

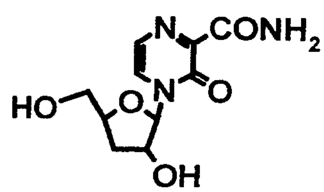

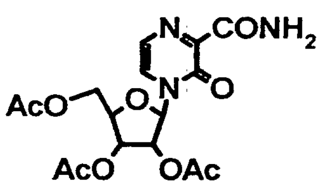

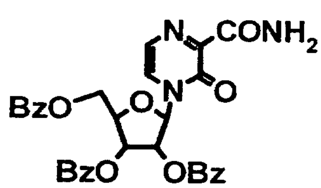

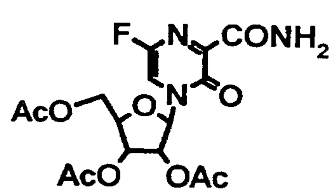

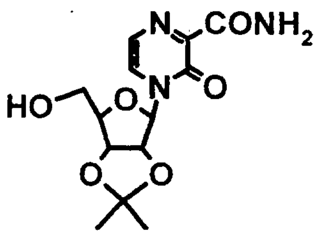
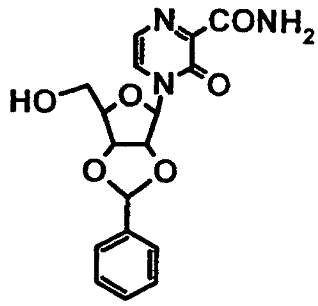
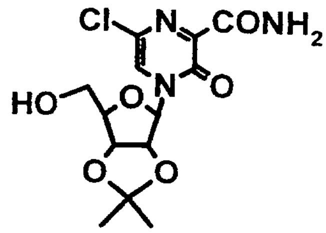

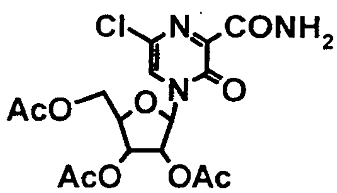
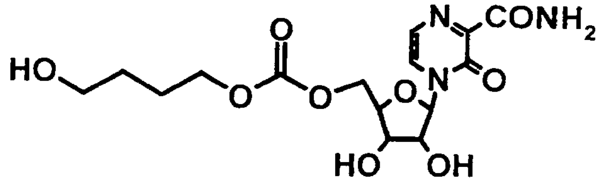

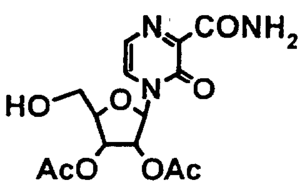
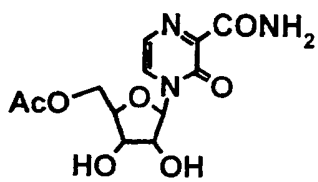
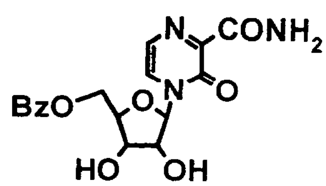

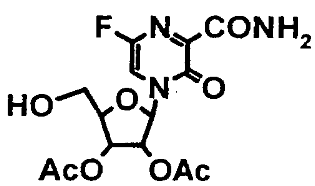
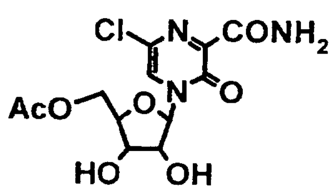
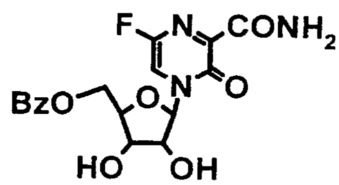

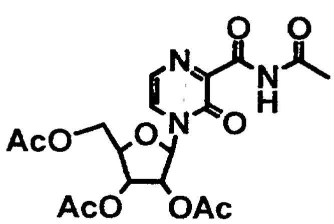
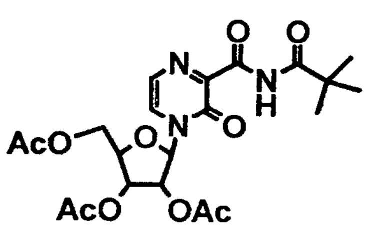
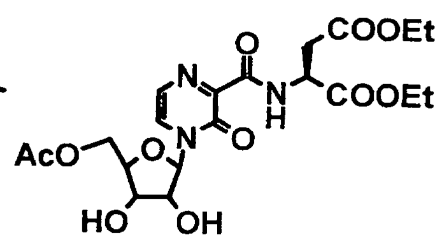

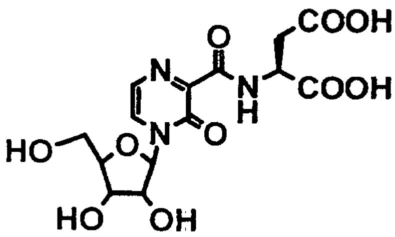
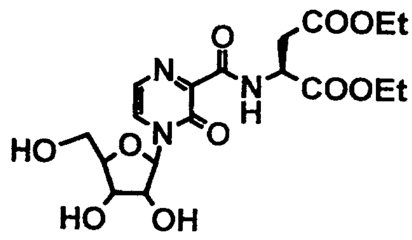

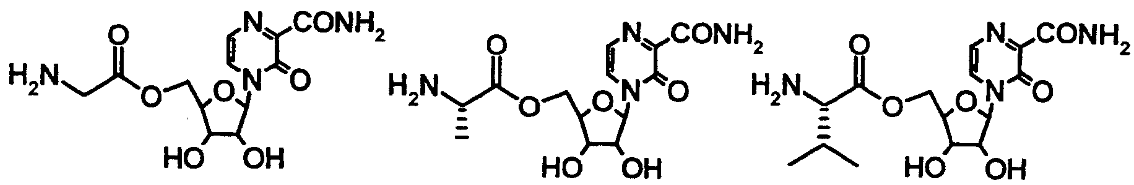

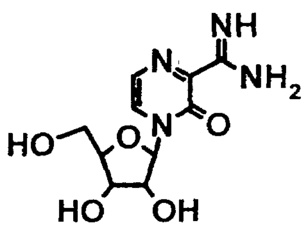
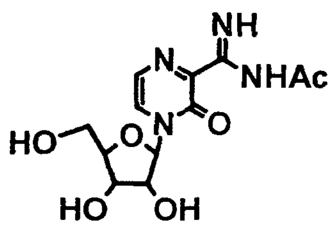
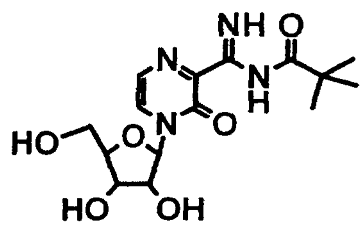

Next, a method for producing the compound of the present invention will be explained.

The pyrazine nucleotide analog represented by general formula [2] or a salt thereof can be produced by known methods, methods equivalent thereto, or a combined use of these methods. Examples of publications which describe the production methods may include Antiviral Research, Vol. 24, pp. 69 to 77 (1994); Antiviral Chemistry, Vol. 9, pp. 389 to 402 (1998); Journal of Chemical Society Perkin Transaction (J. Chem. Soc. PERKIN TRANS.) Vol. 1, pp. 1239 to 1245 (1993); and US Patent No. 5770725. Moreover, the compound of the present invention can also be produced in accordance with the routes of production methods 1 to 5 as shown below.

[Production method 1]

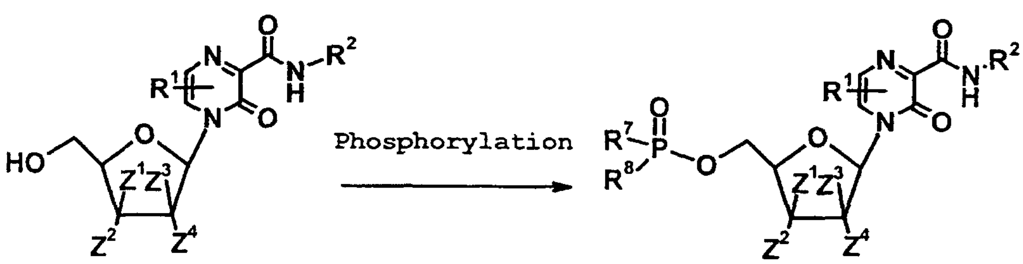


[3a] [2a]

wherein each of $R^1$, $R^2$, $R^7$ and $R^8$ has the meanings as described above; and each of $Z^1$, $Z^2$, $Z^3$ and $Z^4$ identically or

differently represents a hydrogen atom or protected hydroxyl group, however, when $Z^1$, $Z^2$, $Z^3$ and $Z^4$ have hydroxyl groups binding to two or more different carbon atoms, oxygen atoms of each hydroxyl group and carbon atoms to which each hydroxyl group binds form a ring together with protecting groups, so that they may be protected.

(a) The compound represented by general formula [2a] or a salt thereof can be obtained by (1) reacting the compound represented by general formula [3a] or a salt thereof with a phosphorylation agent in the presence or absence of additives, or (2) reacting the same above compound or a salt thereof with a phosphitylation agent in the presence or absence of additives, followed by reacting with an oxidizing agent, according to the method described in e.g., the 4th Jikken Kagaku Koza, Vol. 22, pp. 313 to 438 (1992).

In the method according to (1) above, a solvent used in this reaction is not particularly limited as long as it does not affect the reaction. Examples of such a solvent may include: aromatic hydrocarbons such as benzene, toluene or xylene; ethers such as dioxane, tetrahydrofuran, anisole, diethylene glycol diethyl ether or dimethyl cellosolve; nitriles such as acetonitrile; amides such as N,N-dimethylformamide or N,N-dimethylacetamide; sulfoxides such as dimethyl sulfoxide; phosphoric esters such as trimethyl phosphate; and pyridine. One or more types of these solvents may be used in combination.
Any phosphorylation agent can be used in this reaction as long as it is generally used in phosphorylation of hydroxyl groups. Examples of such a reagent may include phosphoric diesters such as dibenzyl phosphate; phosphoric dithioesters such as S,S'-diphenyl phosphorodithioate monocyclohexylammonium; and phosphoric chlorides such as phosphoryl chloride or methylchlorophenylphosphoryl P→N-L-alaninate. Such a phosphorylation agent may be used in an amount equimolar or greater, and more preferably at a molar ratio of 1.0 : 1.0 to 5.0 : 1.0, with respect to the compound represented by general formula [3a] or a salt thereof.
Examples of an additive used in this reaction may include azo compounds such as diethyl azodicarboxylate or diisopropyl azodicarboxylate, phosphines such as triphenyl phosphine, allene sulfonyl chlorides such as 2,4,6-triisopropyl benzenesulfonyl chloride, and bases such as pyridine or tert-butyl magnesium chloride. These may be used in combination. Such an additive may be used in an amount equimolar or greater, and more preferably at a molar ratio of 1.0 : 1.0 to 5.0 : 1.0, with respect to the compound represented by general formula [3a] or a salt thereof.
This reaction may be carried out generally at - 50°C to 170°C, preferably at 0°C to 100°C and for 1 minute to 72 hours, preferably for 5 minutes to 24 hours.
In the method according to (2) above, a solvent used in this reaction is not particularly limited as long as it does not affect the reaction. Examples of such a solvent may include: aromatic hydrocarbons such as benzene, toluene or xylene; ethers such as dioxane, tetrahydrofuran, anisole, diethylene glycol diethyl ether or dimethyl cellosolve; nitriles such as acetonitrile; amides such as N,N-dimethylformamide or N,N-dimethylacetamide; sulfoxides such as dimethyl sulfoxide; and pyridine. One or more types of these solvents may be used in combination.
Any phosphitylation agent can be used in this reaction as long as it is generally used in phosphitylation of hydroxyl groups. Examples of such a reagent may include phosphoramidites such as diallyl diisopropyl phosphoramidite or bis(S-pivaloyl-2-thioethyl)N,N-diisopropyl phosphoramidite, and phosphorous chlorides such as diallyl phosphorochloridite. Such a phosphitylation agent may be used in an amount equimolar or greater, and more preferably at a molar ratio of 1.0 : 1.0 to 3.0 : 1.0, with respect to the compound represented by general formula [3a] or a salt thereof.
Examples of an additive used in this reaction may include nitrogen-containing heterocyclic rings such as 1H-tetrazole, 4-dimethylaminopyridine, pyridine or collidine, and these may be used in combination. Such an additive may be used in an amount equimolar or greater, and more preferably at a molar ratio of 1.0 : 1.0 to 5.0 : 1.0, with respect to the compound represented by general formula [3a] or a salt thereof.
Examples of an oxidizing agent used in this reaction may include peroxides such as metachloroperbenzoic acid or tert-butylhydroperoxide, and halogenated compounds such as iodine. Such an oxidizing agent may be used in an amount equimolar or greater, and more preferably at a molar ratio of 1.0 : 1.0 to 5.0 : 1.0, with respect to the compound represented by general formula [3a] or a salt thereof.
This reaction may be carried out generally at - 78°C to 100°C, preferably at -50°C to 50°C and for 1 minute to 24 hours, preferably for 5 minutes to 6 hours.

[Production method 2]

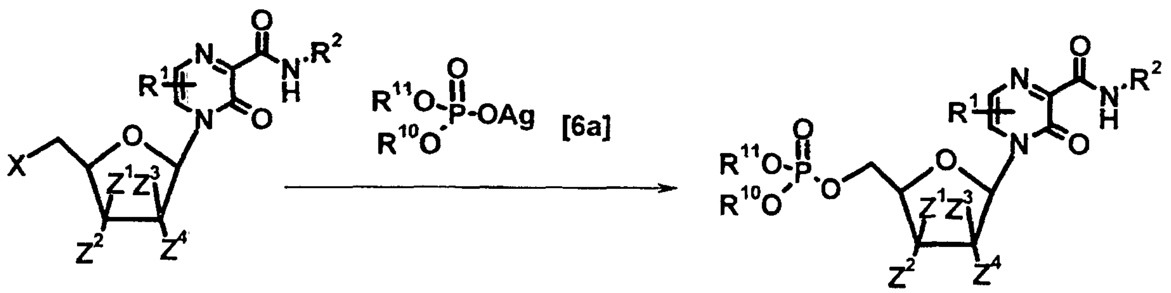


[3b] [2b]

wherein each of $R^1$, $R^2$, $Z^1$, $Z^2$, $Z^3$ and $Z^4$ has the same meaning as given above; each of $R^{10}$ and $R^{11}$ identically or differently represents a protecting group of phosphoric acid decomposed under physiological conditions; and X represents a halogen atom.

The compound represented by general formula [2b] or a salt thereof can be obtained by reacting the compound represented by general formula [3b] or a salt thereof with the compound represented by general formula [6a] according to the method described in e.g., Journal of Medicinal Chemistry, Vol. 37, pp. 3902 to 3909 (1994).

A solvent used in this reaction is not particularly limited as long as it does not affect the reaction. Examples of such a solvent may include: aromatic hydrocarbons such as benzene, toluene or xylene; halogenated hydrocarbons such as methylene chloride or chloroform; ethers such as dioxane, tetrahydrofuran, anisole, diethylene glycol diethyl ether or dimethyl cellosolve; nitriles such as acetonitrile; amides such as N,N-dimethylacetamide; alcohols such as methanol, ethanol or propanol; sulfoxides such as dimethyl sulfoxide; and water. One or more types of these solvents may be used in combination.

The compound represented by general formula [6a] is used in an amount equimolar or greater, and more preferably at a molar ratio of 1.0 : 1.0 to 3.0 : 1.0, with respect of the compound represented by general formula [3b] or a salt thereof.

This reaction may be carried out generally at 0°C to 170°C, preferably at 20°C to 120°C and for 5 minutes to 24 hours, preferably for 1 to 10 hours.

[Production method 3]

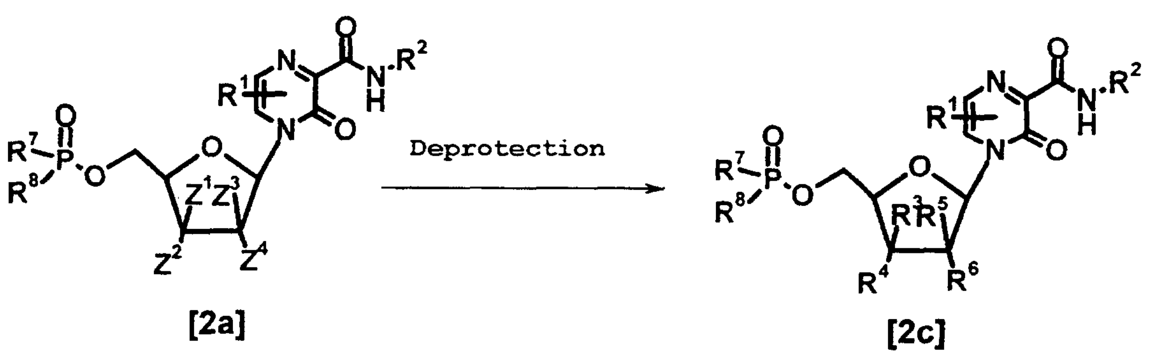


[2a] [2c]

wherein each of $R^1$, $R^2$, $R^3$, $R^4$, $R^5$, $R^6$, $R^7$, $R^8$, $Z^1$, $Z^2$, $Z^3$ and $Z^4$ has the same meaning as given above.

The compound represented by general formula [2c] or a salt thereof can be obtained by subjecting the compound represented by general formula [2a] or a salt thereof to a deprotection reaction.

A solvent used in this reaction is not particularly limited as long as it does not affect the reaction. Examples

of such a solvent may include: aromatic hydrocarbons such as benzene, toluene or xylene; ethers such as dioxane, tetrahydrofuran, anisole, diethylene glycol diethyl ether or dimethyl cellosolve; nitriles such as acetonitrile; amides such as N,N-dimethylacetamide; alcohols such as methanol, ethanol or propanol; sulfoxides such as dimethyl sulfoxide; and water. One or more types of these solvents may be used in combination.

Any deprotecting agent that is generally used in deprotection of hydroxyl groups may be used in this reaction, and preferred examples of such a reagent may include: bases such as sodium methoxide, ammonia gas, ammonia water, butylamine or hydrazine; acids such as formic acid, acetic acid aqueous solution, trifluoroacetic acid aqueous solution, hydrochloric acid, bromotrimethyl silane, Dowex 50WX4-200 ion exchange resin, or Amberlite IR-120 ion exchange resin; palladium catalysts such as tetrakis triphenyl phosphine palladium (0); and phosphines such as triphenyl phosphine. These may be used in combination, or may be produced in the reaction system. Such a deprotecting agent may be used at a molar ratio of 0.01 : 1 or more with respect to the compound represented by general formula [2a] or a salt thereof, and the deprotecting agent may also be used as a solvent.

This deprotection reaction may be carried out generally at -50°C to 170°C, preferably at -20°C to 100°C and for 1 minute to 100 hours, preferably for 5 minutes to 50 hours.

[Production method 4]

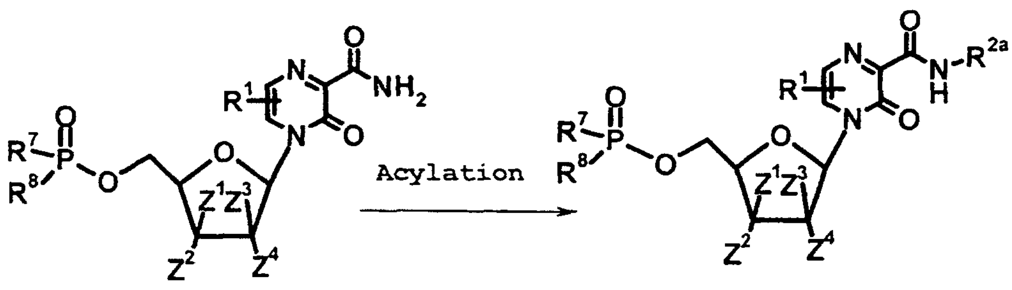


[2a'] [2d]

wherein each of $R^1$, $R^7$, $R^8$, $Z^1$, $Z^2$, $Z^3$ and $Z^4$ has the same meaning as given above; and $R^{2a}$ represents an acyl group.

The compound represented by general formula [2d] or a salt thereof can be obtained by subjecting the compound represented by general formula [2a'] or a salt thereof to an acylation reaction in the presence of a deacidification agent, in the presence or absence of an additive, according to the method described in e.g., the 4th Jikken Kagaku Koza, Vol. 22, pp. 137 to 151 and 166 to 169 (1992); Journal of Medicinal Chemistry, Vol. 44, pp. 777 to 786 (2001); or JP-A-10-195075.

A solvent used in this reaction is not particularly limited as long as it does not affect the reaction. Examples of such a solvent may include: ethers such as dioxane, tetrahydrofuran, anisole, diethylene glycol diethyl ether or dimethyl cellosolve; aromatic hydrocarbons such as benzene, toluene or xylene; halogenated hydrocarbons such as dichloromethane, chloroform or dichloroethane; amides such as N,N-dimethylformamide or N,N-dimethylacetamide; and water. These solvents may also be used in combination.

Examples of an acylating agent used in this reaction may include: carboxylic acids such as acetic acid; protected amino acids such as N-(tert-butoxycarbonyl)-L-valine; acid halides such as pivalic acid chloride; acid anhydrides such as acetic anhydride; imidazoles such as 1,1'-carbonyldiimidazole; carboxylates such as methyl acetate; and amide acetals such as N,N-dimethylacetamide dimethylacetal. These agents may be produced in the reaction system. Such an acylating agent may be used in an amount equimolar or greater, and preferably at a molar ratio of 1.0 : 1.0 to 2.0 : 1.0, with respect to the compound represented by general formula [2a'].

Examples of a deacidification agent used in this reaction may include pyridine, triethylamine, sodium hydride, potassium tert-butoxide, potassium carbonate and sodium bicarbonate. Such a deacidification agent may be used in an amount equimolar or greater, and preferably at a molar ratio of 1.0 : 1.0 to 2.0 : 1.0, with respect to the compound represented by general formula [2a'].

Examples of an additive used in this reaction may include 1,3-dicyclohexylcarbodiimide, diethyl azodicarboxylate and triphenyl phosphine. Such an additive may be used in an amount equimolar or greater, and preferably at a molar ratio of 1.0 : 1.0 to 2.0 : 1.0, with respect to the compound represented by general formula [2a'].

This reaction may be carried out generally at 0°C to 100°C, preferably at 20°C to 60°C and for 5 minutes to 24 hours, preferably for 30 minutes to 10 hours.

[Production method 5]

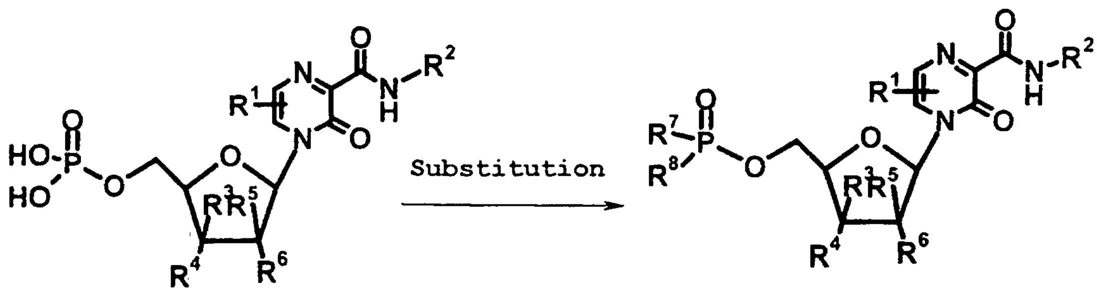


[7b] [2e]

wherein each of $R^1$, $R^2$, $R^3$, $R^4$, $R^5$, $R^6$, $R^7$ and $R^8$ has the same meaning as given above.

The compound represented by general formula [2e] or a salt thereof can be obtained by reacting the compound represented by general formula [7b] or a salt thereof with a reactive agent in the presence or absence of an additive according to the method described in e.g., the 4th Jikken Kagaku Koza, Vol. 22, pp. 371 to 424 (1992); and Journal of Medicinal Chemistry (J. Med. Chem), Vol. 38, pp. 1372 to 1379 (1995).

A solvent used in this reaction is not particularly limited as long as it does not affect the reaction. Examples of such a solvent may include: aromatic hydrocarbons such as benzene, toluene or xylene; ethers such as dioxane, tetrahydrofuran, anisole, diethylene glycol diethyl ether or dimethyl cellosolve; nitriles such as acetonitrile; amides such as N,N-dimethylformamide or N,N-dimethylacetamide; ureas such as N,N'-dimethylpropylene urea; sulfoxides such as dimethylsulfoxide; and pyridine. One or more types of these solvents may also be used in combination.

Any reactive agent commonly used in substitution reaction of phosphate groups may be used in this reaction. Examples of such a reactive agent used in this reaction may include: halogenated alkyl compounds such as pivaloyloxymethyl chloride or 1-(pivaloyloxy) ethyl chloride; alcohols and phenols such as 4-bromophenol, 4-chlorophenol, S-(2-hydroxyethyl) thiopivaloate or S-(4-hydroxybutyl) thioisobutylate; and amines such as alanine methyl ester. Such a reactive agent may be used in an amount equimolar or greater, and preferably at a molar ratio of 1.0 : 1.0 to 5.0 : 1.0, with respect to the compound represented by general formula [7b] or a salt thereof.

Examples of an additive used in this reaction may include: halogenated compounds such as phosphorus pentachloride or sodium iodide; nitrogen-containing heterocyclic compounds such as 1-methylimidazole or 1,1'-carbonyldiimidazole; azo compounds such as diethyl azodicarboxylate or diisopropyl azodicarboxylate; phosphines such as triphenyl phosphine; allene sulfonyl chlorides such as 2,4,6-triisopropyl benzenesulfonyl chloride; and bases such as triethylamine, pyridine or tert-butyl magnesium chloride. These may be used in combination. Such an additive may be used at a molar ratio of 0.01 : 1 to 10 : 1, and preferably at a molar ratio of 0.05 : 1 to 5.0 : 1, with respect to the compound represented by general formula [7b] or a salt thereof.

This reaction may be carried out generally at - 50°C to 170°C, preferably at 0°C to 100°C and for 1 minute to 72 hours, preferably for 5 minutes to 24 hours.

[Production method 6]

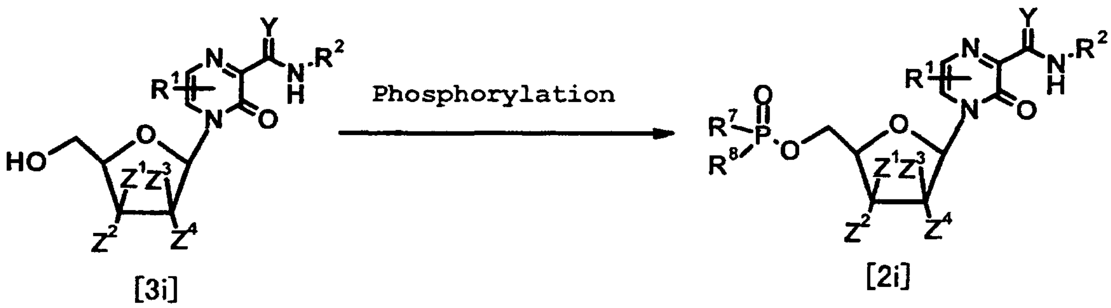


[3i]

[2i]

wherein each of $R^1$, $R^2$, $R^7$, $R^8$, $Z^1$, $Z^2$, $Z^3$, $Z^4$ and Y has the same meaning as given above.

The compound represented by general formula [2i] or a salt thereof can be obtained by performing the reaction according to the production method 1, using the compound represented by general formula [3i] or a salt thereof.

[Production method 7]

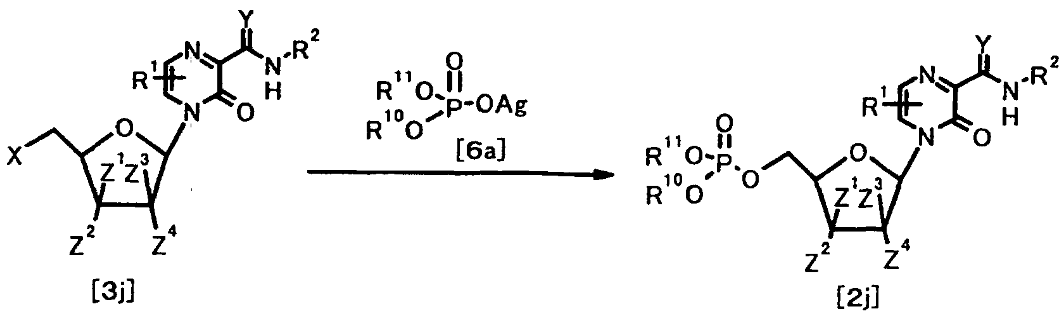

[3j]

[2j]

wherein each of $R^1$, $R^2$, $Z^1$, $Z^2$, $Z^3$, $Z^4$, $R^{10}$, $R^{11}$, X and Y has the same meaning as given above.

The compound represented by general formula [2j] or a salt thereof can be obtained by performing the reaction according to the production method 2, using the compound represented by general formula [3j] or a salt thereof.

[Production method 8]

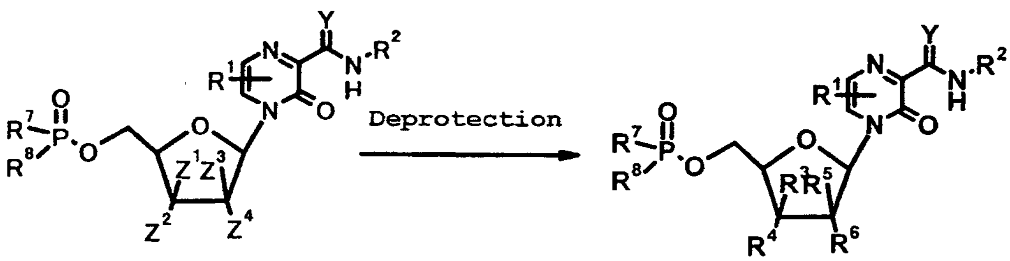


[2i]

[2k]

wherein each of $R^1$, $R^2$, $R^3$, $R^4$, $R^5$, $R^6$, $R^7$, $R^8$, $Z^1$, $Z^2$, $Z^3$, $Z^4$ and Y has the same meaning as given above.

The compound represented by general formula [2k] or a salt thereof can be obtained by performing the reaction according to the production method 3, using the compound represented by general formula [2i] or a salt thereof.

[Production method 9]

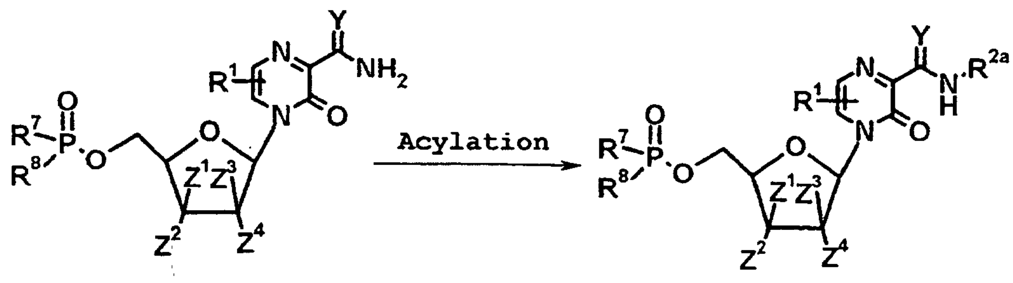


[2 l] [2m]

wherein each of $R^1$, $R^{2a}$, $R^7$, $R^8$, $Z^1$, $Z^2$, $Z^3$, $Z^4$ and Y has the same meaning as given above.

The compound represented by general formula [2m] or a salt thereof can be obtained by performing the reaction according to the production method 4, using the compound represented by general formula [21] or a salt thereof.

Next, method for producing compounds represented by general formulas [3a], [3e] and [3f], or salts thereof will be explained.

[Production method A]

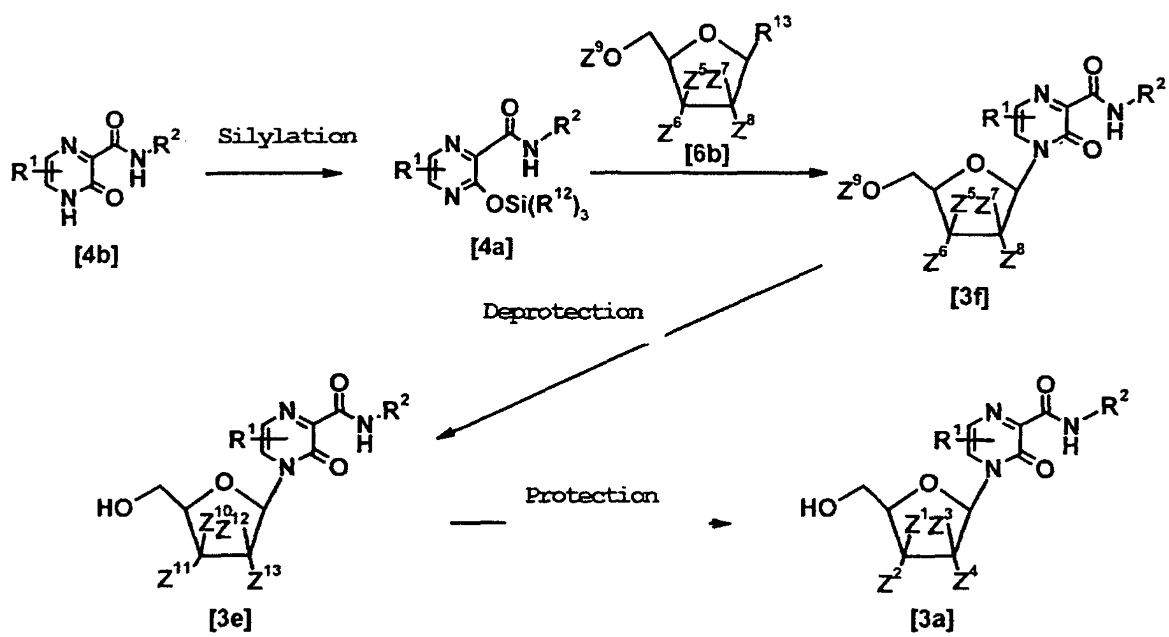


[4b] [4a] [6b] [3f] [3e] [3a]

wherein each of $R^1$, $R^2$, $Z^1$, $Z^2$, $Z^3$ and $Z^4$ has the meanings as given above; $R^{12}$ represents a lower alkyl or aryl group; $R^{13}$ represents a halogen atom, acyloxy group, alkylsulfonyloxy group or arylsulfonyloxy group; each of $Z^5$, $Z^6$, $Z^7$ and $Z^8$ identically or differently represents a hydrogen atom or protected hydroxyl group; $Z^9$ represents a hydrogen atom,

or a protecting group of a hydroxyl group; and each of $Z^{10}$, $Z^{11}$, $Z^{12}$ and $Z^{13}$ identically or differently represents a hydrogen atom or hydroxyl group.

(a) The compound represented by general formula [3f] or a salt thereof can be obtained by (1) inducing the compound represented by general formula [4b] or a salt thereof into the compound represented by general formula [4a] or a salt thereof in the presence or absence of an additive according to a commonly used silylation method, and then (2) reacting the obtained compound represented by general formula [4a] or a salt thereof with the compound represented by general formula [6b] or a salt thereof in the presence or absence of Lewis acid.

A solvent used in these reactions is not particularly limited as long as it does not affect the reaction. Examples of such a solvent may include: aromatic hydrocarbons such as benzene, toluene or xylene; ethers such as dioxane, tetrahydrofuran, anisole, diethylene glycol diethyl ether or dimethyl cellosolve; nitriles such as acetonitrile; amides such as N,N-dimethylformamide or N,N-dimethylacetamide; sulfoxides such as dimethyl sulfoxide; and halogenated hydrocarbons such as methylene chloride, chloroform or dichloroethane. One or more types of these solvents may be used in combination.

Any silylation agent may be used in the reaction of (1) above, as long as it is commonly used in conversion of a carbonyl group into silyl enol ether. Examples of such a silylation agent may include 1,1,1,3,3,3-hexamethyldisilazane, N,0-bis(trimethylsilyl) acetamide, and trimethylsilyl chloride. Such a silylation agent may be used in an amount equimolar or greater, and preferably at a molar ratio of 1.0 : 1.0 to 10.0 : 1.0, with respect to the compound represented by general formula [4b] or a salt thereof.

Ammonium sulfate is an example of the additive used in this reaction as necessary. Such an additive may be used at a molar ratio of 0.01 : 1.0 to 10.0 : 1.0, and preferably at a molar ratio of 0.05 : 1.0 to 5.0 : 1.0, with respect to the compound represented by general formula [4b] or a salt thereof.

This reaction may be carried out generally at 0°C to 200°C, preferably at 0°C to 150°C and for 5 minutes to 24 hours, preferably for 5 minutes to 12 hours.

The compound represented by general formula [6b] or a salt thereof used in the reaction of (2) above may be used at a molar ratio of 0.5 : 1 to 10 : 1, and preferably at a molar ratio of 0.5 : 1 to 5 : 1, with respect to the compound represented by general formula [4a] or a salt thereof.

Examples of Lewis acid used in this reaction as necessary may include trimethylsilyl trifluoromethane sulfonate, tin(IV) chloride, titanium(IV) chloride and zinc chloride. Such Lewis acid may be used in an amount of 0.5 mole or greater, and preferably at a molar ratio of 0.5 : 1 to 10 : 1, with respect to the compound represented by general formula [4a] or a salt thereof.

This reaction may be carried out generally at 0°C to 100°C, preferably at 0°C to 50°C and for 1 minute to 72 hours, preferably for 5 minutes to 24 hours.

(b) The compound represented by general formula [3e] or a salt thereof can be obtained by performing the reaction according to the production method 3, using the compound represented by general formula [3f] or a salt thereof.

(c) The compound represented by general formula [3a] or a salt thereof can be obtained by protecting the compound represented by general formula [3e] or a salt thereof using a reagent in the presence or absence of an acid catalyst or base.

A solvent used in this reaction is not particularly limited, as long as it does not affect the reaction. Examples of such a solvent may include: aromatic hydrocarbons such as benzene, toluene or xylene; ethers such as dioxane, tetrahydrofuran, anisole, diethylene glycol diethyl ether or dimethyl cellosolve; nitriles such as acetonitrile; amides such as N,N-dimethylacetamide; alcohols such as methanol, ethanol or propanol; sulfoxides such as dimethyl sulfoxide; ketones such as acetone; and water. One or more types of these solvents may be used in combination.

Any reagent commonly used in protection of hydroxyl groups may be used in this reaction. Preferred examples may include 2,2-dimethoxypropane, acetyl chloride and benzoyl chloride. These reagents may also be produced in the reaction system. Such a reagent may be used in an amount equimolar or greater, and preferably at a molar ratio of 1.0 : 1.0 to 10 : 1.0, with respect to the compound represented by general formula [3e] or a salt thereof.

Examples of an acid catalyst or base used in this reaction may include pyridinium paratoluenesulfonate, paratoluenesulfonic acid, and triethylamine. Such an acid catalyst or base may be used at a molar ratio of 0.01 : 1 to 10 : 1, and preferably at a molar ratio of 0.05 : 1 to 10 : 1, with respect to the compound represented by general formula [3e] or a salt thereof.

This reaction may be carried out generally at - 50°C to 170°C, preferably at 0°C to 150°C and for 1 minute to 24 hours, preferably for 5 minutes to 10 hours.

The compound represented by general formula [4b] or a salt thereof can be acquired by purchasing commercially available products, or can be produced by known methods, methods equivalent thereto, or the combined use of

them. The production methods are described in publications such as Journal of American Chemical Society (J. Am. Chem. Soc.), Vol. 71, p. 78 (1949); the same publication, Vol. 78, pp. 242 to 244 (1956); Journal of Heterocyclic Chemistry (J. Heterocycl. Chem.), Vol. 15, No. 4, pp. 665 to 670 (1978); Journal of Chemical Society (J. Chem. Soc.), p. 1379 (1955); US Patent No. 5597823; or International Patent Publication WO00/10569.

Next, a method for producing a compound represented by general formula [3b] or a salt thereof will be explained.

[Production method B]

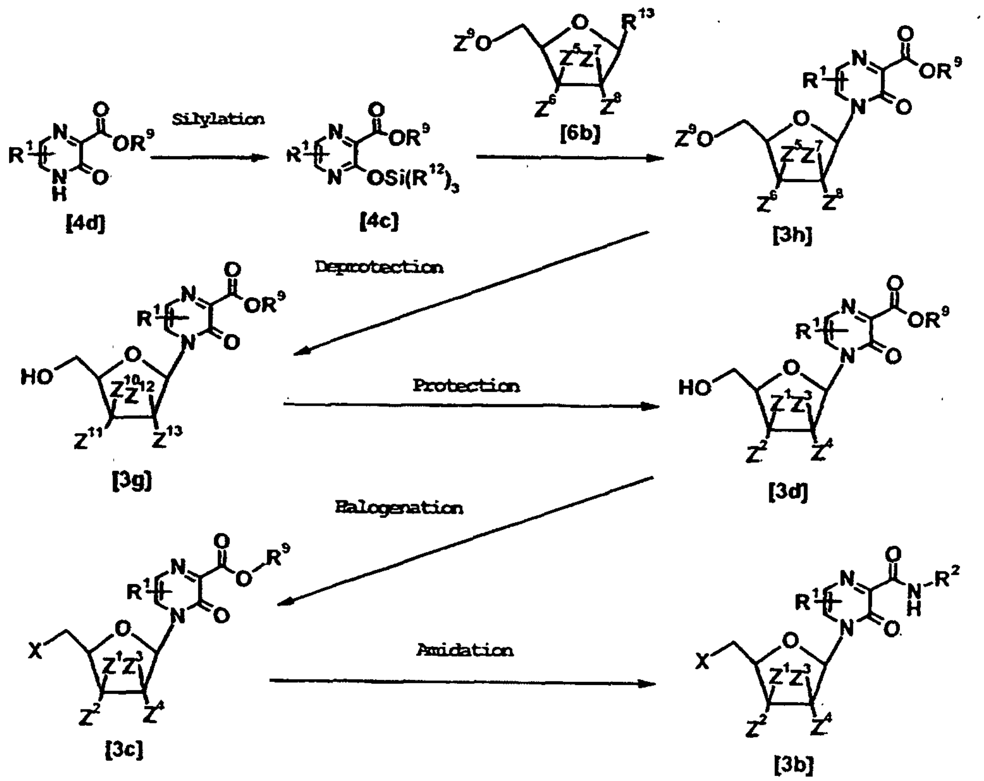


wherein $R^9$ represents an alkyl group; and each of $R^1$, $R^2$, $R^{12}$, $R^{13}$, $Z^1$, $Z^2$, $Z^3$, $Z^4$, $Z^5$, $Z^6$, $Z^7$, $Z^8$, $Z^9$, $Z^{10}$, $Z^{11}$, $Z^{12}$, $Z^{13}$ and X has the same meaning as given above.

(a) The compound represented by general formula [3h] or a salt thereof can be obtained by performing the reaction according to the production method A(a), using the compound represented by general formula [4d] or a salt thereof.
(b) The compound represented by general formula [3g] or a salt thereof can be obtained by performing the reaction according to the production method 3, using the compound represented by general formula [3h] or a salt thereof.
(c) The compound represented by general formula [3d] or a salt thereof can be obtained by performing the reaction according to the production method A(c), using the compound represented by general formula [3g] or a salt thereof.
(d) The compound represented by general formula [3c] or a salt thereof can be obtained, according to the method described in e.g., the 4th Jikken Kagaku Koza, Vol. 19, pp. 416 to 482 (1992), (1) by reacting the compound represented by general formula [3d] or a salt thereof with a halogenating agent in the presence or absence of an additive, or (2) by reacting the same compound or a salt thereof with a sulfonating agent in the presence of an deacidification agent and then reacting with a halogenating agent.

In the method according to (1) above, a solvent used in this reaction is not particularly limited as long as it does not affect the reaction. Examples of such a solvent may include: aromatic hydrocarbons such as benzene, toluene or xylene; halogenated hydrocarbons such as methylene chloride, chloroform; ethers such as dioxane, tetrahydrofuran, anisole, diethylene glycol diethyl ether or dimethyl cellosolve; nitriles such as acetonitrile; amides such as N,N-dimethylformamide or N,N-dimethylacetamide; alcohols such as methanol, ethanol or propanol; sulfoxides such as dimethyl sulfoxide; and water. One or more types of these solvents may be used in combination.

A halogenating agent used in this reaction is not particularly limited as long as it is a reagent commonly used in halogenation reaction of hydroxyl groups. Preferred examples of such a halogenating agent may include iodine, bromine, chlorine, hydriodic acid, hydrobromic acid, sodium bromide, potassium iodide, sulfuryl chloride, N-bromosuccinimide, N-chlorosuccinimide, carbon tetrabromide, or phosphorus compounds such as triphenyl iodine phosphonate. Such a halogenating agent may be used at a molar ratio of 1 : 1 to 50 : 1, and preferably at a molar ratio of 1 : 1 to 20 : 1, with respect to the compound represented by general formula [3d] or a salt thereof.

An additive used in this reaction as necessary is not particularly limited as long as it is a reagent commonly used in halogenation reaction of hydroxyl groups. Preferred examples of such an additive may include: phosphines such as triphenylphosphine; azo compounds such as diethyl azodicarboxylate; and silanes such as trimethyl silyl chloride or hexamethyldisiloxane. One or more types of these additives may be used in combination. Such an additive may be used at a molar ratio of 0.01 : 1 to 10 : 1, and preferably at a molar ratio of 0.1 : 1 to 10 : 1, with respect to the compound represented by general formula [3d] or a salt thereof.

This reaction may be carried out generally at - 80°C to 170°C, preferably at -80°C to 100°C and for 1 minute to 72 hours, preferably for 5 minutes to 48 hours.

In the method according to (2) above, a solvent used in the sulfonation reaction is not particularly limited as long as it does not affect the reaction. Examples of such a solvent may include: aromatic hydrocarbons such as benzene, toluene or xylene; halogenated hydrocarbons such as methylene chloride or chloroform; ethers such as dioxane, tetrahydrofuran, anisole, diethylene glycol diethyl ether or dimethyl cellosolve; nitriles such as acetonitrile; amides such as N,N-dimethylformamide or N,N-dimethylacetamide; alcohols such as methanol, ethanol or propanol; sulfoxides such as dimethyl sulfoxide; and water. One or more types of these solvents may be used in combination.

A sulfonating agent used in this reaction is not particularly limited as long as it is a reagent commonly used in sulfonation reaction of hydroxyl groups. Preferred examples of such a sulfonating agent may include halogenated sulfonyls such as methanesulfonyl chloride or p-toluenesulfonyl chloride; sulfonic acid anhydrides such as trifluoromethanesulfonic acid anhydride; and sulfonic acids such as trifluoromethanesulfonic acid. Such a sulfonating agent may be used at a molar ratio of 1 : 1 to 20 : 1, and preferably at a molar ratio of 1.0 : 1.0 to 5.0 : 1.0, with respect to the compound represented by general formula [3d] or a salt thereof.

A deacidification agent used in this reaction as necessary is not particularly limited as long as it is a reagent commonly used in sulfonation reaction of hydroxyl groups. Preferred examples of such a deacidification agent may include bases such as pyridine, 2,6-lutidine, triethylamine or sodium methoxide, and one or more types of these deacidification agents may be used in combination. Such a deacidification agent may be used at a molar ratio of 0.01 : 1 to 20 : 1, and preferably at a molar ratio of 0.1 : 1 to 10 : 1, with respect to the compound represented by general formula [3d] or a salt thereof.

This reaction may be carried out generally at -80°C to 100°C, preferably at -20°C to 50°C and for 1 minute to 24 hours, preferably for 5 minutes to 12 hours.

A solvent used in halogenation reaction is not particularly limited as long as it does not affect the reaction. Examples of such a solvent may include: aromatic hydrocarbons such as benzene, toluene or xylene; halogenated hydrocarbons such as methylene chloride or chloroform; ethers such as dioxane, tetrahydrofuran, anisole, diethylene glycol diethyl ether or dimethyl cellosolve; nitriles such as acetonitrile; amides such as N,N-dimethylformamide or N, N-dimethylacetamide; ketones such as acetone; alcohols such as methanol, ethanol or propanol; sulfoxides such as dimethyl sulfoxide; and water. One or more types of these solvents may be used in combination.

A halogenating agent used in this reaction is not particularly limited as long as it is a reagent commonly used in halogenation reaction of sulfonate groups. Preferred examples of such a halogenating agent may include sodium bromide, sodium iodide, potassium iodide and magnesium iodide. Such a halogenating agent may be used at a molar ratio of 1 : 1 to 50 : 1, and preferably at a molar ratio of 1 : 1 to 20 : 1, with respect to the compound represented by general formula [3d] or a salt thereof.

This reaction may be carried out generally at - 80°C to 170°C, preferably at -80°C to 100°C and for 1 minute to 72 hours, preferably for 5 minutes to 12 hours.

(e) The compound represented by general formula [3b] or a salt thereof can be obtained by subjecting the compound represented by general formula [3c] or a salt thereof to ammonolysis reaction of carboxylate in the presence or absence of a catalyst.

A solvent used in this reaction is not particularly limited as long as it does not affect the reaction. Examples of such a solvent may include: aromatic hydrocarbons such as benzene, toluene or xylene; halogenated hydrocarbons such as methylene chloride or chloroform; ethers such as dioxane, tetrahydrofuran, anisole, diethylene glycol diethyl ether or dimethyl cellosolve; nitriles such as acetonitrile; amides such as N,N-dimethylformamide or N,N-dimethylacetamide; alcohols such as methanol, ethanol or propanol; sulfoxides such as dimethyl sulfoxide; and water. One or more types of these solvents may be used in combination. This reaction may be carried out, using a reagent and conditions that are commonly used in ammonolysis reaction of aromatic carboxylate. Ammonia gas, liquid ammonia or ammonia water may be preferably used. Such a reagent may be used in an amount equimolar or greater with respect to the compound represented by general formula [3c] or a salt thereof. In addition, the reagent may also be used as a solvent. Examples of a catalyst used in this reaction as necessary may include: acid ammonium salts such as ammonium chloride; bases such as sodium methoxide or butyllithium; and alkali metal amide such as sodium amide. Such a catalyst may be used at a molar ratio of 0.01 : 1 to 100 : 1, and preferably at a molar ratio of 0.01 : 1 to 20 : 1, with respect to the compound represented by general formula [3c] or a salt thereof.

This reaction may be carried out generally at -100°C to 250°C, preferably at -78°C to 100°C and for 1 minute to 72 hours, preferably for 30 minutes to 50 hours.

The compound represented by general formula [4d] or a salt thereof can be acquired by purchasing commercially available products, or can be produced by known methods, methods equivalent thereto, or the combined use of them. The publication as described above for the production method of the compound represented by general formula [4b] is an example of publications describing the production methods of the compound represented by general formula [4d] or a salt thereof.

Next, a method for producing a compound represented by general formula [7b] or a salt thereof will be explained.

[Production method C]

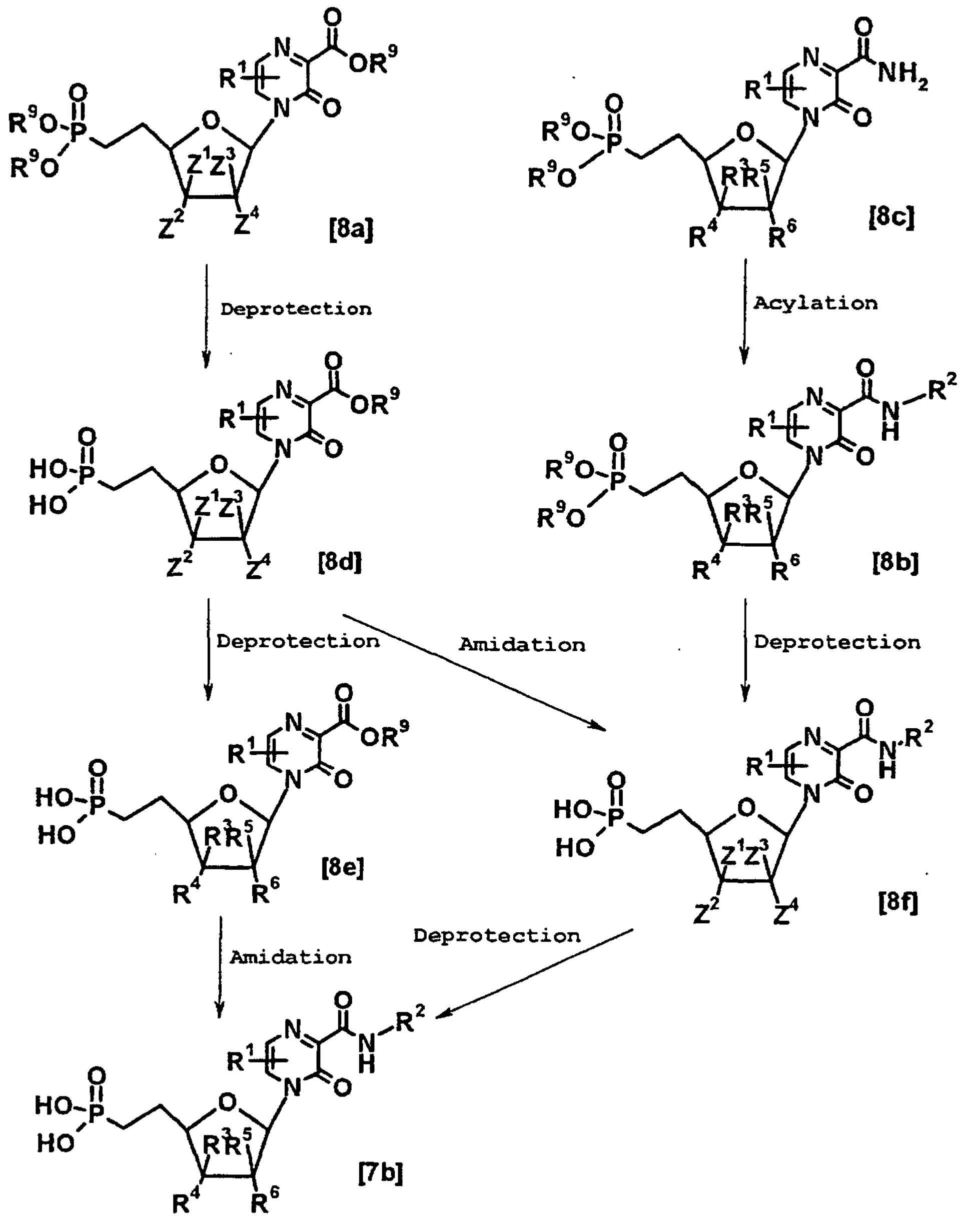


wherein each of $R^1$, $R^2$, $R^3$, $R^4$, $R^5$, $R^6$, $R^9$, $Z^1$, $Z^2$, $Z^3$ and $Z^4$ has the same meaning as given above.

(a) The compound represented by general formula [8d] or a salt thereof can be obtained by performing the reaction according to the production method 3, using the compound represented by general formula [8a] or a salt thereof.
(b) The compound represented by general formula [7b] or a salt thereof can be obtained: (1) by deprotecting the compound represented by general formula [8d] or a salt thereof according to the production method 3, and then amidating the obtained product according to the production method B(e); or (2) by amidating the above compound or a salt thereof according to the production method B(e), and then deprotecting the obtained product according to the production method 3.
(c) The compound represented by general formula [8b] or a salt thereof can be obtained by performing the reaction according to the production method 4, using the compound represented by general formula [8c] or a salt thereof.
(d) The compound represented by general formula [8f] or a salt thereof can be obtained by deprotecting the compound represented by general formula [8b] according to the production method 3.

Next, a method for producing compounds represented by general formulas [8a] and [8b'] or salts thereof will be explained.

[Production method D]

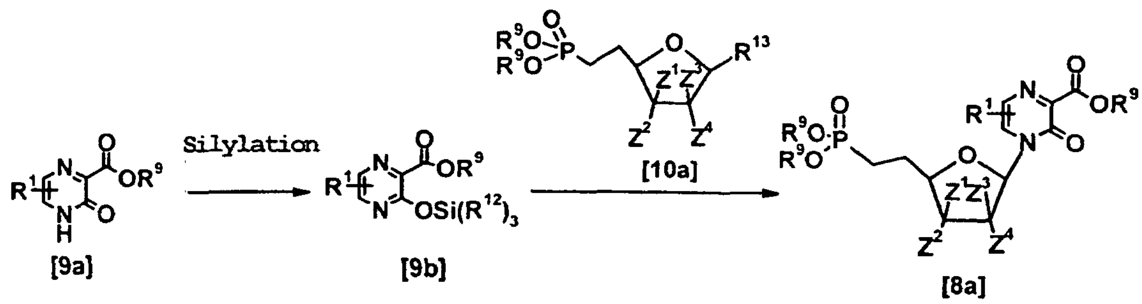


[9a] [9b] [8a]

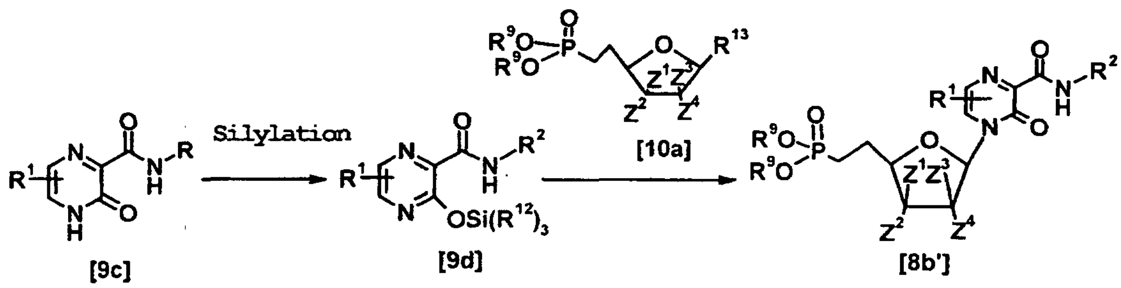


[9c] [9d] [8b']

wherein each of $R^1$, $R^2$, $R^9$, $R^{12}$, $R^{13}$, $Z^1$, $Z^2$, $Z^3$ and $Z^4$ has the same meaning as given above.

(a) The compound represented by general formula [8a] or a salt thereof can be obtained by reacting the compound represented by general formula [9a] or a salt thereof with the compound represented by general formula [10a] or a salt thereof according to the production method B(a).
(b) The compound represented by general formula [8b'] or a salt thereof can be obtained by reacting the compound represented by general formula

[9c] or a salt thereof with the compound represented by general formula [10a] or a salt thereof according to the production method B(a).

Next, a method for producing a compound represented by general formula [10a] or a salt thereof will be explained.

[Production method E]

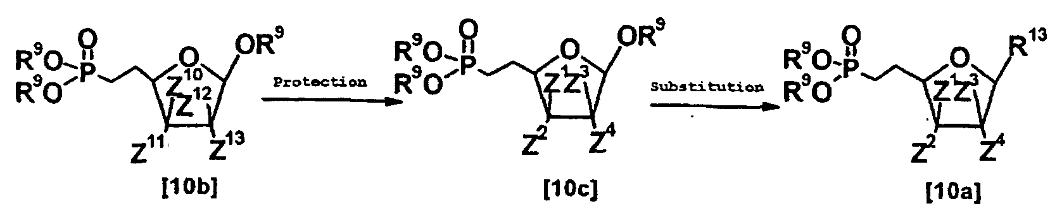


[10b] [10c] [10a]

wherein each of $R^9$, $R^{13}$, $Z^1$, $Z^2$, $Z^3$, $Z^4$, $Z^{10}$, $Z^{11}$, $Z^{12}$ and $Z^{13}$ has the same meaning as given above.

(a) The compound represented by general formula [10c] or a salt thereof can be obtained by performing the reaction according to the production method A(c), using the compound represented by general formula [10b] or a salt thereof.

(b) The compound represented by general formula [10a] or a salt thereof can be obtained by subjecting the compound represented by general formula [10c] or a salt thereof to a substitution reaction according to the method described in e.g., Journal of Organic Chemistry (J. Org. Chem.) Vol. 55, pp. 3853 to 3857 (1990).

A solvent used in this reaction is not particularly limited as long as it does not affect the reaction. Examples of such a solvent may include: aromatic hydrocarbons such as benzene, toluene or xylene; halogenated hydrocarbons such as methylene chloride or chloroform; ethers such as dioxane, tetrahydrofuran, anisole, diethylene glycol diethyl ether or dimethyl cellosolve; nitriles such as acetonitrile; amides such as N,N-dimethylacetamide; alcohols such as methanol, ethanol or propanol; sulfoxides such as dimethyl sulfoxide; and water. One or more types of these solvents may be used in combination.

A reactive agent used in this reaction may be one commonly used in a substitution reaction on lactol under acidic conditions. Examples of such a reactive agent may include: organic acids and acid anhydrides such as acetic acid or acetic acid anhydride; inorganic acids such as hydrogen chloride gas, hydrochloric acid, hydrobromic acid, sulfuric acid or hydrofluoric acid; halogen compounds such as chlorine or bromine; Lewis acids such as titanium tetrabromide or chlorotrimethyl silane; and thio compounds such as thiophenol or methylthiotrimethyl silane. Such a reactive agent may be used at a molar ratio of 1 : 1 to 20 : 1, and preferably at a molar ratio of 1 : 1 to 10 : 1, with respect to the compound represented by general formula [10c] or a salt thereof and may be employed as a solvent.

This reaction may be carried out generally at 0°C to 200°C, preferably at 0°C to 100°C and for 5 minutes to 48 hours, preferably for 30 minutes to 24 hours.

The compound represented by general formula [10b] can be obtained according to the method described in Journal of Chemical Society Chemical Communication (J. Chem. Soc., Chem. Commun.) pp. 40 to 41 (1989).

Next, a method for chemically synthesizing the pyrazine nucleotide analog represented by general formula [1] in which A is an oxygen atom will be explained.

[Production method F]

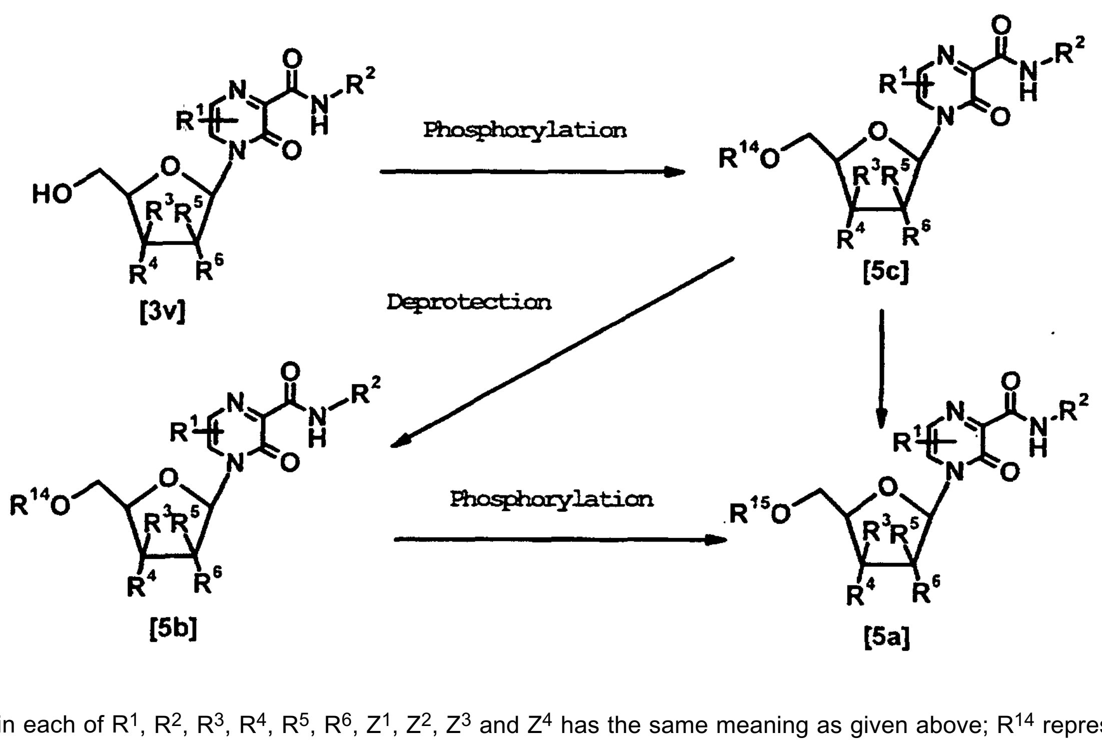


[3v] [5c] [5b] [5a]

wherein each of $R^1$, $R^2$, $R^3$, $R^4$, $R^5$, $R^6$, $Z^1$, $Z^2$, $Z^3$ and $Z^4$ has the same meaning as given above; $R^{14}$ represents a monophosphate group or monophosphoric chloride that may be protected; and $R^{15}$ represents a diphosphate acid or

triphosphate group that may be protected.

(a) The compound represented by general formula [5c] or a salt thereof can be obtained by performing the reaction according to the production method 1, using the compound represented by general formula [3v] or a salt thereof.
(b) The compound represented by general formula [5b] or a salt thereof can be obtained by performing the reaction according to the production method 3, using the compound represented by general formula [5c] or a salt thereof.
(c) The compound represented by general formula [5a] or a salt thereof can be obtained by reacting the compound represented by general formula [5b] or a salt thereof with a phosphorylation agent in the presence or absence of a condensation agent according to the methods described in e.g., Chemical Review (Chem. Rev.), Vol. 100, pp. 2047 to 2059 (2000); Journal of Organic Chemistry (J. Org. Chem.), Vol. 55, pp. 1834 to 1841 (1990); or Acta Biochimica Biophysica Academia Scientiarum Hungaricae (Acta Biochim. et Biophys. Acad. Sci. Hung.), Vol. 16, pp. 131 to 133 (1981).

A solvent used in this reaction is not particularly limited as long as it does not affect the reaction. Examples of such a solvent may include: aromatic hydrocarbons such as benzene, toluene or xylene; ethers such as dioxane, tetrahydrofuran, anisole, diethylene glycol diethyl ether or dimethyl cellosolve; nitriles such as acetonitrile; amides such as N,N-dimethylformamide or N,N-dimethylacetamide; sulfoxides such as dimethylsulfoxide; phosphoric esters such as trimethyl phosphate; and pyridine. One or more types of these solvents may be used in combination.
A phosphorylation agent used in this reaction may be one commonly used in phosphorylation of monophosphate groups. Examples of such a phosphorylation agent may include phosphates such as tri-n-butyl ammonium phosphate or tri-n-butyl ammonium pyrophosphate. These agents may also be produced in the reaction system. Such a phosphorylation agent may be used in an amount equimolar or greater, and preferably at a molar ratio of 1 : 1 to 10 : 1, with respect to the compound represented by general formula [5b] or a salt thereof. Examples of a condensation agent may include imidazoles such as 1,1'-carbonyldiimidazole or N-methylimidazole, and amines such as morpholine or diisopropylamine. These may also be used in combination. Such a condensation agent may be used in an amount equimolar or greater, and preferably at a molar ratio of 1 : 1 to 5 : 1, with respect to the compound represented by general formula [5b] or a salt thereof.
This reaction may be carried out generally at -50°C to 100°C, preferably at 0°C to 50°C and for 1 minute to 72 hours, preferably for 5 minutes to 24 hours.

(d) The compound represented by general formula [5a] or a salt thereof can be obtained by performing the reaction according to the production method F(c), using the compound represented by general formula [5c] or a salt thereof.
(e) The compound represented by general formula [5a] or a salt thereof can be obtained by reacting the compound represented by general formula

[3v] or a salt thereof according to the production method 1, so as to induce it into the compound represented by general formula [5c] or a salt thereof, and then reacting the obtained compound or a salt thereof in the same system according to the production method F(d).

[Production method G]

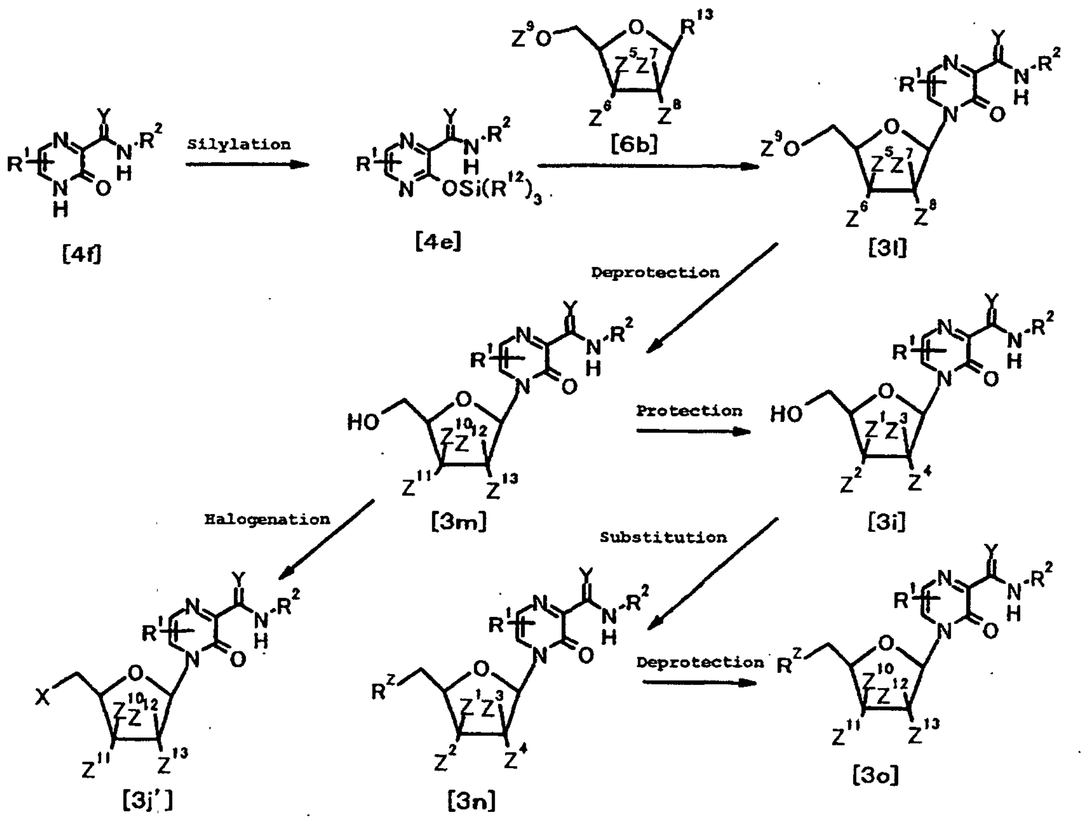


wherein each of $R^1$, $R^2$, $R^{12}$, $R^{13}$, $R^Z$, $Z^1$, $Z^2$, $Z^3$, $Z^4$, $Z^5$, $Z^6$, $Z^7$, $Z^8$, $Z^9$, $Z^{10}$, $Z^{11}$, $Z^{12}$, $Z^{13}$ and Y has the same meaning as given above.

(a) The compounds represented by general formulas [3l], [3m] and [3i] or salts thereof can be obtained by performing the reaction according to the production method A, using the compound represented by general formula [4f] or a salt thereof.

(b) The compound represented by general formula [3n] or a salt thereof can be obtained by performing the reaction according to the production method 4, using the compound represented by general formula [3i] or a salt thereof; or it can be obtained by subjecting the compound represented by general formula [3i] or a salt thereof to an alkylation reaction in the presence or absence of acid or base according to the method described in e.g., Shin Jikken Kagaku Koza, Vol. 14, pp. 567 to 587 (edited by The Chemical Society of Japan, 1977).

A solvent used in this reaction is not particularly limited as long as it does not affect the reaction. Examples of such a solvent may include: ethers such as dioxane, tetrahydrofuran, anisole, diethylene glycol diethyl ether or dimethyl cellosolve; aromatic hydrocarbons such as benzene, toluene or xylene; halogenated hydrocarbons such as dichloromethane, chloroform or dichloroethane; amides such as N,N-dimethylformamide or N,N-dimethylacetamide; sulfoxides such as dimethylsulfoxide; and water. These solvents may be used in combination.

Examples of an alkylating agent used in this reaction may include: halogenated alkyls such as benzyl bromide; esters such as diethyl sulfate; diazo compounds such as diphenyldiazomethane; olefins such as 2-methylpropene; and amide acetals such as N,N-dimethylacetamide dimethylacetal. Such an alkylating agent may be used in an amount equimolar or greater, and preferably at a molar ratio of 1.0 : 1.0 to 2.0 : 1.0, with respect to the compound represented by general formula [3i].

Examples of acid used in this reaction may include p-toluenesulfonic acid and sulfuric acid. Examples of base used in this reaction may include triethylamine, sodium methoxide, sodium hydride, potassium tert-butoxide, potassium carbonate and metallic sodium. Such acid or base may be used in an amount equimolar or greater, and preferably at

a molar ratio of 1.0 : 1.0 to 2.0 : 1.0, with respect to the compound represented by general formula [3i].

This reaction may be carried out generally at 0°C to 100°C, preferably at 20°C to 60°C and for 5 minutes to 24 hours, preferably for 30 minutes to 10 hours.

(c) The compound represented by general formula [3o] or a salt thereof can be obtained by performing the reaction according to the production method 3, using the compound represented by general formula [3n] or a salt thereof.
(d) The compound represented by general formula [3j'] or a salt thereof can be obtained by performing the reaction according to the production method B(d), using the compound represented by general formula [3m] or a salt thereof.

[Production method H]

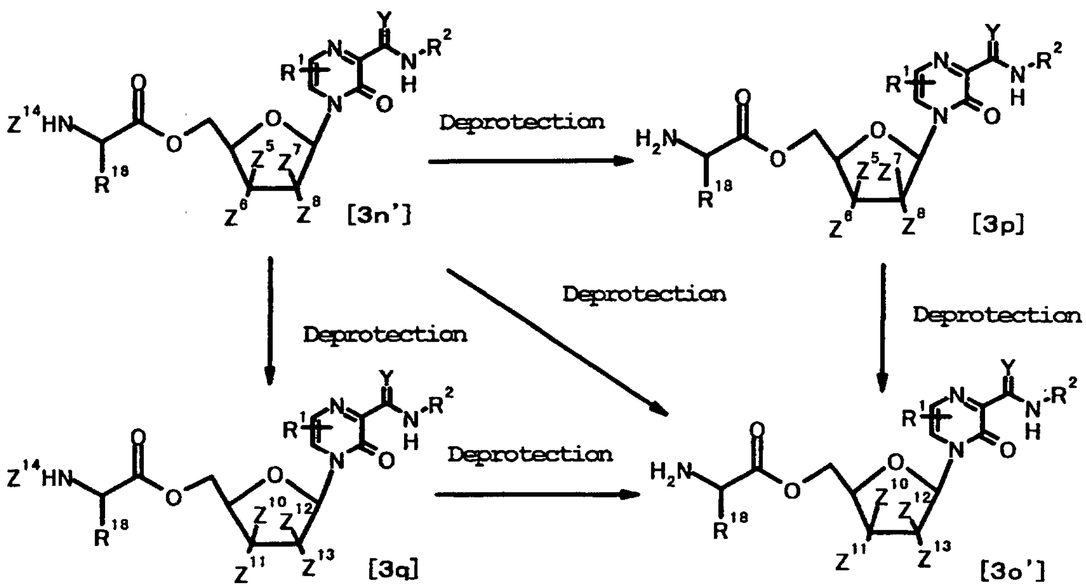


wherein each of $R^1$, $R^2$, $Z^5$, $Z^6$, $Z^7$, $Z^8$, $Z^{10}$, $Z^{11}$, $Z^{12}$, $Z^{13}$ and Y has the same meaning as given above; $R^{14}$ represents a protecting group of an amino group; and $R^{18}$ represents an amino acid residue that may be protected.

(a) The compound represented by general formula [3p] or a salt thereof can be obtained by reacting the compound represented by general formula [3n'] or a salt thereof with a deprotecting agent in the presence or absence of a catalyst according to common methods such as one described in Protective Groups in Organic Synthesis, Third Edition, Theodora W. Greene, pp. 494 to 653 (1999).

A solvent used in this reaction is not particularly limited as long as it does not affect the reaction. Examples of such a solvent may include: water; alcohols such as methanol, ethanol or propanol; thioalcohols such as ethanethiol or thiophenol; aromatic hydrocarbons such as benzene, toluene or xylene; halogenated hydrocarbons such as methylene chloride, chloroform or 1,2-dichloroethane; ethers such as dioxane, tetrahydrofuran, anisole, diethylene glycol diethyl ether or dimethyl cellosolve; thioethers such as dimethyl sulfide; ketones such as acetone or methyl ethyl ketone; nitriles such as acetonitrile; amides such as N,N-dimethylformamide or N,N-dimethylacetamide; sulfoxides such as dimethylsulfoxide; inorganic acids such as sulfuric acid or hydrochloric acid; carboxylic acids such as acetic acid or trifluoroacetic acid; sulfonic acids such as trifluoromethanesulfonic acid; nitroalkanes such as nitromethane; and organic bases such as pyridine or triethylamine. One or more types of these solvents may be used in combination.

A deprotecting agent used in this reaction is not particularly limited, and those commonly used in deprotection of protected amino groups may be used herein. Preferred examples of such a deprotecting agent may include: hydrogen gas; ammonium formate; zinc; sodium; acid chlorides such as vinylchloroformate or acetyl chloride; organic silanes such as triethylsilane or trimethylsilyliodide; tributyltin hydride; alkali metal alkoxide such as potassium tert-butoxide; alkali metal thioalkoxide such as sodium thiomethoxide; 2,3-dichloro-5,6-dicyano-1,4-benzoquinone; sodium borohydride; alkali metal salts such as potassium fluoride or sodium iodide; Lewis acids such as boron tribromide, aluminum chloride, ruthenium chloride or zinc chloride; inorganic acids such as hydrochloric acid, hydrobromic acid or sulfuric

acid; organic acids such as trifluoroacetic acid, methanesulfonic acid or paratoluenesulfonic acid; inorganic bases such as potassium carbonate, sodium bicarbonate or sodium hydroxide; organic bases such as piperidine; amines such as ammonia or hydrazine; organic lithium such as methyllithium; cerium diammonium nitrate; and peroxides such as hydrogen peroxide, ozone or permanganic acid. Such a deprotecting agent may be used at a molar ratio of 0.01 : 1 to 1000 : 1, and preferably 0.1 : 1 to 100 : 1, with respect to the compound represented by general formula [3n'] or a salt thereof.

A catalyst used in this reaction as necessary is not particularly limited, as long as it is commonly used in deprotection of protected amino groups. Preferred examples of such a catalyst may include: palladium catalysts such as palladium-carbon; rhodium; Raney nickel; and platinum oxide (IV). A catalyst such as palladium-carbon or Raney nickel may be used at a weight ratio of 0.01 : 1 to 10 : 1, and more preferably of 0.01 : 1 to 5 : 1, with respect to the compound represented by general formula [3n'] or a salt thereof. Catalysts other than palladium-carbon and Raney nickel may be used at a molar ratio of 0.01 : 1 to 10 : 1, and preferably of 0.01 : 1 to 5 : 1, with respect to the compound represented by general formula [3n'] or a salt thereof.

This reaction may be carried out generally at - 80°C to 200°C, preferably at 0°C to 160°C and for 1 minute to 48 hours, preferably for 5 minutes to 12 hours.

(b) The compound represented by general formula [3o'] or a salt thereof can be obtained by performing the reaction according to the production method 3, using the compound represented by general formula [3p] or a salt thereof.
(c) The compound represented by general formula [3q] or a salt thereof can be obtained by performing the reaction according to the production method 3, using the compound represented by general formula [3n'] or a salt thereof.
(d) The compound represented by general formula [3o'] or a salt thereof can be obtained by performing the reaction according to the production method H(a), using the compound represented by general formula [3q] or a salt thereof.
(e) The compound represented by general formula [3o'] or a salt thereof can be obtained by performing the reaction according to the production method 3 or the production method H(a), using the compound represented by general formula [3n'] or a salt thereof.

[Production method I]

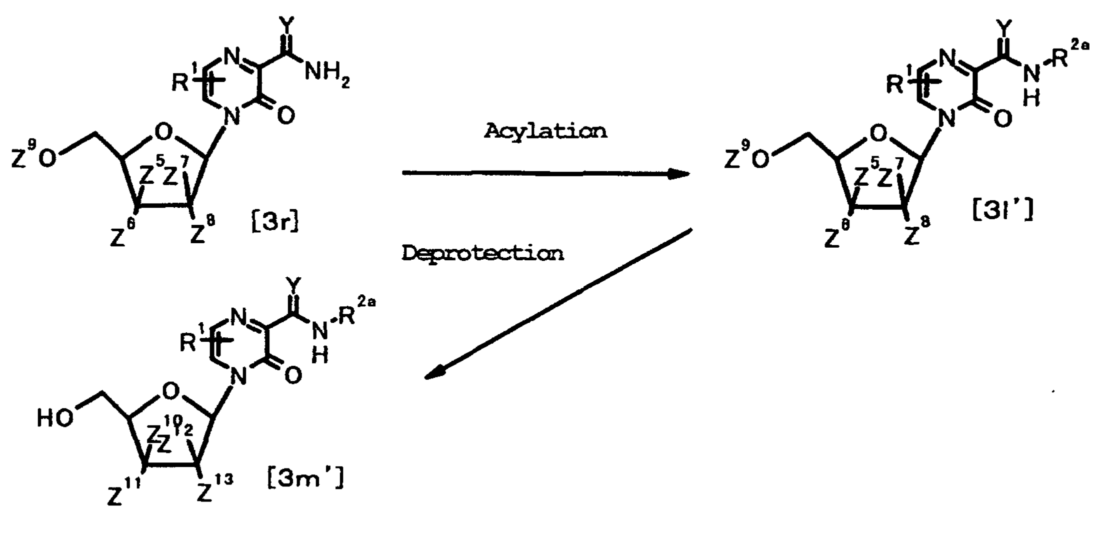


wherein each of $R^1$, $R^{2a}$, $Z^5$, $Z^6$, $Z^7$, $Z^8$, $Z^9$, $Z^{10}$, $Z^{11}$, $Z^{12}$, $Z^{13}$ and Y has the same meaning as given above.

(a) The compound represented by general formula [3l'] or a salt thereof can be obtained by performing the reaction according to the production method 4, using the compound represented by general formula [3r] or a salt thereof.
(b) The compound represented by general formula [3m'] or a salt thereof can be obtained by performing the reaction according to the production method 3, using the compound represented by general formula [3l'] or a salt thereof.

[Production method J]

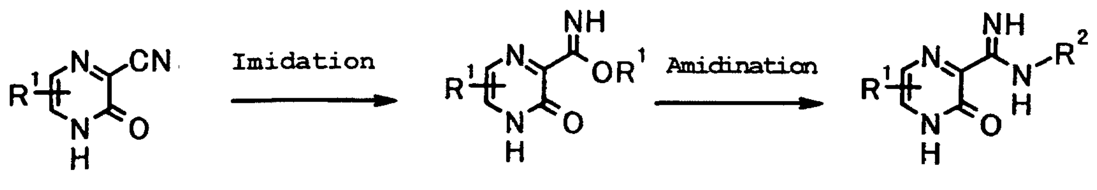


[4g] [4h] [4f']

wherein each of $R^1$, $R^2$ and $R^{12}$ has the same meaning as given above.

(a) The compound represented by general formula [4h] or a salt thereof can be obtained by reacting the compound represented by general formula [4g] or a salt thereof with alcohol in the presence or absence of an acid catalyst or base according to the method described in e.g., Shin Jikken Kagaku Koza, Vol. 14, pp. 1599 to 1602 (1978).

A solvent used in this reaction is not particularly limited as long as it does not affect the reaction. Examples of such a solvent may include: aromatic hydrocarbons such as benzene, toluene or xylene; halogenated hydrocarbons such as methylene chloride, chloroform or dichloroethane; ethers such as dioxane, tetrahydrofuran, anisole, diethylene glycol diethyl ether or dimethyl cellosolve; amides such as N,N-dimethylformamide or N,N-dimethylacetamide; and sulfoxides such as dimethylsulfoxide. One or more types of these solvents may be used in combination.
Examples of alcohol used in this reaction may include methanol, ethanol and phenol. Such alcohol may be used in an amount equimolar or greater with respect to the compound represented by general formula [4g] or a salt thereof. Moreover, alcohol may also be used as a solvent.
A reagent commonly used in imidation of nitrile may be used as an acid catalyst used in this reaction. Hydrogen chloride is an example of such an acid catalyst. Such an acid catalyst may be used at a molar ratio of 0.1 : 1 or more with respect to the compound represented by general formula [4g] or a salt thereof.
Examples of a base used in this reaction may include metal alkoxides such as sodium methoxide, sodium ethoxide or sodium phenoxide. These bases may also be produced in the reaction system. Such a base may be used in this reaction at a molar ratio of 0.01 : 1 or more, and preferably at a molar ratio of 1.0 : 1.0 to 5.0 : 1.0, with respect to the compound represented by general formula [4g] or a salt thereof.
This reaction may be carried out generally at -78°C to 170°C, preferably at -40°C to 120°C and for 1 minute to 72 hours, preferably for 5 minutes to 24 hours.

(b) The compound represented by general formula [4f'] or a salt thereof can be obtained by reacting the compound represented by general formula [4h] or a salt thereof with a reagent according to the method described in e.g., Shin Jikken Kagaku Koza, Vol. 14, pp. 1614 to 1617 (1978).

A solvent used in this reaction is not particularly limited as long as it does not affect the reaction. Examples of such a solvent may include: aromatic hydrocarbons such as benzene, toluene or xylene; halogenated hydrocarbons such as methylene chloride, chloroform or dichloroethane; ethers such as dioxane, tetrahydrofuran, anisole, diethylene glycol diethyl ether or dimethyl cellosolve; amides such as N,N-dimethylformamide or N,N-dimethylacetamide; and sulfoxides such as dimethylsulfoxide. One or more types of these solvents may be used in combination.
A reagent commonly used in amidination of imidates may be used in this reaction. Examples of such a reagent may include: ammonia gas, ammonia alcohol solution, ammonia water, or acid ammonium salts such as ammonium chloride; and amino acids that may be protected, such as glycine ethyl ester, or salts thereof. Such a reagent may be used in this reaction in an amount equimolar or greater with respect to the compound represented by general formula [4h] or a salt thereof, and it may also be used as a solvent.
This reaction may be carried out generally at -78°C to 170°C, preferably at 0°C to 120°C and for 1 minute to 72 hours, preferably for 5 minutes to 24 hours.

[Production method K]

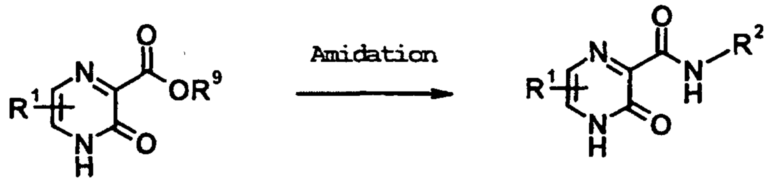


[4i]

[4b]

wherein each of $R^1$, $R^2$ and $R^9$ has the same meaning as given above.

The compound represented by general formula [4b] or a salt thereof can be obtained by subjecting the compound represented by general formula [4i] or a salt thereof to a condensation reaction with carboxylate and amines such as ammonia or a primary amine in the presence or absence of a catalyst.

A solvent used in this reaction is not particularly limited as long as it does not affect the reaction. Examples of such a solvent may include: aromatic hydrocarbons such as benzene, toluene or xylene; halogenated hydrocarbons such as methylene chloride or chloroform; ethers such as dioxane, tetrahydrofuran, anisole, diethylene glycol diethyl ether or dimethyl cellosolve; nitriles such as acetonitrile; amides such as N,N-dimethylformamide or N,N-dimethylacetamide; alcohols such as methanol, ethanol or propanol; sulfoxides such as dimethylsulfoxide; and water. One or more types of these solvents may be used in combination. This reaction may be carried out, using a reagent and conditions that are commonly used in a condensation reaction with aromatic carboxylate and amines. Examples of amines preferably used herein may include ammonia such as ammonia gas, liquid ammonia or ammonia water, and primary amines such as L-aspartic acid diethyl ester. Such amine may be used in an amount equimolar or greater with respect to the compound represented by general formula [4i] or a salt thereof. These reagents may also be used as solvents. Examples of a catalyst used in this reaction as necessary may include: acid ammonium salts such as ammonium chloride; bases such as triethylamine, sodium methoxide or butyllithium; and alkali metal amides such as sodium amide. Such a catalyst may be used at a molar ratio of 0.01 : 1 to 100 : 1, and preferably at a molar ratio of 0.01 : 1 to 20 : 1, with respect to the compound represented by general formula [4i] or a salt thereof.

This reaction may be carried out generally at - 100°C to 250°C, preferably at -78°C to 100°C and for 1 minute to 72 hours, preferably for 30 minutes to 50 hours.

[Production method L]

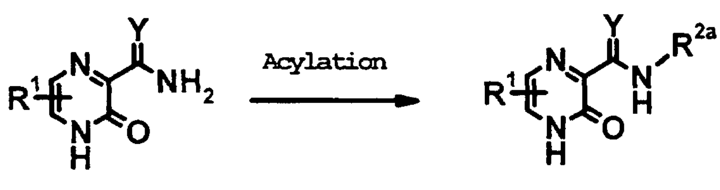


[4j]

[4f"]

wherein each of $R^1$, $R^{2a}$ and Y has the same meaning as given above.

The compound represented by general formula [4f"] or a salt thereof can be obtained by performing the reaction according to the production method 4, using the compound represented by general formula [4j] or a salt thereof.

[Production method M]

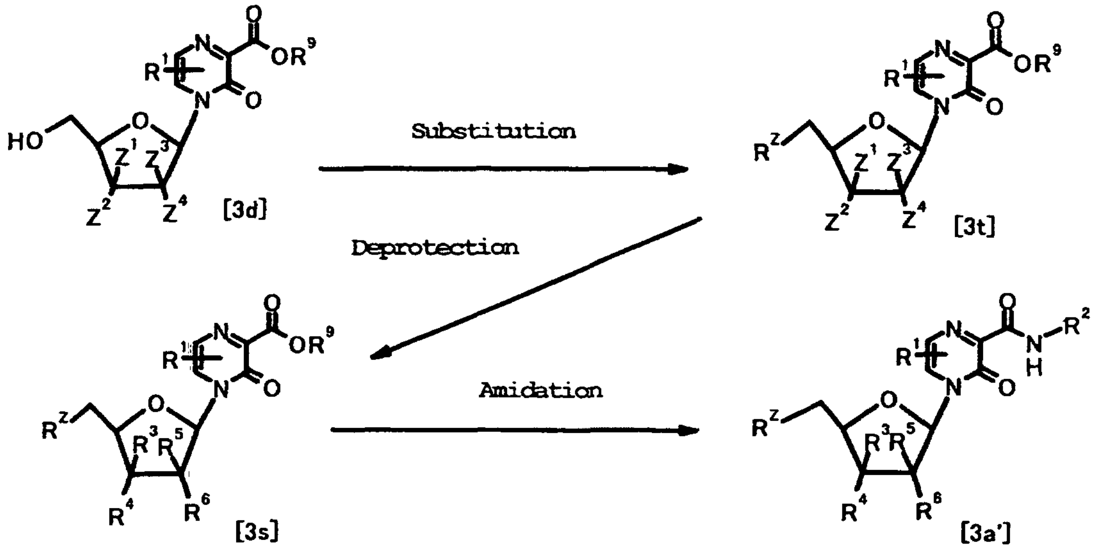


[3d] [3t] [3s] [3a']

wherein each of $R^1$, $R^2$, $R^3$, $R^4$, $R^5$, $R^6$, $R^9$, $R^Z$, $Z^1$, $Z^2$, $Z^3$ and $Z^4$ has the same meaning as given above.

(a) The compound represented by general formula [3t] or a salt thereof can be obtained by performing the reaction according to the production method G(b), using the compound represented by general formula [3d] or a salt thereof.
(b) The compound represented by general formula [3s] or a salt thereof can be obtained by performing the reaction according to the production method 3, using the compound represented by general formula [3t] or a salt thereof.
(c) The compound represented by general formula [3a'] or a salt thereof can be obtained by performing the reaction according to the production method K, using the compound represented by general formula [3s] or a salt thereof.

[Production method N]

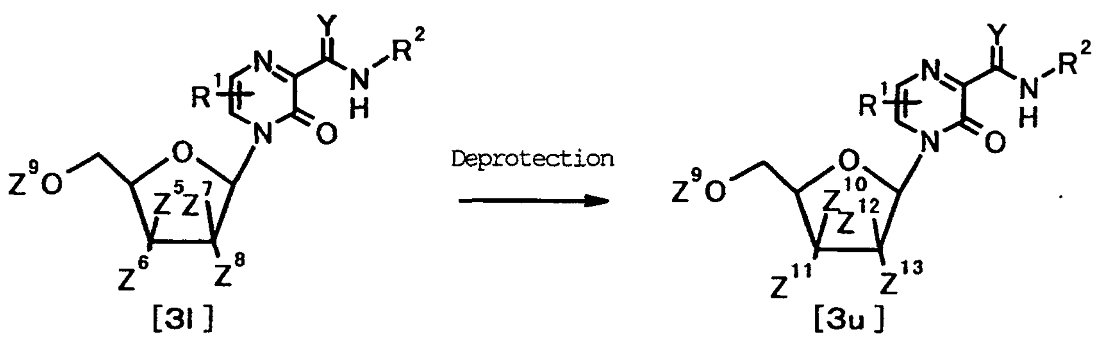


[3l ] [3u ]

wherein each of $R^1$, $R^2$, $Z^5$, $Z^6$, $Z^7$, $Z^8$, $Z^9$, $Z^{10}$, $Z^{11}$, $Z^{12}$, $Z^{13}$ and Y has the same meaning as given above.

The compound represented by general formula [3u] or a salt thereof can be obtained by performing the reaction according to the production method 3, using the compound represented by general formula [31] or a salt thereof.

When the compound obtained by the above production method has isomers (e.g., optical isomers, geometric isomers, tautomers, etc.), these isomers may also be used. In addition, solvates, hydrates, and various forms of crystals may also be used. Moreover, after completion of the reaction, the reaction product of interest may directly be used in

the following reaction without isolating it.

Where the compound obtained by the above production method has an amino, hydroxyl or carboxyl group, it is also possible that these groups are previously protected by common protecting groups, and that after completion of the reaction, these protecting groups are removed by common methods.

The compound represented by general formula [1] or a salt thereof can be isolated, purified or recrystallized according to common methods such as extraction, crystallization and/or column chromatography.

When the compound of the present invention is used as a pharmaceutical, it can be prepared as a pharmaceutical composition by an ordinary method using a pharmaceutical carrier used in common pharmaceutical preparation. Various types of carriers that are commonly used for ordinary pharmaceuticals, such as an excipient, binder, disintegrator, lubricant, coloring agent, corrective agent, flavoring agent or surfactant, may be used herein.

The administration form of the compound of the present invention is not particularly limited, but it can be appropriately selected depending on therapeutic purposes. More specifically, examples of such an administration form may include: parenteral agents such as an injection, suppository or external preparation (ointment, fomentation, etc.); aerosols; and oral agents such as a tablet, powder, fine granule, granule, capsule, liquid, pill, suspension, syrup or emulsion.

Various types of agents described above can be prepared as pharmaceuticals by common methods.

When the present compound is prepared into the form of a solid pharmaceutical for oral administration, such as a tablet, powder, fine granule or granule, examples of a carrier used herein may include: excipients (lactose, sucrose, sodium chloride, glucose, starch, calcium carbonate, kaolin, crystalline cellulose, calcium diphosphate anhydride, alginic acid, etc.); binders (simple syrup, glucose solution, starch solution, gelatin solution, polyvinyl alcohol, polyvinyl ether, polyvinylpyrrolidone, carboxymethyl cellulose, shellac, methylcellulose, ethylcellulose, sodium alginate, gum Arabic, hydroxypropylmethylcellulose, hydroxypropylcellulose, their water and/or ethanol solution, etc.); disintegrators (starch, alginic acid, crosslinked polyvinylpyrrolidone, crosslinked carboxymethylcellulose sodium, carboxymethylcellulose calcium, sodium glycolate starch, etc.); release-controlling agents (higher fatty acid, higher fatty alcohol, cacao butter, hydrogenated oil, water-soluble polymer, polymer soluble in gastric juice, polymer soluble in intestinal juice, etc.); absorbefacients (surfactants such as quaternary ammonium salt, sodium lauryl sulfate or sorbitan monooleate); absorbents (starch, lactose, kaolin, bentonite, silicic acid anhydride, hydrated silicon dioxide, magnesium aluminometasilicate, colloidal silicic acid, etc.); and lubricants (purified talc, stearate, silicic acids, polyethylene glycol, etc.)

Tablets may be converted, as necessary, into those coated with common coatings, such as a sugar-coated tablet, gelatin-coated tablet, tablet coated with a coating that is soluble in gastric juice, tablet coated with a coating that is soluble in intestinal juice, or tablet coated with a water-soluble film.

Capsules can be prepared by mixing the compound with the aforementioned various types of carriers and then filling the obtained mixture into a hard gelatin capsule, a soft capsule, etc.

Liquid pharmaceutical can be a water or oil suspension, solution, syrup or elixir, and these can be prepared by common methods using ordinary additives.

When the compound of the present invention is prepared into the form of an injection, examples of a carrier used herein may include: diluents (water, ethyl alcohol, Macrogol, propylene glycol, etc.); pH controllers or buffers (citric acid, acetic acid, phosphoric acid, lactic acid and their salts, sulfuric acid, sodium hydroxide, etc.); and stabilizers (sodium pyrosulfite, ethylenediaminetetraacetic acid, thioglycolic acid, thiolactic acid, etc.). In this case, common salts, glucose, mannitol or glycerine may be contained in the pharmaceutical composition in an amount sufficient to prepare an isotonic solution. In addition, a common solubilizer, soothing agent or local anesthetic may also be added thereto.

The administration method, the dosage, and the number of doses can be appropriately selected depending on a patient's age, body weight and symptom. When the patient is an adult, the compound of the present invention may be administered orally or parenterally (e.g., injection, infusion, administration to the rectum, etc.) at a dosage of 0.1 to 1000 mg/kg, once per day or divided into several times.

The virus growth inhibition and/or virucidal method of the present invention is characterized in that it comprises the following steps.

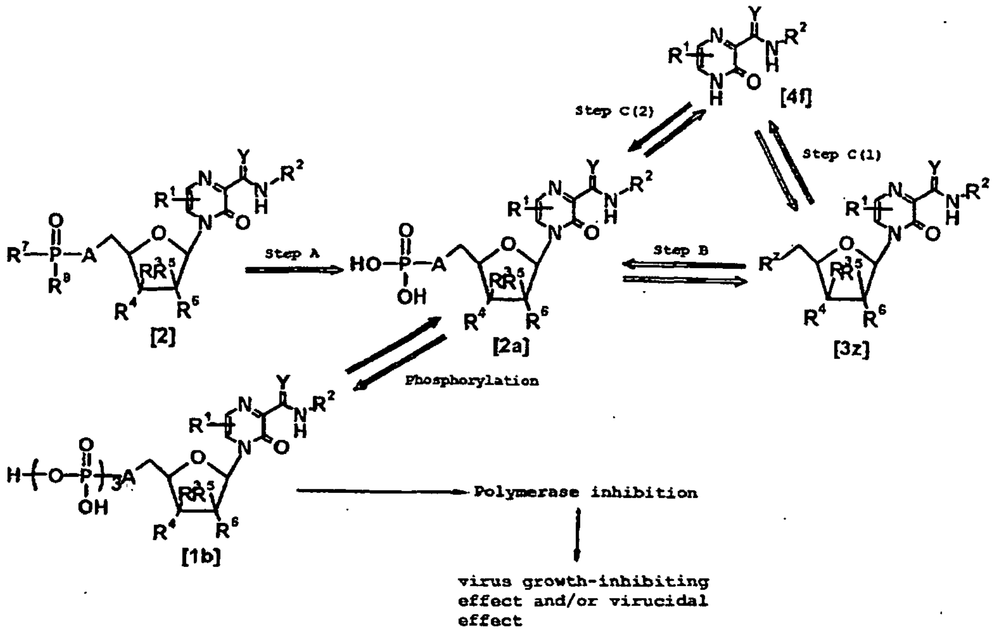


wherein each of $R^1$, $R^2$, $R^3$, $R^4$, $R^5$, $R^6$, $R^7$, $R^8$, $R^Z$, A and Y has the same meaning as given above.

Step A: the pyrazine nucleotide analog represented by general formula [2] or a salt thereof is converted in vivo into the compound represented by general formula [2a] or a salt thereof.

Step B: the pyrazine nucleoside analog represented by general formula [3z] or a salt thereof is converted in vivo into the compound represented by general formula [2a] or a salt thereof.

Step C: (1) the pyrazine nucleoside analog represented by general formula [3z] or a salt thereof is converted in vivo by enzyme such as nucleosidase into the compound represented by general formula [4f], and then (2) the obtained compound is converted in vivo by enzyme such as phosphoribosyltransferase into the compound represented by general formula [2a] or a salt thereof.

With regard to steps B, C(1) and C(2), the reverse conversion may also occur in vivo.

The compound represented by general formula [2a] or a salt thereof generated as a result of the above steps is further converted in vivo by enzyme such as nucleotide kinase [Advances in Antiviral Drug Design, Vol. 2, pp. 167 to 172 (1996)] into the compound represented by general formula [1b] (a pyrazine nucleotide triphosphate) or a salt thereof. This compound or a salt thereof exhibits a virus growth inhibition and/or virucidal effect by inhibiting virus polymerase. Moreover, the reverse conversion may also occur in vivo.

Furthermore, the compound wherein, in the above steps, Y is an imino group is converted in vivo into a compound as an oxygen atom, thereby exhibiting pharmacological effects.

EXAMPLES

The present invention will be described in the following Test Examples. However, the present invention is not limited thereto. In the following test examples, compound A represents the compound 4-[(2R, 3R, 4S, 5R)-3,4-dihydroxy-5-(hydroxymethyl)tetrahydro-2-furanyl]-3-oxo-3,4-dihydro-2-pyrazinecarboxamide that is obtained in Example 29; compound B represents the compound 6-chloro-4-[(2R, 3R, 4S, 5R)-3,4-dihydroxy-5-(hydroxymethyl)tetrahydro-2-furanyl]-3-oxo-3,4-dihydro-2-pyrazinecarboxamide that is obtained in Reference Example 7; and compounds shown in tables showing test results indicate products obtained in Reference Examples and Examples.

Test Example 1

[Detection of phosphate compound in cell]

5 ml of an E'-MEM culture medium (containing 1% bovine serum albumin and 3% vitamin solution) containing a compound, 6-fluoro-3-hydroxy-2-[2-$^{14}$C] pyrazinecarboxamide, which had been $^{14}$C-labeled at position 2 of the pyrazine ring, was added to MDCK cells that had been monolayer-cultured on a 55 $cm^2$ culture plate. After completion of culture under conditions of 35°C and 5% $CO_2$ for 20 hours, 6-fluoro-3-hydroxy-2-[2-$^{14}$C] pyrazinecarboxamide and a converted compound were extracted from cell fractions, using a 66.6% acetonitrile solution. The extract was lyophilized and concentrated, and then analysis was carried out under the HPLC analysis conditions indicated below.

As a result, a monophosphate (the compound in Reference Example 15; recovery time: 23.3 minutes) and a triphosphate (the compound in Reference Example 16; recovery time: 34.0 minutes) were detected.

HPLC analysis conditions

Instruments

Pump: HITACHI L-6200
Detector: HITACHI L-4000
Radioactivity detector: Packard FLO-ONE500

Analysis conditions

Separation column: Develosil ODS-MG-5 (4.6 x 250 mm)
Mobile phase A: 0.2 M TEAA, pH 6.6
Mobile phase B: 10% acetonitrile, 0.2 M TEAA, pH 6.6
Mixing ratio: 0 to 10 minutes; B 5%
10 to 35 minutes; B 5% to 75% (linear)
35 to 50 minutes; B 75%

Test Example 2

[Detection of deprotected compound in cell]

The compound of Example 2 was added to MDCK cells suspended in a Hank's balanced salt solution, and the mixture was incubated at 37°C for 1 hour. Thereafter, the obtained product was layered on silicon oil (KF-99), followed by centrifugation at 4°C. Cell fractions from the precipitate were suspended and frozen-thawed in a mobile phase indicated below, and the obtained solution was analyzed under the HPLC analysis conditions indicated below.

As a result, a monophosphate (recovery time: 14.3 minutes) was detected as a deprotected compound. HPLC analysis conditions

Instruments

Pump: HITACHI L-6000
Detector: HITACHI L-7500

Analysis conditions

Separation column: Develosil ODS-MG-5 (4.6 x 250 mm)
Mobile phase: 0.02 M phosphate buffer solution, pH 3.0

Test Example 3-1

[Polymerase inhibition test (influenza virus)]

Influenza virus particles were treated with a dissolving solution (100 mM Tris-HCl (pH 8), 100 mM KCl, 5 mM

$MgCl_2$, 1.5 mM DTT, 5% glycerol, 1.5% Triton N101, 1% LPC), and these particles were used as polymerase crude enzymes. Each of the test compounds with different concentrations was added to a reaction buffer (100 mM Tris-HCl (pH 8.0), 100 mM KCl, 5 mM $MgCl_2$, 1 mM DTT, 0.25% Triton N101, 0.25 mM ApG, 0.1 mM ATP, 0.05 mM CTP, 0.05 mM UTP, 0.0005 mM GTP, $^{32}$P-GTP, crude enzyme), followed by incubation at 30°C for 60 minutes. Thereafter, 10% trichloroacetic acid (TCA) was added thereto, and the mixture was retained on ice for 60 minutes. Thereafter, it was dropped on a GF/C filter, and the filter was then washed with 5% TCA. The filter was dried, and scintillation cocktail was added thereto. The radioactivity was determined using a liquid scintillation counter. The measurement value was given as a 50% inhibition concentration, providing that a group in which no test compounds were added was defined as 100%. The results are shown in Table 1.

[Table 1]

| Compound | 50% inhibition concentration (μM) |
|---|---|
| Reference Example 16 | 0.14 |
| Reference Example 17 | 0.33 |
| Reference Example 22 | 0.076 |
| Reference Example 23 | 0.25 |
| Reference Example 25 | 28 |

Test Example 3-2

[Polymerase inhibition test (hepatitis C virus (HCV))]

The NS5B region of hepatitis C virus was produced in Escherichia coli, and it was used as HCV polymerase for the test. The sequence of the 3'-region of HCV was prepared by the in vitro transcription method, and it was used as an RNA template for the test.

Each of the test compounds with different concentrations was added to a reaction buffer (20 mM Tris-HCl (pH 8.0), 0.05 mM $MnCl_2$, 1 mM DTT, 20 units RNase inhibitor, [α-$^{32}$P]GTP, 0.05 mM each of ATP, CTP and UTP, and 2 μg/mL RNA template), followed by incubation at 30°C for 2 hours. Thereafter, 10% TCA was added to terminate the reaction. Thereafter, the reaction solution was dropped on a DE81 filter, and the filter was then washed with 5% TCA. The filter was dried, and scintillation cocktail was added thereto. The radioactivity was determined using a liquid scintillation counter. The measurement value was given as a 50% inhibition concentration, providing that a group in which no test compounds were added was defined as 100%. The results are shown in Table 1-2.

[Table 1-2]

| Compound | 50% inhibition concentration (μM) |
|---|---|
| Reference Example 16 | 0.75 |
| Reference Example 17 | 2.7 |
| Reference Example 22 | 0.088 |
| Reference Example 23 | 2.2 |
| Reference Example 25 | 1.6 |

Test Example 3-3

[Human RNA polymerase inhibition test]

A Hela cell nucleus extract (Promega) was used as human RNA polymerase for the test. The pCMP script was cleaved with restriction enzymes and then purified, and it was used as a DNA template for the test.

Each of the test compounds with different concentrations was added to a reaction buffer (Hela cell nucleus extract, 3 mM $MgCl_2$, 0.4 mM each of ATP, UTP and CTP, 0.016 mM GTP, 16 μg/mL DNA template, 0.4 mCi/mL [α-$^{32}$P] GTP), followed by incubation at 30°C for 1 hour. After completion of the reaction, the reaction solution was dropped on a DE81 filter, and the filter was then washed with 5% $Na_2HPO_4$ solution 3 times for 30 minutes, and then with distilled water once for 1 minute. The filter was dried, and scintillation cocktail was added thereto. The radioactivity was determined using a liquid scintillation counter. The measurement value was given as a 50% inhibition concentration, providing that a group in which no test compounds were added was defined as 100%. The results are shown in Table 1-3.

[Table 1-3]

| Compound | 50% inhibition concentration (μM) |
|---|---|
| Reference Example 16 | >200 |
| Reference Example 17 | >200 |
| Reference Example 22 | >200 |
| Reference Example 23 | >200 |
| Reference Example 25 | >200 |

Test Example 4

[Anti-influenza effect]

MDCK cells that had fully grown in a 6-well culture plate were infected with influenza virus A/PR/8/34 at 70 PFU/well. After 60 minutes, the infection solution was removed, and an E'-MEM culture medium containing 0.6% agar noble, 1% bovine serum albumin and 3 μg/mL acetyltrypsin that contained a 100 μg/mL test compound was added thereto. The mixture was fully solidified and then turned upside down. It was cultured for 3 days under conditions of 35°C, a humidity of 100% and 5% $CO_2$. After completion of the culture, surviving cells were stained with 1% neutral red, and then fixed with 10% formalin. Thereafter, the agar medium was removed with running water, and then the number of plaques was counted. The plaque inhibition rate was expressed by the percentage obtained by comparing with a control to which no test compounds were added. The results are shown in Table 2.

[Table 2]

| Compound | Inhibition rate (%) |
|---|---|
| Reference Example 7 | 42 |
| Reference Example 12 | 78 |
| Example 2 | 100 |
| Example 6 | 100 |
| Example 11 | 24 |
| Example 22 | 33 |
| Example 29 | 100 |
| Example 31 | 44 |
| Example 32 | 100 |

Test Example 5

[Anti-BVDV effect]

MDBK cells that had fully grown in a 6-well culture plate were infected with bovine diarrhea virus (BVDV) NADL at 70 PFU/well. After 60 minutes, the infection solution was removed, and a test culture solution (E'-MEM) containing 5% horse serum and 1% agar (SeaPlaque Agar) that contained a 100 μg/mL test compound was added thereto. The mixture was fully solidified and then cultured for 3 days under conditions of 37°C, a humidity of 100% and 5% $CO_2$. After completion of the culture, the test plate was fixed with a 3% formaldehyde solution, and the agar medium was removed with running water, followed by staining with a 1% crystal violet solution, so as to count the number of plaques. The plaque inhibition rate was expressed by the percentage obtained by comparing with a control to which no test compounds were added. The results are shown in Table 3.

[Table 3]

| Compound | Inhibition rate (%) |
|---|---|
| Example 2 | 100 |
| Example 4 | 68 |
| Example 6 | 57 |
| Example 29 | 100 |

[Table 3] (continued)

| Compound | Inhibition rate (%) |
|---|---|
| Example 32 | 100 |

Test Example 6

[Test to confirm presence of phosphate form in the liver of mouse administered with compound A]

Compound A was administered into the caudal vein of a mouse at a dose of 300 mg/kg. 30 minutes after the administration, 1.6 g of the liver was excised, and it was then ground under ice cooling, while adding thereto 22.5 mL of 70 % methanol that had been cooled to -20°C, so that compound A and a phosphate(s) form were extracted. 10 mL of the supernatant of the extract centrifuged at 4°C for 10 minutes was purified under the condition indicated below, using a solid extraction cartridge (Varian BOND ELUT SAX HF 2 g/12 mL; eluted with 0.01 M to 1.0 M KCl) pretreated with 12 mL of methanol and 12 mL each of 1.0 M and 0.005 M KCl.

The phosphate form contained in 5 mL of No. 3 0.05 M KCl eluate and a synthetic monophosphate compound (a compound obtained in Reference Example 30) were identical in terms of HPLC retention time and UV spectrum in HPLC analysis (HPLC-1) described in HPLC conditions. In addition, the phosphate form contained in 5 mL of No. 1 0.5 M KCl eluate and a synthetic diphosphate compound (a compound obtained in Reference Example 31) were identical in terms of HPLC retention time in HPLC analysis (HPLC-2) described in HPLC conditions. Moreover, the phosphate form contained in 5 mL of No. 2 0.5 M KCl eluate and a synthetic triphosphate compound (a compound obtained in Reference Example 22) were identical in terms of HPLC retention time in HPLC analysis (HPLC-3) described in HPLC conditions.

0.5 mL of 0.5 M Tris-HCl (pH 8.0) and 0.5 mL of 0.1 M $MgCl_2$ were added to 4 mL of the above No. 3 0.05 M KCl eluate, and then 0.5 mL of alkali phosphatase prepared from the intestine of a calf (EC3.1.3.1., 20 U/mL) was further added thereto, followed by incubation at 37°C for 1 hour. Likewise, 0.5 mL of 0.5 M Tris-HCl (pH 8.0) and 0.5 mL of 0.1 M $MgCl_2$ were added to 4 mL of the above No. 1 0.5 M KCl eluate, and then 0.5 mL of alkali phosphatase prepared from the intestine of a calf (EC3.1.3.1., 20 U/mL) was further added thereto, followed by incubation at 37°C for 1 hour. Likewise, 0.2 mL of 0.5 M Tris-HCl (pH 8.0) and 0.2 mL of 0.1 M $MgCl_2$ were added to 1.6 mL of the above No. 2 0.5 M KCl eluate, and then 0.1 mL of alkali phosphatase prepared from the intestine of a calf (EC3.1.3.1., 20 U/mL) was further added thereto, followed by incubation at 37°C for 1 hour.

By the HPLC analysis (HPLC-4) described in HPLC conditions, it was confirmed that the monophosphate disappeared from the No. 3 0.05 M KCl eluate, and that a newly generated compound and compound A were identical in terms of HPLC retention time and UV spectrum. Likewise, by the HPLC analysis (HPLC-4) described in HPLC conditions, it was confirmed that the diphosphate form disappeared from the No. 1 0.5 M KCl eluate, and that a newly generated compound and compound A were identical in terms of HPLC retention time and UV spectrum. Likewise, by the HPLC analysis (HPLC-4) described in HPLC conditions, it was confirmed that the triphosphate disappeared from the No. 2 0.5 M KCl eluate, and that a newly generated compound and compound A were identical in terms of HPLC retention time and UV spectrum.

Based on these results, it was confirmed that compound A was converted in vivo into a monophosphate, then into a diphosphate form, and then into a triphosphate.

Purification conditions using solid extraction cartridge

Washing: washing was carried out using the following 3 solvents in the following order.
Water (cooled with ice) 12 mL
60% methanol (cooled with ice) 12 mL
Water (cooled with ice) 12 mL
Elution: elution was successively carried out under the following concentration conditions. (Elution was carried out by unit of 5 mL. "x 2" and "x 3" mean that elution of 5 mL was performed two and three times, respectively with each concentration. Fractions were defined as No. 1 eluate, No. 2 eluate, and No. 3 eluate, successively.)

0.01 M KCl (5 mL × 2)

0.05 M KCl (5 mL × 3)

0.1 M KCl (5 mL × 3)

0.5 M KCl (5 mL × 2)

1.0 M KCl (5 mL × 2)

HPLC analysis conditions

HPLC-1

Column: 4.6 × 250 mm, Nomura Chemical Co., Ltd., Develosil ODS-MG-5
Mobile phase: 0.02 M phosphate buffer solution (pH 7.0)
Detection: UV 200 to 400 nm

HPLC-2

Column: 4.6 × 250 mm, Whatman Partisil 10-SAX
Mobile phase: 0.2 M phosphate buffer solution (pH 3.5)
Detection: UV 200 to 400 nm

HPLC-3

Column: 4.6 × 250 mm, Whatman Partisil 10-SAX
Mobile phase: 0.6 M phosphate buffer solution (pH 3.5)
Detection: UV 200 to 400 nm

HPLC-4

Column: 4.6 × 250 mm, Nomura Chemical Co., Ltd., Develosil ODS-MG-5
Mobile phase: 2% acetonitrile 0.02 M phosphate buffer solution (pH 5.0)
Detection: UV 200 to 400 nm

Measuring instruments used
Diode Array Detector Agilent 1100 Series
Quatemary Pump Agilent 1100 Series
Autosampler Agilent 1100 Series
ChemStation Agilent 1100 Series

Test Example 7

[Determination of concentration of compound A in plasma of mouse orally administered with test compound]

The test compound was orally administered once to two mice (ICR) in a group. Blood was collected 30 minutes after the administration. 400 μL of acetonitrile was added to 200 μL of the centrifuged plasma. The mixture was centrifuged, and the precipitated protein was removed. The obtained supernatant was concentrated under reduced pressure, and then the concentration of compound A in the plasma was determined under the following HPLC conditions. The results are shown in Table 4.

HPLC conditions

Column: Develosil ODS-MG-5, 4.6 × 250 mm
(Nomura Chemical Co., Ltd.)
Guard column: Develosil ODS-MG-5, 4.6 × 10 mm
(Nomura Chemical Co., Ltd.)
Detection: UV 350 nm
Mobile phase: 2% acetonitrile 0.02 M
phosphate buffer solution (pH 5.0)

Measuring instruments

Detector: Shimadzu SPD-6A
Pump: HITACHI L-6000

[Table 4]

| Test compound | Dosage mg/kg | Concentration of compound A in plasma µg/mL |
|---|---|---|
| Reference Example 4 | 200 | 0.2 |
| Example 17 | 50 | 1.6 |
| Example 19 | 50 | 3.5 |
| Example 21 | 50 | 0.4 |
| Example 22 | 50 | 3.1 |
| Example 28 | 50 | 0.3 |
| Example 34 | 50 | 12.5 |
| Example 36 | 41 | 5.7 |

Test Example 8

[Determination of concentration of compound B in plasma of mouse orally administered with test compound]

The compound in Reference Example 6 was orally administered at 200 mg/kg once to two ICR mice in a group. Blood was collected 30 minutes and 60 minutes after the administration. The same operation as in Test Example 7 was carried out, and the concentration of compound B in the plasma was determined by HPLC. The results are shown in Table 5. HPLC conditions

Column: Develosil ODS-MG-5, 4.6 × 250 mm
(Nomura Chemical Co., Ltd.)
Guard column: Develosil ODS-MG-5, 4.6 × 10 mm
(Nomura Chemical Co., Ltd.)
Detection wavelength: UV 350 nm
Mobile phase: 5% acetonitrile 0.04 M
phosphate buffer solution (pH 6.0)

[Table 5]

| Test compound | Concentration of plasma (μg/mL) 30 minutes later | compound B in 60 minutes later |
|---|---|---|
| Reference Example 6 | 1.6 | 4.6 |

Text example 9

[Detection of phosphate compound in cell by addition of 3-hydroxy-2-pyrazinecarboxamide]

5 ml of an E'-MEM culture medium (containing 1% bovine serum albumin and 3% vitamin solution) containing 3-hydroxy-2-pyrazinecarboxamide (final concentration: 5000 μM) was added to MDCK cells that had been monolayer-cultured on a 55 $cm^2$ culture plate, and culture was carried out under conditions of 37°C and 5% $CO_2$ for 24 hours. 1 mL of 70% methanol that had been cooled to -20°C was added to cell fractions under ice cooling, and 3-hydroxy-2-pyrazinecarboxamide, compound A, and a phosphate(s) were extracted therefrom. 0.7 mL of the supernatant of the extract centrifuged at 4°C for 10 minutes was purified under the condition indicated below, using a solid extraction cartridge (Varian BOND ELUT SAX HF 100 mg/l mL; eluted with 0.01 M to 1.0 M KCl) pretreated with 1 mL of methanol and 1 mL each of 1.0 M and 0.005 M KCl.

The phosphate form contained in 1 mL of 0.05 M KCl eluate and a synthetic monophosphate compound (a compound obtained in Reference Example 30) were identical in terms of HPLC retention time and UV spectrum in HPLC analysis (HPLC-5). In addition, the phosphate form contained in 1 mL of 0.25 M KCl eluate and a synthetic diphosphate compound (a compound obtained in Reference Example 31) were identical in terms of HPLC retention time in HPLC analysis (HPLC-6). Moreover, the phosphate form contained in 1 mL of 0.5 M KCl eluate and a synthetic triphosphate compound (a compound obtained in Reference Example 22) were identical in terms of HPLC retention time in HPLC analysis (HPLC-6).

To 0.8 mL each of the above KCl eluates, 0.1 mL of 0.5 M Tris-HCl (pH 8.0) and 0.1 mL of 0.1 M $MgCl_2$ were added. Then 0.8 mL of the mixture was taken, to which 0.08 mL of alkali phosphatase prepared from the intestine of a calf (EC3.1.3.1., 20 U/mL) was added, followed by incubation at 37°C for 1 hour.

By the HPLC analysis (HPLC-7), it was confirmed that the monophosphate disappeared from the 0.05 M KCl eluate, and that a newly generated compound and compound A were identical in terms of HPLC retention time. Moreover, by the HPLC analysis (HPLC-8), it was confirmed that a newly generated compound and compound A were identical in terms of UV spectrum. Likewise, by the HPLC analysis (HPLC-7), it was confirmed that the diphosphate form disappeared from the 0.25 M KCl eluate, and that a newly generated compound and compound A were identical in terms of HPLC retention time. Furthermore, by the HPLC analysis (HPLC-8), it was confirmed that a newly generated compound and compound A were identical in terms of UV spectrum. Likewise, by the HPLC analysis (HPLC-7), it was confirmed that the triphosphate disappeared from the 0.5 M KCl eluate, and that a newly generated compound and compound A were identical in terms of HPLC retention time.

Based on these results, it was confirmed that 3-hydroxy-2-pyrazinecarboxamide, was converted in a cell into compound A, a monophosphate (a compound obtained in Reference Example 30), then into a diphosphate form (a compound obtained in Reference Example 31), and then into a triphosphate (a compound obtained in Reference Example 22).

Purification conditions using solid extraction cartridge

Washing: Washing was carried out using the following 3 solvents in the following order.
Water (cooled with ice) 1 mL
60% methanol (cooled with ice) 1 mL
Water (cooled with ice) 1 mL
Elution: Elution was successively carried out under the following concentration conditions. (Elution was carried out by unit of 1 mL.)

0.01 M KCl (1 mL)
0.05 M KCl (1 mL)
0.1 M KCl (1 mL)
0.25 M KCl (1 mL)
0.5 M KCl (1 mL)

1.0 M KCl (1 mL)

HPLC analysis conditions

HPLC-5

Column: 4.6 × 250 mm, Nomura Chemical Co., Ltd., Develosil ODS-MG-5
Mobile phase: 0.02 M phosphate buffer solution (pH 7.0)
Detection: UV 200 to 400 nm
Measuring instruments used
Diode Array Detector Agilent 1100 Series
Quatemary Pump Agilent 1100 Series
Autosampler Agilent 1100 Series
ChemStation Agilent 1100 Series

HPLC-6

Column: 4.6 × 250 mm, Whatman Partisil 10-SAX
Mobile phase: 0.75 M phosphate buffer solution (pH 3.5)
Detection: UV 350 nm
Measuring instruments used
Shimadzu SPD-6A UV Spectrophotometric

Detector

HITACHI L-6000 Pump

HPLC-7

Column: 4.6 × 250 mm, Nomura Chemical Co., Ltd., Develosil ODS-MG-5
Mobile phase: 5% acetonitrile 0.02 M phosphate buffer solution (pH 5.0)
Detection: UV 350 nm
Measuring instruments used
Shimadzu SPD-6A UV Spectrophotometric

Detector

HITACHI L-6000 Pump

HPLC-8

Column: 4.6 × 250 mm, Nomura Chemical Co., Ltd., Develosil ODS-MG-5
Mobile phase: 2% acetonitrile 0.02 M phosphate buffer solution (pH 5.0)
Detection: UV 200 to 400 nm
Measuring instruments used
Diode Array Detector Agilent 1100 Series

Quatemary Pump Agilent 1100 Series
Autosampler Agilent 1100 Series
ChemStation Agilent 1100 Series

Text example 10

[Detection of triphosphate compound in cell by addition of compound A]

10 ml of an E'-MEM culture medium (containing 5% fetal bovine serum) containing compound A was added to MDBK cells that had been monolayer-cultured on a 55 $cm^2$ culture plate. After completion of culture under conditions of 37°C and 5% $CO_2$ for 24 hours, the culture was removed using a cell scraper, and the culture medium was removed by centrifugation. Thereafter, 5% trichloroacetic acid was added to the obtained cell fractions, so that a converted compound was extracted. An equal amount of 20% trioctylamine-containing pentane was added to the extract, and the obtained water layer was then concentrated using a centrifugal concentrator. In the above concentrate, a component whose HPLC retention time matches that of a synthetic compound of triphosphates (a compound obtained in Reference Example 22) was detected.

HPLC conditions

Instruments

Pump: HITACHI L-6000
Detector: HITACHI L-4000

Analysis conditions

Separation column: Develosil ODS-MG-5 (4.6 x 250 mm)
Mobile phase: 7% acetonitrile, 5 mM tetrabutylammonium bromide, 0.1 M phosphate buffer solution (pH 7.0)
Measurement wavelength: 350 nm

Text example 11

[Detection of monophosphate and diphosphate compounds in cell by addition of compound A]

5 ml of an E'-MEM culture medium (containing 1% bovine serum albumin and 3% vitamin solution) containing compound A (final concentration: 5000 μM) was added to MDCK cells that had been monolayer-cultured on a 55 $cm^2$ culture plate, and culture was carried out under conditions of 37°C and 5% $CO_2$ for 24 hours. 1 mL of 70% methanol that had been cooled to - 20°C was added to cell fractions under ice cooling, and compound A, and a phosphate(s) were extracted therefrom. 0.7 mL of the supernatant of the extract centrifuged at 4°C for 10 minutes was purified under the condition indicated below, using a solid extraction cartridge (Varian BOND ELUT SAX HF 100 mg/l mL; eluted with 0.01 M to 1.0 M KCl) pretreated with 1 mL of methanol and 1 mL each of 1.0 M and 0.005 M KCl.

The phosphate form contained in 1 mL of 0.05 M KCl No. 1 eluant and a synthetic monophosphate compound (a compound obtained in Reference Example 30) were identical in terms of HPLC retention time and UV spectrum in HPLC analysis (HPLC-9). In addition, the phosphate form contained in 1 mL of 0.5 M KCl eluate and a synthetic diphosphate compound (a compound obtained in Reference Example 31) were identical in terms of HPLC retention time in HPLC analysis (HPLC-10).

To 0.8 mL of the above 0.05 M KCl No. 1 eluant, 0.1 mL of 0.5 M Tris-HCl (pH 8.0) and 0.1 mL of 0.1 M $MgCl_2$ were added. Then 0.5 mL of the mixture was taken, to which 0.06 mL of alkali phosphatase prepared from the intestine of a calf (EC3.1.3.1., 20 U/mL) was added, followed by incubation at 37°C for 1 hour. By the HPLC analysis (HPLC-11), it was confirmed that the monophosphate disappeared from the 0.05 M KCl eluate, and that a newly generated compound and compound A were identical in terms of HPLC retention time and UV spectrum.

Based on these results, it was confirmed that compound A was converted in a cell into a monophosphate (a compound obtained in Reference Example 30), and a diphosphate form (a compound obtained in Reference Example 31).

Purification conditions using solid extraction cartridge

Washing: Washing was carried out using the following 3 solvents in the following order.
Water (cooled with ice) 1 mL
60% methanol (cooled with ice) 1 mL
Water (cooled with ice) 1 mL
Elution: elution was successively carried out under the following concentration conditions. (Elution was carried out by unit of 1 mL. "x 2" means that elution of 1 mL was performed twice with each concentration twice. Fractions were defined as No. 1 eluate and No. 2 eluate, successively.)

0.01 M KCl (1 mL)
0.05 M KCl (1 mL x 2)
0.1 M KCl (1 mL)
0.5 M KCl (1 mL)
1.0 M KCl (1 mL)

HPLC analysis conditions

HPLC-9

Column: 4.6 × 250 mm, Nomura Chemical Co., Ltd., Develosil ODS-MG-5
Mobile phase: 0.02 M phosphate buffer solution (pH 7.0)
Detection: UV 200 to 400 nm
Measuring instruments used
Diode Array Detector Agilent 1100 Series
Quatemary Pump Agilent 1100 Series
Autosampler Agilent 1100 Series
ChemStation Agilent 1100 Series

HPLC-10

Column: 4.6 × 250 mm, Whatman Partisil 10-SAX
Mobile phase: 0.2 M phosphate buffer solution

(pH 3.5)

Detection: UV 350 nm
Measuring instruments used
Shimadzu SPD-6A UV Spectrophotometric

Detector

HITACHI L-6000 Pump

HPLC-11

Column: 4.6 × 250 mm, Nomura Chemical Co., Ltd., Develosil ODS-MG-5

Mobile phase: 2% acetonitrile 0.02 M phosphate buffer solution (pH 5.0)
Detection: UV 200 to 400 nm
Measuring instruments used
Diode Array Detector Agilent 1100 Series
Quatemary Pump Agilent 1100 Series
Autosampler Agilent 1100 Series
ChemStation Agilent 1100 Series

Test Example 12

[Inosine monophosphate dehydrogenase (IMPDH) inhibition test]

MDCK cells monolayer-cultured on a culture plate were suspended in 0.05 M Tris-HCl (pH 8.0), and the suspension was homogenated with a Downs homogenizer to obtain a cell homogenate. The cell homogenate was centrifuged at 16000 × g, and the thus obtained supernatant was used as an IMPDH enzyme solution.

As reaction compositions, 0.1 M Tris-HCl (pH 8.0), 0.1 M KCl, 30 mM EDTA, 5 mM NAD, 5 mg/mL bovine serum albumin, and 0.04 mM [8-$^{14}$C]-inosine 5'-monophosphate were used. After completion of reaction at 37°C for 1 hour, 2 volumes of acetonitrile were added to terminate the reaction, and the reaction product was concentrated. The obtained concentrate was analyzed under the HPLC conditions indicated below. The ratio between the reaction substrate ([$^{14}$C]-inosine 5'-monophosphate) and the reaction product ([$^{14}$C]-xanthosine 5'-monophosphate) was obtained, and the reaction rate was calculated. Ribavirin monophosphate was used as a control compound. The results are shown in Table 6.

HPLC analysis conditions

Separation column: Develosil ODS-MG-5 (4.6 × 250 mm)
Mobile phase: 20% acetonitrile, 5 mM butylammonium bromide, 0.02 M phosphate buffer solution (pH 7.0)
Radiation detector: Packard FLO-ONE500

[Table 6]

| Test compound | 50% inhibition concentration (μM) |
|---|---|
| Reference Example 30 | 980 |
| Example 37 | 740 |
| Control compound | 1.9 |

Next, the compound of the present invention will be explained in Reference Examples and examples. However, the present invention is not limited thereto.

Mixing ratios in eluants are all expressed by volume ratios. Silica gel BW-127ZH (Fuji Silysia Chemical Ltd.) was used as a medium for column chromatography; YMC GEL ODS-AM 120-S50 (YMC Co., Ltd.) was used as a carrier for reverse phase silica gel column chromatography; and DEAE cellulose (Wako Pure Chemical Industries, Ltd.) was used as a carrier for ion exchange column chromatography. The symbols used in Reference Examples and Examples mean the following: DMSO-$d_6$: Deuterated dimethyl sulfoxide, Ms: Methanesulfonyl group, Ph: Phenyl group, and Et: Ethyl group

Reference Example 1

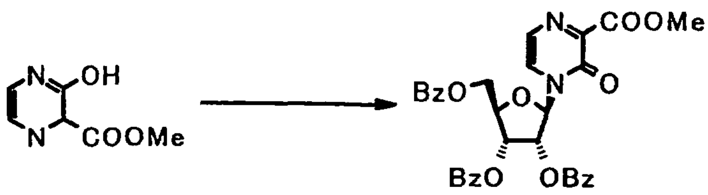

1.52 g of methyl 3-hydroxy-2-pyrazinecarboxylate was suspended in 12.2 mL of 1,1,1,3,3,3-hexamethyldisilazane, and the suspension was heated under reflux for 1 hour. After standing to cool, the solvent was removed under reduced pressure, and the obtained residue was dissolved in 30 mL of dichloroethane under nitrogen atmosphere. 4.98 g of β-D-ribofuranose-1-acetate-2,3,5-tribenzoate and 1.73 mL of tin(IV) chloride were successively added thereto, and the mixture was further stirred at room temperature for 14 hours. The reaction mixture was diluted with 30 mL of chloroform and 30 mL of a saturated sodium bicarbonate aqueous solution, and the precipitate was removed by filtration, so that the organic layer was obtained. The obtained organic layer was successively washed with water and then with a saturated saline solution. Thereafter, the layer was dried with anhydrous magnesium sulfate, and the solvent was removed under reduced pressure. The obtained residue was purified by silica gel column chromatography [eluant; n-hexane : ethyl acetate = 1 : 1], so as to obtain 3.4 g of a white solid, methyl 4-{(2R, 3R, 4R, 5R)-3,4-bis(benzoyloxy)-5-[(benzoyloxy)methyl]tetrahydro-2-furanyl}-3-oxo-3,4-dihydro-2-pyrazinecarboxylate.
IR(KBr)$cm^{-1}$: 1734, 1660
$^1$H-NMR($CDCl_3$)δ: 3.96(3H,s), 4.71(1H,dd,J=4.0,12.4Hz), 4.8-4.9(2H,m), 5.8-5.9(2H,m), 6.45(1H,d,J=4.0Hz), 7.34 (1H,d,J=4.2Hz), 7.3-7.6(9H,m), 7.70(1H,d,J=4.2Hz), 7.9-8.0(4H,m), 8.0-8.1(2H,m)

Reference Example 2

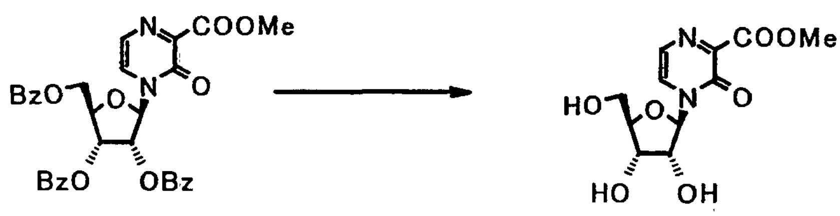

36.3 g of methyl 4-{(2R, 3R, 4R, 5R)-3,4-bis(benzoyloxy)-5-[(benzoyloxy)methyl]tetrahydro-2-furanyl}-3-oxo-3,4-dihydro-2-pyrazinecarboxylate was suspended in 400 mL of methanol, and 11.7 g of a 28% sodium methoxide methanol solution was added thereto under ice cooling. The mixture was stirred at the same temperature for 1 hour. The reaction solution was adjusted to pH 4 using 2M hydrochloric acid, and the solvent was then removed under reduced pressure. The obtained residue was purified by reverse phase silica gel column chromatography [eluant; acetonitrile : water = 1 : 4], so as to obtain 12.6 g of a light yellow solid, methyl 4-[(2R, 3R, 4S, 5R)-3,4-dihydroxy-5-(hydroxymethyl)tetrahydro-2-furanyl]-3-oxo-3,4-dihydro-2-pyrazinecarboxylate.
IR(KBr)$cm^{-1}$: 1740
$^1$H-NMR(DMSO-$d_6$)δ: 3.6-3.65(1H,m), 3.75-3.8(1H,m), 3.83(3H,s), 3.9-4.0(3H,m), 5.13(1H,d,J=5.2Hz), 5.29(1H,t, J=5.2Hz), 5.64(1H,d,J=2.4Hz), 5.91(1H,d,J=2.4Hz), 7.48(1H,d,J=4.4Hz), 8.31(1H,d,J=4.4Hz).

Reference Example 3

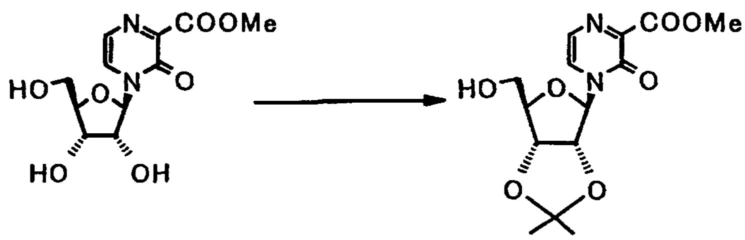

0.5 g of methyl 4-[(2R, 3R, 4S, 5R)-3,4-dihydroxy-5-(hydroxymethyl)tetrahydro-2-furanyl]-3-oxo-3,4-dihydro-2-pyrazinecarboxylate was suspended in 5 mL of acetone, and 1 mL of trimethyl orthoformate and 33 mg of paratoluenesulfonic acid monohydrate were successively added thereto. The mixture was heated under reflux for 1 hour, and the solvent was then removed under reduced pressure. The obtained residue was purified by column chromatography [eluant; ethyl acetate], so as to obtain 0.49 g of a white solid, methyl 4-[(3aR,4R,6R,6aR)-6-(hydroxymethyl)-2,2-dimethyltetrahydrofuro[3,4-d][1,3]dioxol-4-yl]-3-oxo-3,4-dihydro-2-pyrazinecarboxylate.
IR(KBr)$cm^{-1}$: 1728
$^1$H-NMR($CDCl_3$)δ: 1.35(3H,s), 1.60(3H,s), 2.55(1H,t,J=4.6Hz), 3.8-3.9(1H,m), 3.97(3H,s), 3.95-4.0(1H,m), 4.4-4.5(1H, m), 4.97(1H,dd,J=3.2,6.3Hz), 5.01(1H,dd,J=2.4,6.3Hz), 5.80(1H,d,J=2.4Hz), 7.49(1H,d,J=4.3Hz), 7.69(1H,d, J=4.3Hz).

Reference Example 4

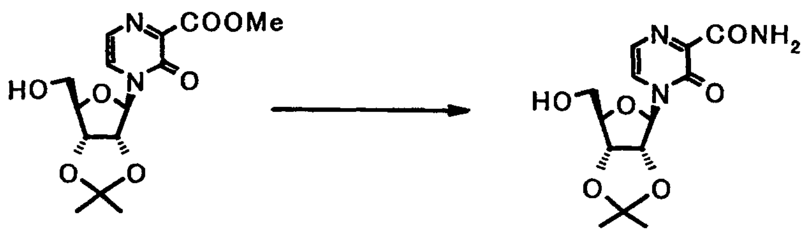

6.78 g of methyl 4-[(3aR,4R,6R,6aR)-6-(hydroxymethyl)-2,2-dimethyltetrahydrofuro[3,4-d][1,3]dioxol-4-yl]-3-oxo-3,4-dihydro-2-pyrazinecarboxylate was dissolved in 68 mL of methanol, and ammonia gas was then introduced therein under ice cooling for saturation. After reaction at the same temperature for 1.5 hours, the deposited solid was collected by filtration, so as to obtain 2.34 g of a light yellow solid, 4-[(3aR,4R,6R,6aR)-6-(hydroxymethyl)-2,2-dimethyltetrahydrofuro[3,4-d][1,3]dioxol-4-yl]-3-oxo-3,4-dihydro-2-pyrazinecarboxamide. The filtrate was concentrated, so as to further obtain 2.54 g of the above compound.
IR(KBr)$cm^{-1}$: 1701, 1654
$^1$H-NMR(DMSO-$d_6$)δ: 1.29(3H,s), 1.51(3H,s), 3.5-3.6(1H,m), 3.6-3.7(1H,m), 4.3-4.4(1H,m), 4.7-4.8(1H,m), 4.8-4.9 (1H,m), 5.22(1H,t,J=4.7Hz), 5.98(1H,s), 7.55(1H,d,J=4.0Hz), 7.76(1H,brs), 8.04(1H,d,J=4.0Hz), 8.36(1H,brs).

Reference Example 5

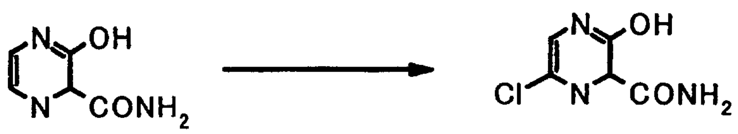

15 mL of sulfuryl chloride was dropped into 80 mL of an N,N-dimethylformamide suspension containing 20 g of 3-hydroxy-2-pyrazinecarboxamide at a temperature between 80°C and 90°C. The mixture was stirred at a temperature between 95°C and 100°C for 1 hour, and it was then poured into a mixed solution of 200 mL of ice water and 200 mL of ethyl acetate. The organic layer was separated. The aqueous layer was extracted with 100 mL of ethyl acetate 5 times, and it was then combined with the organic layer. The mixture was washed with a saturated saline solution.
Thereafter, the mixture was treated with activated carbon, and the solvent was removed under reduced pressure. The obtained residue was suspended in 50 mL of water, and 3.2 g of sodium bicarbonate was added thereto and dissolved. Thereafter, concentrated hydrochloric acid was added thereto to adjust the solution to pH 2. The deposit was collected by filtration, so as to obtain 4.8 g of a white solid, 6-chloro-3-hydroxy-2-pyrazinecarboxamide.
IR(KBr)$cm^{-1}$: 1660
$^1$H-NMR(DMSO-$d_6$)δ: 8.51(2H,brs), 8.73(1H,s), 13.60(1H,brs)

Reference Example 6

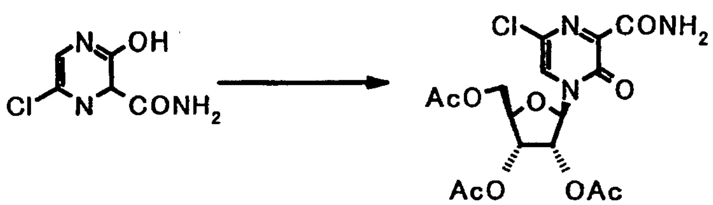

7.5 mL of a 1,1,1,3,3,3-hexamethyldisilazane suspension containing 1.5 g of 6-chloro-3-hydroxy-2-pyrazinecarboxamide was heated under reflux for 30 minutes. After cooling, it was concentrated under reduced pressure. 5 mL of toluene was added thereto, and the solvent was removed under reduced pressure. Thereafter, 5 mL of toluene was added thereto, and the solvent was removed under reduced pressure again. 15 mL of acetonitrile was added to the obtained residue and dissolved, and under ice cooling, β-D-ribofuranose-1,2,3,5-tetraacetate and tin(IV) chloride were successively added thereto, followed by stirring at room temperature for 5 hours. The reaction mixture was diluted with 30 mL of ethyl acetate and 20 mL of water, and it was then adjusted to pH 7 by addition of a saturated sodium bicarbonate aqueous solution. Thereafter, the precipitate was removed by filtration, and the organic layer was separated. The aqueous layer was extracted with 10 mL of ethyl acetate 3 times, and it was then combined with the organic layer. The obtained mixture was washed with a saturated saline solution and then dried with anhydrous magnesium sulfate, and the solvent was removed under reduced pressure. Diethyl ether was added to the obtained residue, and the mixture was collected by filtration, so as to obtain 2.8 g of a light yellow solid, (2R, 3R, 4R, 5R)-4-(acetyloxy)-2-[(acetyloxy)methyl]-5-[3-(aminocarbonyl)-5-chloro-2-oxo-1(2H)-pyrazinyl] tetrahydro-3-furanyl acetate.
IR(KBr)$cm^{-1}$: 1756, 1733, 1701, 1648
$^1$H-NMR($CDCl_3$)δ: 2.09(3H,s), 2.18(3H,s), 2.23(3H,s), 4.45(2H,s), 4.5-4.6(1H,m), 5.2-5.3(1H,m), 5.45-5.5(1H,m), 6.14 (1H,d,J=2.0Hz), 6.22(1H,brs), 8.06(1H,s), 8.84(1H,s).

Reference Example 7

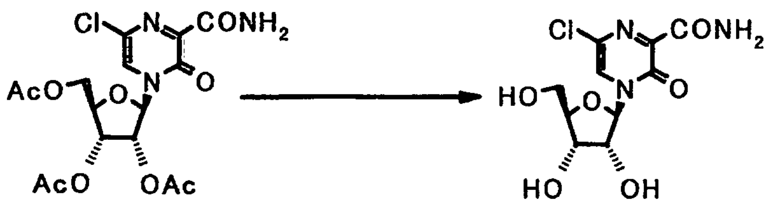

1.8 g of (2R, 3R, 4R, 5R)-4-(acetyloxy)-2-[(acetyloxy)methyl]-5-[3-(aminocarbonyl)-5-chloro-2-oxo-1(2H)-pyrazinyl] tetrahydro-3-furanyl acetate was suspended in 27 mL of methanol, and 2.4 g of a 28% sodium methoxide methanol solution was added thereto under ice cooling, followed by stirring at the same temperature for 30 minutes. 0.95 mL of acetic acid was added thereto, and the solvent was removed under reduced pressure. The obtained residue was purified by silica gel column chromatography [eluant; chloroform : methanol = 3 : 1], so as to obtain 0.73 g of a light yellow solid, 6-chloro-4-[(2R,3R,4S,5R)-3,4-dihydroxy-5-(hydroxymethyl)tetrahydro-2-furanyl]-3-oxo-3,4-dihydro-2-pyrazinecarboxamide.
IR(KBr)$cm^{-1}$: 1693
$^1$H-NMR(DMSO-$d_6$)δ: 3.35(3H,brs), 3.65(1H,d,J=12.0Hz), 3.8-3.9(1H,m), 3.9-4.0(3H,m), 5.81(1H,s), 7.92(1H,brs), 8.44(1H,brs), 8.70(1H,s).

Reference Example 8

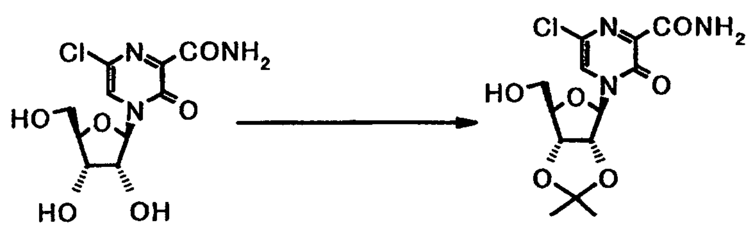

0.3 g of 6-chloro-4-[(2R,3R,4S,5R)-3,4-dihydroxy-5-(hydroxymethyl)tetrahydro-2-furanyl]-3-oxo-3,4-dihydro-2-pyrazinecarboxamide was dissolved in a mixed solvent of 0.6 mL of acetone and 1.5 mL of N,N-dimethylformamide, and then, 3 mL of 2,2-dimethoxypropane and 0.12 g of p-toluenesulfonic acid pyridinium salts were successively added thereto, followed by stirring at 50°C for 5 hours. After cooling, a mixed solvent of 5 mL of ethyl acetate and 5 mL of water was added thereto, and the organic layer was separated. The layer was washed with water and then with a saturated saline solution, and it was then dried with anhydrous magnesium sulfate, and thereafter, the solvent was removed under reduced pressure. The obtained residue was purified by silica gel column chromatography [eluant; chloroform : methanol = 10 : 1], so as to obtain 0.18 g of a light yellow solid, 4-[(3aR, 4R, 6R, 6aR)-6-(hydroxymethyl)-2,2-dimethyltetrahydrofuro[3,4-d][1,3]dioxol-4-yl]-6-chloro-3-oxo-3,4-dihydro-2-pyrazinecarboxamide.
IR(KBr)$cm^{-1}$: 1700
$^1$H-NMR(DMSO-$d_6$)δ: 1.29(3H,s), 1.50(3H,s), 3.5-3.6(1H,m), 3.7-3.8(1H,m), 4.3-4.4(1H,m), 4.74(1H,dd,J=2.9,6.1Hz), 4.88(1H,dd,J=2.0,6.1Hz), 5.37(1H,t,J=4.6Hz), 5.95(1H,d,J=1.7Hz), 7.93(1H,brs), 8.32(1H,s), 8.41(1H,brs).

Reference Example 9

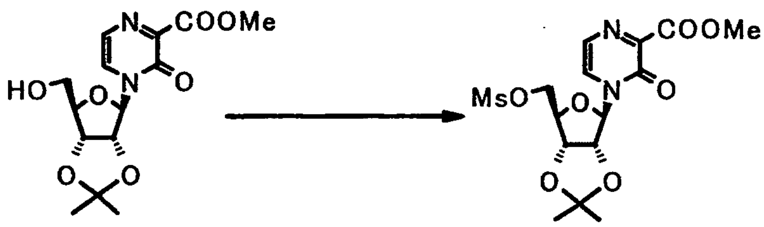

1.0 g of methyl 4-[(3aR,4R,6R,6aR)-6-(hydroxymethyl)-2,2-dimethyltetrahydrofuro[3,4-d][1,3]dioxol-4-yl]-3-oxo-3,4-dihydro-2-pyrazinecarboxylate was dissolved in 5.0 mL of pyridine, and 0.36 mL of methanesulfonyl chloride was then added thereto at 10°C, followed by stirring at room temperature for 0.5 hours. The reaction mixture was poured into a mixed solution of 20 mL of ethyl acetate and 20 mL of water. The organic layer was separated, and the aqueous layer was extracted with 20 mL of ethyl acetate 3 times. It was then combined with the organic layer. The mixture was washed with a saturated saline solution and then dried with anhydrous magnesium sulfate. Thereafter, the solvent was removed under reduced pressure, so as to obtain 1.2 g of a colorless oil product, methyl 4-[(3aR, 4R, 6R, 6aR)-2,2-dimethyl-6-[[(methylsulfonyl)oxy]methyl]tetrahydrofuro[3,4-d][1,3]dioxol-4-yl]-3-oxo-3,4-dihydro-2-pyrazinecarboxylate.
IR(KBr)$cm^{-1}$: 1734, 1669
$^1$H-NMR($CDCl_3$)δ: 1.35(3H,s), 1.58(3H,s), 3.02(3H,s), 3.98(3H,s), 4.51(2H,s), 4.8-5.2(3H,m), 5.73(1H,brs), 7.43(2H, brs)

Reference Example 10

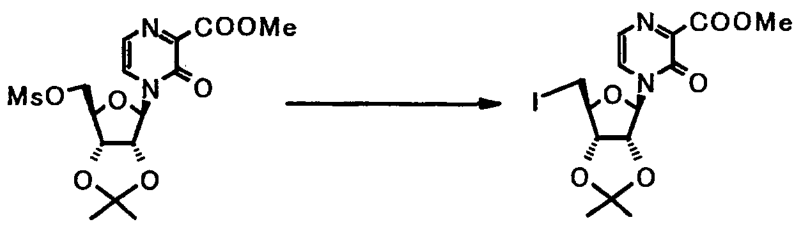

1.2 g of methyl 4-[(3aR, 4R, 6R, 6aR)-2,2-dimethyl-6-[[(methylsulfonyl)oxy]methyl]tetrahydrofuro[3,4-d][1,3] dioxol-4-yl]-3-oxo-3,4-dihydro-2-pyrazinecarboxylate was dissolved in 12 mL of acetone. 2.3 g of sodium iodide was added thereto, and the mixture was heated under reflux for 2 hours. The reaction mixture was cooled to room temperature, and it was then poured into a mixed solution of 20 mL of ethyl acetate and 20 mL of water. The organic layer was separated, and the aqueous layer was extracted with 20 mL of ethyl acetate. It was then combined with the organic layer. The mixture was successively washed with a sodium thiosulfate aqueous solution and then with a saturated saline solution, and it was then dried with anhydrous magnesium sulfate. Thereafter, the solvent was removed under reduced pressure. The obtained residue was purified by silica gel column chromatography [eluant; toluene : ethyl acetate = 2 : 1], so as to obtain 1.0 g of a yellow oil product, methyl 4-[(3aR, 4R, 6S, 6aR)-6-(iodomethyl)-2,2-dimethyltetrahydrofuro[3,4-d][1,3]dioxol-4-yl]-3-oxo-3,4-dihydro-2-pyrazinecarboxylate.
IR(KBr)$cm^{-1}$: 1734,1670,1654
$^1$H-NMR($CDCl_3$)δ: 1.36(3H,s), 1.59(3H,s), 3.3-3.7(2H,m), 3.98(3H,s), 4.3-4.5(1H,m), 4.9-5.1(2H,m), 5.76(1H,d, J=1.7Hz), 7.5-7.6(2H,m)

Reference Example 11

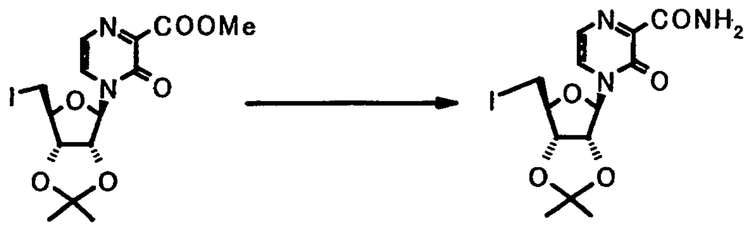

0.55 g of methyl 4-[(3aR, 4R, 6S, 6aR)-6-(iodomethyl)-2,2-dimethyltetrahydrofuro[3,4-d][1,3]dioxol-4-yl]-3-oxo-3,4-dihydro-2-pyrazinecarboxylate was dissolved in 5 mL of methanol, and ammonia gas was introduced therein under ice cooling for saturation. After stirring at the same temperature for 1 hour, the solvent was removed under reduced pressure. Diisopropyl ether was added to the obtained residue, and the precipitate was collected by filtration, so as to obtain 0.45 g of a yellow solid, 4-[(3aR,4R,6S,6aR)-6-(iodomethyl)-2,2-dimethyltetrahydrofuro[3,4-d][1,3]dioxol-4-yl]-3-oxo-3,4-dihydro-2-pyrazinecarboxamide.
IR(KBr)$cm^{-1}$: 1684, 1654
$^1$H-NMR(DMSO-$d_6$)δ: 1.30(3H,s), 1.51(3H,s), 3.3-3.5(2H,m), 4.3-4.4(1H,m), 4.79(1H,dd,J=3.6,6.4Hz), 5.13(1H,dd, J=1.2,6.0Hz), 6.00(1H,d,J=1.6Hz), 7.53(1H,d,J=4.4Hz), 7.76(1H,brs), 7.90(1H,d,J=4.4Hz), 8.20(1H,brs)

Reference Example 12

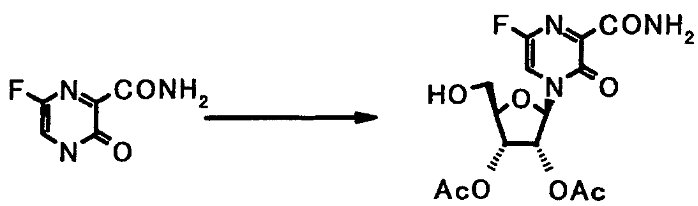

5.3 g of 6-fluoro-3-hydroxy-2-pyrazinecarboxamide was suspended in 53 mL of acetonitrile under nitrogen current, and 8.4 mL of N,O-bis(trimethylsilyl) acetamide was added thereto under ice cooling, followed by stirring at room temperature for 1.5 hours. 53 mL of an acetonitrile solution containing 9.4 g of (2R, 3R, 4R)-4,5-bis(acetyloxy)-2-(hydroxymethyl)tetrahydro-3-furanyl acetate that had been separately prepared by the method described in Carbohydrate Research (Carbohydr. Res.), Vol. 203, No. 9, pp. 324 to 329 (1990), and 7.2 mL of tin(IV) chloride were successively added to the reaction mixture under ice cooling, and the thus obtained mixture was stirred at room temperature for 20 minutes. The reaction mixture was poured into a mixed solution of 100 mL of ethyl acetate and 300 mL of a saturated sodium bicarbonate aqueous solution. The organic layers were separated, and the aqueous layer was then extracted with 700 mL of ethyl acetate. Such organic layers were combined, and the organic layers were then dried with anhydrous magnesium sulfate. Thereafter, the solvent was removed under reduced pressure. The obtained residue was dissolved in 200 mL of methanol, and 100 mL of an 80% acetic acid aqueous solution was added thereto, followed by stirring at room temperature for 2 hours. The solvent was removed under reduced pressure, and the obtained residue was purified by silica gel column chromatography [eluant; chloroform: methanol = 40 : 1]. Thereafter, chloroform and diisopropyl ether were added thereto, and the mixture was collected by filtration, so as to obtain 9.3 g of a light yellow solid, (2R, 3R, 4R, 5R)-4-(acetyloxy)-2-[3-(aminocarbonyl)-5-fluoro-2-oxo-1(2H)-pyrazinyl]-5-(hydroxymethyl)tetrahydro-3-furanyl acetate.
IR(KBr)$cm^{-1}$: 1752,1686
$^1$H-NMR(DMSO-$d_6$)δ: 2.04(3H,s), 2.10(3H,s), 3.64(1H,ddd,J=2.5,5.0,13Hz), 3.86(1H,ddd,J=2.5,5.0,13Hz), 4.29(1H, d,J=6.0Hz), 5.35(1H,t,J=6.0Hz), 5.49(1H,dd,J=3.0,5.0Hz), 5.65(1H,t,J=5.0Hz), 6.11(1H,d,J=3.0Hz), 7.96(1H,brs), 8.42(1H,d,J=5.0Hz), 8.49(1H,brs)

Reference Example 13

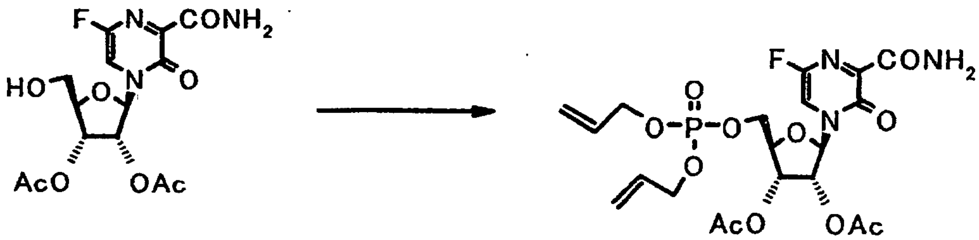

1.5 g of (2R,3R,4R,5R)-4-(acetyloxy)-2-[3-(aminocarbonyl)-5-fluoro-2-oxo-1(2H)-pyrazinyl]-5-(hydroxymethyl)tetrahydro-3-furanyl acetate and 0.84 g of 1H-tetrazole were dissolved in 30 mL of acetonitrile under nitrogen current. Thereafter, 20 mL of an acetonitrile solution containing 1.4 mL of diallyl diisopropyl phosphoramidite was added thereto under ice cooling, and the mixture was stirred for 20 minutes. 10 mL of an acetonitrile solution containing 1.4 g of m-chloroperbenzoic acid was added to the reaction mixture, followed by stirring for 10 minutes. 60 mL of ethyl acetate was added to the reaction mixture, and the obtained mixture was then poured into 60 mL of water. The organic layers were separated, and the aqueous layer was extracted with 90 mL of ethyl acetate. The organic layers were combined, and 30 mL of water was added thereto. The mixture was adjusted to pH 8 by addition of a saturated sodium bicarbonate aqueous solution, and then the aqueous layer was separated. The organic layer was washed with a saturated saline solution and then dried with anhydrous magnesium sulfate, and thereafter, the solvent was removed under reduced pressure. The obtained residue was purified by silica gel column chromatography [eluant; chloroform : methanol = 40 : 1], so as to obtain 1.3 g of a yellow solid, (2R,3R,4R,5R)-4-(acetyloxy)-2-[3-(aminocarbonyl)-5-fluoro-2-oxo-1(2H)-pyrazinyl]-5-([[bis(allyloxy)phosphoryl]oxy]methyl)-tetrahydro-3-furanyl acetate.
IR(KBr)$cm^{-1}$: 1753, 1694,
$^1$H-NMR($CDCl_3$)δ: 2.11(3H,s), 2.15(3H,s), 4.32-4.35(1H,m), 4.47-4.52(2H,m), 4.58-4.64(4H,m), 5.27(2H,dt, J=1.0,10.5Hz), 5.37-5.44(4H,m), 5.90-6.00(2H,m), 6.28(1H,d,J=4.0Hz), 6.32(1H,brs), 7.99(1H,d,J=6.0Hz), 9.02(1H, brs)

Reference Example 14

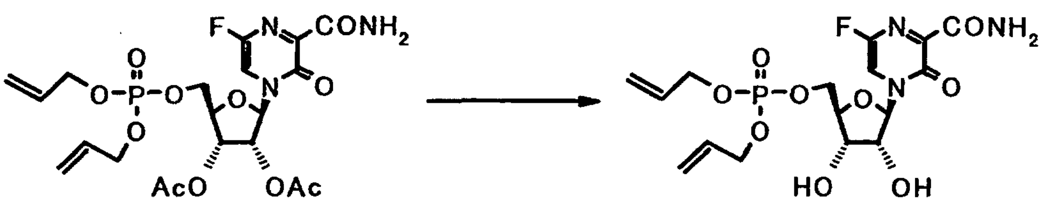

0.23 g of (2R, 3R, 4R, 5R)-4-(acetyloxy)-2-[3-(aminocarbonyl)-5-fluoro-2-oxo-1(2H)-pyrazinyl]-5-([[bis(allyloxy)phosphoryl]oxy]methyl)tetrahydro-3-furanyl acetate was dissolved in 4.0 mL of methanol, and 0.17 g of a 28% sodium methoxide methanol solution was added thereto under ice cooling, followed by stirring for 5 minutes. 0.15 mL of acetic acid was added thereto, and the solvent was removed under reduced pressure. 1.0 g of (2R, 3R, 4R, 5R)-4-(acetyloxy)-2-[3-(aminocarbonyl)-5-fluoro-2-oxo-1(2H)-pyrazinyl]-5-([[bis(allyloxy)phosphoryl]oxy]methyl)tetrahydro-3-furanyl acetate was subjected to the same above reaction. The reaction mixtures were combined, and the obtained mixture was purified by silica gel column chromatography [eluant; chloroform : methanol = 40 : 1], so as to obtain 0.35 g of a yellow solid, [(2R, 3S, 4R, 5R)-5-[3-(aminocarbonyl)-5-fluoro-2-oxo-1(2H)-pyrazinyl]-3,4-dihydroxytetrahydro-2-furanyl] methyl diallyl phosphate.
IR(KBr)$cm^{-1}$: 1684
$^1$H-NMR(DMSO-$d_6$,$D_2O$)δ: 3.1-4.7(9H,m), 5.1-5.5(4H,m), 5.7-6.2(3H,m), 7.94(1H,d,J=6.0Hz)

Reference Example 15

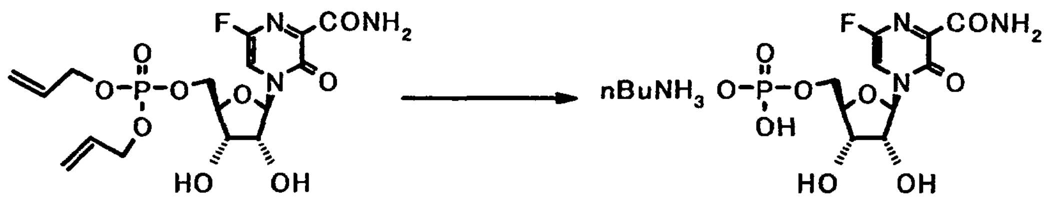

0.82 g of [(2R, 3S, 4R, 5R)-5-[3-(aminocarbonyl)-5-fluoro-2-oxo-1(2H)-pyrazinyl]-3,4-dihydroxytetrahydro-2-furanyl] methyl diallyl phosphate was dissolved in a mixed solution of 8.2 mL of methanol and 8.2 mL of tetrahydrofuran under nitrogen current. 0.11 g of tetrakis triphenyl phosphine palladium (0) and 0.28 g of triphenyl phosphine were successively added thereto, and the mixture was stirred at room temperature for 30 minutes. 1.9 mL of a tetrahydrofuran solution containing 0.68 mL of formic acid and 8.2 mL of a tetrahydrofuran solution containing 1.1 mL of n-butylamine were successively added to the reaction mixture under water cooling, and the obtained mixture was stirred at a temperature between 30°C and 35°C for 1 hour, and at a temperature between 40°C and 45°C for 2 hours. The reaction mixture was diluted with 10 mL of water, and the organic solvent was then removed under reduced pressure. The obtained aqueous solution was washed with 20 mL of chloroform, and the washing was extracted with 30 mL of water. All the aqueous layers were combined, and the solvent was removed under reduced pressure. The obtained residue was purified by reverse phase silica gel column chromatography [eluant; water], so as to obtain 0.29 g of n-butyl ammonium salts of a yellow solid, [(2R, 3S, 4R, 5R)-5-[3-(aminocarbonyl)-5-fluoro-2-oxo-1(2H)-pyrazinyl]-3,4-dihydroxytetrahydro-2-furanyl] methyl dihydrogen phosphate.
IR(KBr)$cm^{-1}$: 1685
$^1$H-NMR(DMSO-$d_6$)δ: 0.75-0.90(3H,m), 1.25-1.40(2H,m), 1.45-1.70(2H,m), 2.70-2.80(2H,m), 3.3-4.7(8H,m), 5.33(1H,d,J=10Hz), 5.42(1H,d,J=17Hz), 5.90(2H,brs), 7.95(1H,brs), 8.34(1H,d,J=5.0Hz), 8.63(1H,brs)

Reference Example 16

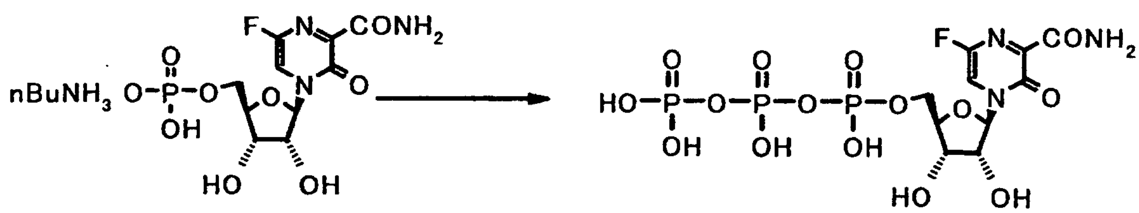

0.21 g of n-butyl ammonium salts of [(2R, 3S, 4R, 5R)-5-[3-(aminocarbonyl)-5-fluoro-2-oxo-1(2H)-pyrazinyl]-3,4-dihydroxytetrahydro-2-furanyl] methyl dihydrogen phosphate was suspended in a mixed solution of 4.2 mL of acetonitrile and 8.4 mL of N,N-dimethylformamide, and thereafter, 0.15 g of 1,1'-carbonyldiimidazole was added thereto, followed by stirring at room temperature for 2 hours. Thereafter, 19 μL of methanol was added to the reaction mixture, followed by stirring for 30 minutes. Thereafter, 2.0 mL of an N,N-dimethylformamide solution containing 0.86 g of tri-n-butyl ammonium pyrophosphate was added to the reaction mixture, and the obtained mixture was further stirred for 14 hours. The solvent was removed under reduced pressure, and the obtained residue was successively purified by ion exchange column chromatography [eluant; 0.10 mol/L triethylammonium bicarbonate solution] and reverse column chromatography [eluant; water]. 0.90 mL of methanol was added to the obtained solid, and 4.5 mL of an acetone solution containing 0.17 g of sodium perchlorate was added thereto. The precipitate was centrifuged and then washed with acetone, so as to obtain 60 mg of sodium salts of a light yellow solid, [[2R, 3S, 4R, 5R]-5-[3-(aminocarbonyl)-5-fluoro-2-oxo-1(2H)-pyrazinyl]-3,4-dihydroxytetrahydro-2-furanyl] methyl triphosphate. IR(KBr)$cm^{-1}$: 3422,1686,1252,1108
$^1$H-NMR($D_2O$)δ: 4.3-4.5(5H,m), 6.09(1H,s), 8.41(1H,d,J=5.1Hz)

Reference Example 17

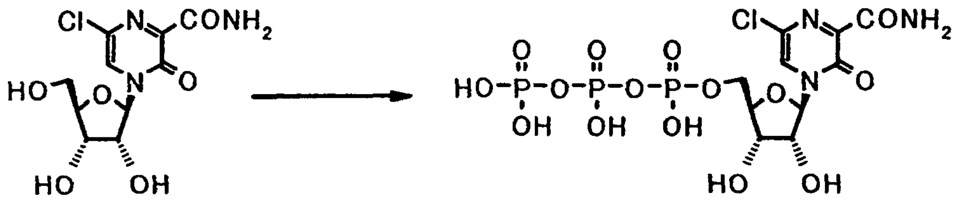

0.12 g of 6-chloro-4-[(2R,3R,4S,5R)-3,4-dihydroxy-5-(hydroxymethyl)tetrahydro-2-furanyl]-3-oxo-3,4-dihydro-2-pyrazinecarboxamide was suspended in 1.2 mL of trimethyl phosphate, and 38 μL of phosphorus oxychloride was added thereto under ice cooling, followed by stirring at the same temperature for 1 hour. The reaction mixture was poured into 3.0 mL of a dimethylformamide solution containing 0.30 mL of n-tributylamine and 0.72 g of n-tributyl ammonium pyrophosphate under ice cooling, and the obtained solution was stirred at the same temperature for 5 minutes. 10 mL of a 0.1 mol/L triethyl ammonium bicarbonate solution and 10 mL of water were successively added to the reaction mixture, and the obtained mixture was purified by ion exchange column chromatography [eluant; 0.07 mol/L triethyl ammonium bicarbonate solution] and reverse phase silica gel column chromatography [eluant; water], so as to obtain 41 mg of a solid consisting of triethyl ammonium salts of {(2R, 3S, 4R, 5R)-5-[3-(aminocarbonyl)-5-chloro-2-oxo-1(2H)-pyrazinyl]-3,4-dihydroxytetrahydro-2-furanyl} methyl triphosphate. 41 mg of the obtained triethyl ammonium salts of {(2R, 3S, 4R, 5R)-5-[3-(aminocarbonyl)-5-chloro-2-oxo-1(2H)-pyrazinyl]-3,4-dihydroxytetrahydro-2-furanyl} methyl triphosphate was dissolved in 0.43 mL of methanol, and thereafter, 2.2 mL of an acetone solution containing 78 mg of sodium perchlorate was added thereto. The obtained solid was centrifuged and then washed with 2.2 mL of acetone, so as to obtain 26 mg of sodium salts of a white solid, {(2R, 3S, 4R, 5R)-5-[3-(aminocarbonyl)-5-chloro-2-oxo-1(2H)-pyrazinyl]-3,4-dihydroxytetrahydro-2-furanyl}methyl triphosphate.
IR(KBr)$cm^{-1}$: 1700, 1654
$^1$H-NMR($D_2O$)δ: 4.25-4.5(5H,m), 6.08(1H,s), 8.44(1H,s)

Reference Example 18

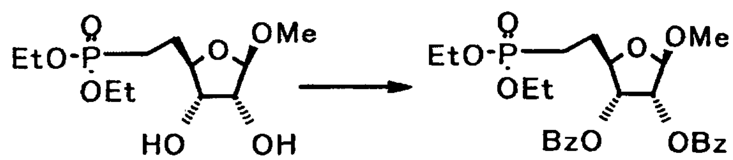

43 mg of diethyl 2-[(2R, 3S, 4R, 5R)-3,4-dihydroxy-5-methoxytetrahydro-2-furanyl] ethyl phosphonate that had been prepared by the method described in Journal of Chemical Society Chemical Communication (J. Chem. Soc., Chem. Commun.), pp. 40 to 41 (1989) and 82 μL of triethylamine were dissolved in 1 mL of dichloromethane. Thereafter, 0.21 mL of benzoyl chloride and 10 mg of 4-dimethylaminopyridine were successively added thereto, and the mixture was stirred at room temperature for 1 hour. 0.80 g of diethyl 2-[(2R, 3S, 4R, 5R)-3,4-dihydroxy-5-methoxytetrahydro-2-furanyl] ethyl phosphonate was treated in the same above manner. Thereafter, 10 mL of water was added to the obtained reaction mixture, the organic layers were separated, and the aqueous layer was extracted twice with 20 mL of chloroform. The organic layers were combined and then successively washed with water and with a saturated saline solution. Thereafter, it was dried with anhydrous magnesium sulfate, and the solvent was removed under reduced pressure. The obtained residue was purified by silica gel column chromatography [eluant; chloroform], so as to obtain 1.38 g of a colorless oil product, (2R, 3R, 4R, 5R)-4-(benzoyloxy)-5-[2-(diethoxyphosphoryl)ethyl]-2-methoxytetrahydro-3-furanyl benzoate.
IR(neat)$cm^{-1}$: 1729
$^1$H-NMR($CDCl_3$)δ: 1.3-1.35(6H,m), 1.8-2.2(4H,m), 3.46(3H,s), 4.0-4.2(4H,m), 4.3-4.45(1H,m), 5.10(1H,s), 5.52(1H,t, J=5.1Hz), 5.59(1H,d,J=5.1Hz), 7.33(2H,t,J=7.8Hz), 7.41(2H,t,J=7.8Hz), 7.5-7.6(2H,m), 7.90(2H,d,J=7.3Hz), 7.99(2H, d,J=7.3Hz)

Reference Example 19

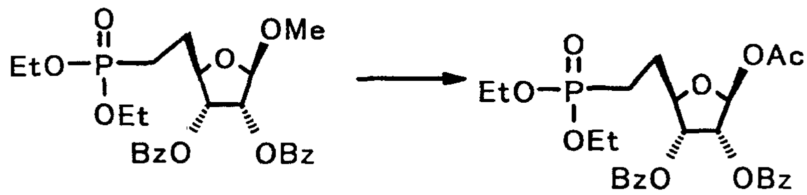

1.34 g of (2R, 3R, 4R, 5R)-4-(benzoyloxy)-5-[2-(diethoxyphosphoryl)ethyl]-2-methoxytetrahydro-3-furanyl benzoate and 1.30 mL of acetic anhydride were dissolved in 20 mL of acetic acid, and thereafter, 0.13 mL of concentrated sulfuric acid was added thereto under ice cooling. After raising the temperature, the mixture was left at room temperature for 16 hours. The obtained reaction mixture was poured into a mixed solution of 50 mL of ethyl acetate, 50 mL of ice and 100 mL of a saturated sodium bicarbonate aqueous solution. The organic layers were separated, and the aqueous layer was extracted twice with 50 mL of ethyl acetate. The organic layers were combined and then washed with a saturated saline solution. Thereafter, they were dried with anhydrous magnesium sulfate, and the solvent was removed under reduced pressure. The obtained residue was purified by silica gel column chromatography [eluant; toluene : ethyl acetate = 1 : 1], so as to obtain 1.19 g of a colorless oil product, (2S, 3R, 4R, 5R)-2-(acetyloxy)-4-(benzoyloxy)-5-[2-(diethoxyphosphoryl)ethyl] tetrahydro-3-furanyl benzoate.
IR(neat)$cm^{-1}$: 1729
$^1$H-NMR($CDCl_3$)δ: 1.3-1.35(6H,m), 1.8-2.2(4H,m), 2.11,2.16(3H,2s), 4.05-4.2(4H,m), 4.45-4.5(1H,m), 5.45-5.7(2H,m), 6.37,6.62(1H,2d,J=1.0,3.9Hz), 7.3-7.5(4H,m), 7.5-7.6(2H,m), 7.85-7.9(2H,m), 7.95-8.05(2H,m)

Reference Example 20

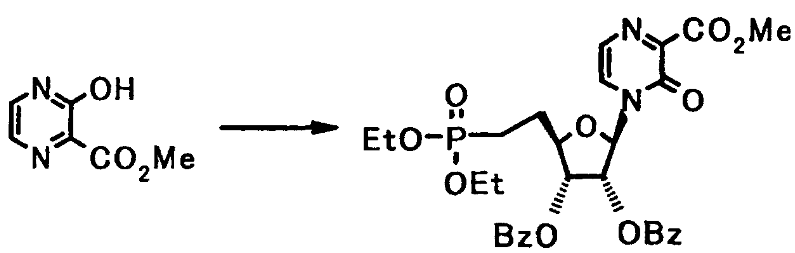

50 mg of methyl 3-hydroxy-2-pyrazinecarboxylate was suspended in 1.6 mL of 1,1,1,3,3,3-hexamethyldisilazane, and the solution was heated under reflux for 1 hour under nitrogen atmosphere. After standing to cool, the solvent was removed under reduced pressure, and an acetonitrile solution containing 0.17 g of (2S, 3R, 4R, 5R)-2-(acetyloxy)-4-(benzoyloxy)-5-[2-(diethoxyphosphoryl)ethyl]tetrahydro-3-furanyl benzoate was added thereto. Thereafter, the solvent was removed under reduced pressure. The obtained residue was suspended in 2.00 mL of acetonitrile under nitrogen atmosphere, and thereafter, 67 μL of tin(IV) chloride was added thereto under ice cooling, followed by leaving at room temperature for 24 hours. 300 mg of methyl 3-hydroxy-2-pyrazinecarboxylate was treated in the same above manner, and the obtained reaction mixture was poured into a mixed solution of 50 mL of ethyl acetate, 50 mL of ice and 100 mL of a saturated sodium bicarbonate aqueous solution. The precipitate was removed by filtration, the organic layers were separated, and the aqueous layer was extracted with 50 mL of ethyl acetate. The organic layers were combined, and the obtained organic layers were washed with a saturated saline solution and then dried with anhydrous magnesium sulfate. The solvent was then removed under reduced pressure. The obtained residue was purified by silica gel column chromatography [eluant; ethyl acetate : methanol = 100 : 1], so as to obtain 0.76 g of a colorless oil product, methyl 4-{(2R, 3R, 4R, 5R)-3,4-bis(benzoyloxy)-5-[2-(diethoxyphosphoryl)ethyl]tetrahydro-2-furanyl}-3-oxo-3,4-dihydro-2-pyrazinecarboxylate.

IR(neat)$cm^{-1}$: 1734, 1670
$^1$H-NMR($CDCl_3$)δ: 1.3-1.35(6H,m), 1.85-2.3(4H,m), 3.97(3H,s), 4.05-4.2(4H,m), 4.45-4.55(1H,m), 5.65(1H,t,J=6.5Hz), 5.74(1H,dd,J=3.6,5.9Hz), 6.24(1H,d,J=3.6Hz), 7.3-7.4(4H,m), 7.5-7.6(4H,m), 7.85-7.95(4H,m).

Reference Example 21

6-fluoro-3-hydroxy-2-[2-$^{14}$C] pyrazinecarboxamide (radiochemical purity: 99.0%) was produced from diethyl [2-$^{14}$C] malonate as a starting material by known methods, methods equivalent thereto, or a combined use thereof. Such a production method is described in International Patent Publication WO00/10569, for example.

Reference Example 22

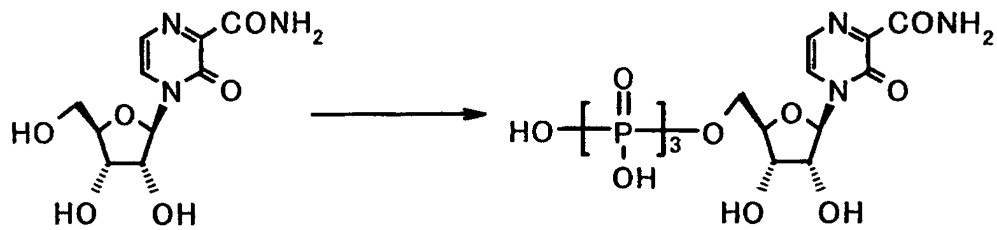

140 mg of sodium salts of a white solid, {(2R, 3S, 4R, 5R)-5-[3-(aminocarbonyl)-2-oxo-1(2H)-pyrazinyl]-3,4-dihydroxytetrahydro-2-furanyl} methyl triphosphate was obtained from 136 mg of 4-[(2R, 3R, 4S, 5R)-3,4-dihydroxy-5-(hydroxymethyl)tetrahydro-2-furanyl]-3-oxo-3,4-dihydro-2-pyrazinecarboxamide in the same manner as in Reference Example 17.
$^1$H-NMR($D_2O$)δ: 4.30-4.39(4H,m), 4.45-4.48(1H,m), 6.14(1H,s), 7.86(1H,d,J=3.6Hz), 8.34(1H,d,J=3.6Hz)

Reference Example 23

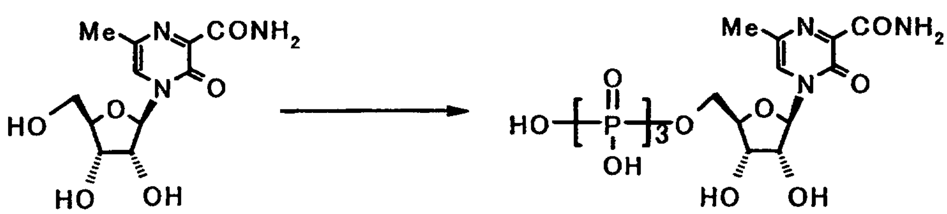

0.06 g of sodium salts of a white solid, {(2R, 3S, 4R, 5R)-5-[3-(aminocarbonyl)-5-methyl-2-oxo-1(2H)-pyrazinyl]-3,4-dihydroxytetrahydro-2-furanyl} methyl triphosphate was obtained from 0.1 g of 4-[(2R, 3R, 4S, 5R)-3,4-dihydroxy-5-(hydroxymethyl)tetrahydro-2-furanyl]-6-methyl-3-oxo-3,4-dihydro-2-pyrazinecarboxamide in the same manner as in Reference Example 17.
IR(KBr)$cm^{-1}$: 1684,1654
$^1$H-NMR($D_2O$)δ: 2.44(3H,s), 4.31-4.47(5H,m), 6.12(1H,s), 8.20(1H,s)

Reference Example 24

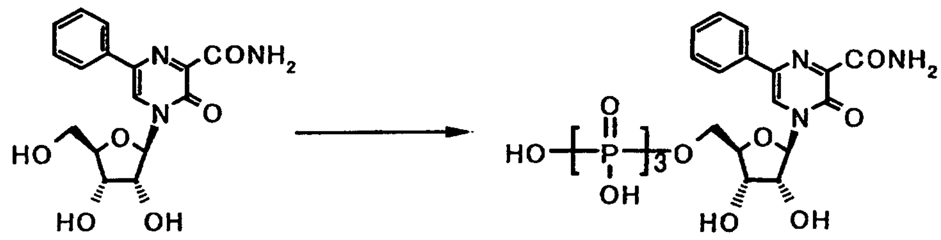

0.02 g of sodium salts of a yellow solid, {(2R, 3S, 4R, 5R)-5-[3-(aminocarbonyl)-2-oxo-5-phenyl-1(2H)-pyrazinyl]-3,4-dihydroxytetrahydro-2-furanyl} methyl triphosphate was obtained from 0.06 g of 4-[(2R, 3R, 4S, 5R)-3,4-dihydroxy-5-(hydroxymethyl)tetrahydro-2-furanyl]-3-oxo-6-phenyl-3,4-dihydro-2-pyrazinecarboxamide in the same manner as in Reference Example 17.
IR(KBr)$cm^{-1}$: 1684,1654
$^{1}$H-NMR($D_2O$)δ: 4.07-4.41(5H,m), 6.22(1H,s), 7.47-7.60(3H,m), 7.99(2H,d,J=7.8Hz), 8.58(1H,s)

Reference Example 25

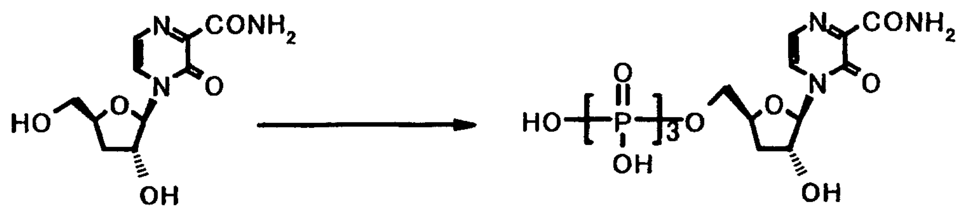

146 mg of sodium salts of a white solid, {(2S, 4R, 5R)-5-[3-(aminocarbonyl)-2-oxo-1(2H)-pyrazinyl]-4-hydroxytetrahydro-2-furanyl} methyl triphosphate was obtained from 128 mg of 4-[(2R, 3R, 5S)-3-hydroxy-5-(hydroxymethyl) tetrahydro-2-furanyl]-3-oxo-3,4-dihydro-2-pyrazinecarboxamide in the same manner as in Reference Example 17.
$^{1}$H-NMR($D_2O$)δ: 2.04-2.18(2H,m), 4.23-4.29(1H,m), 4.50-4.58(2H,m), 4.78-4.88(1H,m), 6.03(1H,s), 7.86(1H,d, J=3.8Hz), 8.41(1H,d,J=3.8Hz)

Reference Example 26

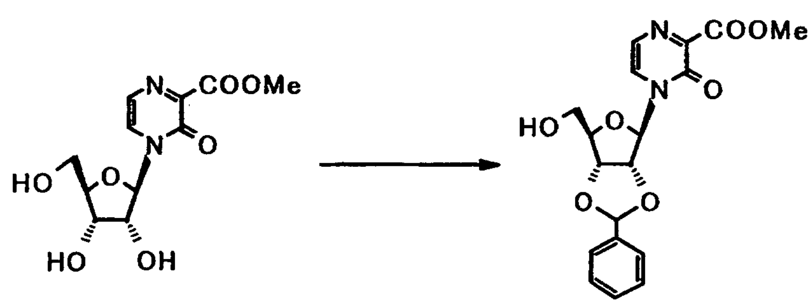

0.9 g of methyl 4-[(2R, 3R, 4S, 5R)-3,4-dihydroxy-5-(hydroxymethyl)tetrahydro-2-furanyl]-3-oxo-3,4-dihydro-2-pyrazinecarboxylate was suspended in 9 mL of N,N-dimethylacetamide. 2.3 mL of benzaldehyde dimethylacetal and

160 mg of pyridinium-p-toluenesulfonate were added thereto, and the mixture was stirred at 65°C for 7 hours. Subsequently, the reaction solution was poured into a mixed solution of 10 mL of ethyl acetate and 5 mL of water. The deposited solid was collected by filtration, so as to obtain 0.24 g of a white solid, methyl 4-[(3aR, 4R, 6R, 6aR)-6-(hydroxymethyl)-2-phenyltetrahydrofuro[3,4-d][1,3]dioxol-4-yl]-3-oxo-3,4-dihydro-2-pyrazinecarboxylate. Thereafter, the filtrate was separated, and the obtained organic layer was successively washed with 5 mL of water and 5 mL of a saturated sodium chloride aqueous solution, and then dried with anhydrous magnesium sulfate. The solvent was removed under reduced pressure, and the residue was washed with ethyl acetate, so as to obtain 0.40 g of a white solid, methyl 4-[(3aR, 4R, 6R, 6aR)-6-(hydroxymethyl)-2-phenyltetrahydrofuro[3,4-d][1,3]dioxol-4-yl]-3-oxo-3,4-dihydro-2-pyrazinecarboxylate.
IR(KBr)$cm^{-1}$: 3440, 1731
$^1$H-NMR(DMSO-$d_6$)δ: 3.61-3.71(2H,m), 3.83(3H,s), 4.50-4.53(1H,m), 4.86(1H,dd,J=2.2, 6.6Hz), 5.01(1H,dd,J=2.0, 6.3Hz), 5.23(1H,t,J=4.9Hz), 5.95(1H,s), 6.07(1H,d,J=2.0Hz), 7.45-7.47(3H,m), 7.49(1H,d,J=4.4Hz), 7.54-7.57(2H,m), 8.09(1H,d,J=4.4Hz)

Reference Example 27

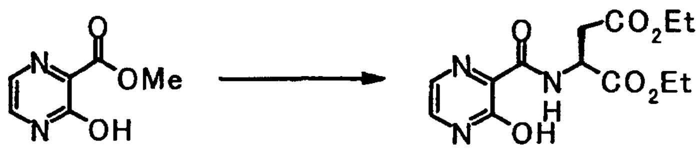

0.30 g of methyl 3-hydroxy-2-pyrazinecarboxylate was dissolved in 1.5 mL of dimethyl sulfoxide, and thereafter, 0.70 mL of triethylamine and 0.54 g of L-aspartic acid diethyl ester hydrochloride were successively added thereto, followed by stirring at room temperature for 8 hours. After chloroform and water were added to the reaction solution, the mixture was adjusted to pH 2 with 2 mol/L hydrochloric acid, and the organic layer was separated. The obtained organic layer was washed with water and then dried with anhydrous magnesium sulfate, and the solvent was removed under reduced pressure. Toluene and n-hexane were added to the obtained residue, and the precipitate was collected by filtration, so as to obtain 0.18 g of diethyl (2S)-2-{[(3-hydroxy-2-pyrazinyl)carbonyl]amino} butanedioate.
$^1$H-NMR($CDCl_3$)δ: 1.27(3H,t,J=7.2Hz), 1.30(3H,t,J=7Hz), 2.94(1H,dd,J=4.4,17.2Hz), 3.15(1H,dd,J=4.8,17.2Hz), 4.14-4.23(2H,m), 4.24-4.32(2H,m), 4.99-5.02(1H,m), 8.15(1H,d,J=2.6Hz), 8.40(1H,d,J=1.5Hz), 8.78(1H,d,J=5.9Hz), 12.4(1H,brs)

Reference Example 28

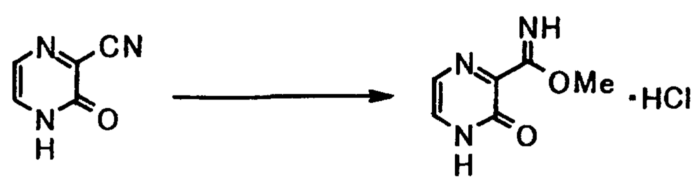

2.0 g of 3-oxo-3,4-dihydro-2-pyrazinecarbonitrile was dissolved in 20 mL of methanol, and hydrogen chloride gas was then introduced therein under ice cooling for saturation. After stirring at the same temperature for 6 hours, ethyl acetate was added thereto, and the precipitate was collected by filtration, so as to obtain 2.3 g of a yellow solid, methyl 3-oxo-3,4-dihydro-2-pyrazine carboximidate hydrochloride.
$^1$H-NMR(DMSO-$d_6$)δ: 4.27(3H,s), 7.88(1H,d,J=3.4Hz), 7.91(1H,brs), 8.07(1H,brs), 8.15(1H,d,J=3.4Hz), 8.71(1H,brs)

Reference Example 29

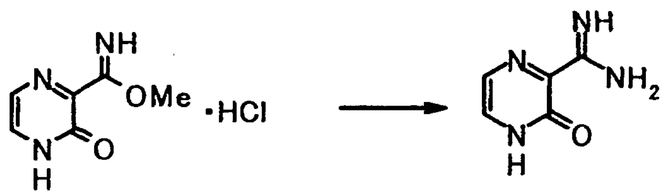

0.40 g of methyl 3-oxo-3,4-dihydro-2-pyrazine carboximidate hydrochloride was dissolved in 4 mL of a 25% ammonia water, followed by stirring at room temperature for 2 hours. Methanol was added thereto, and the deposit was collected by filtration, so as to obtain 0.21 g of a light yellow solid, 3-oxo-3,4-dihydro-2-pyrazine carboximidamide.
$^1$H-NMR(DMSO-$d_6$, $D_2O$)δ: 7.60(1H,s), 8.19(1H,s)

Reference Example 30

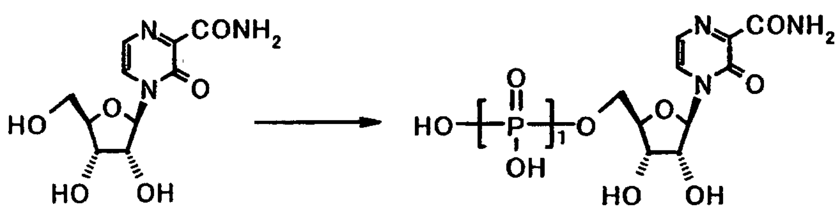

0.2 g of 4-[(2R, 3R, 4S, 5R)-3,4-dihydroxy-5-(hydroxymethyl)tetrahydro-2-furanyl]-3-oxo-3,4-dihydro-2-pyrazinecarboxamide was suspended in 4 mL of acetonitrile, and thereafter, 0.2 mL of diphosphoryl chloride was added thereto under ice cooling, followed by stirring at the same temperature for 20 minutes. The reaction solution was adjusted to pH 7 with a 1 mol/L triethyl ammonium bicarbonate solution, and it was then concentrated under reduced pressure. The obtained residue was purified by reverse phase silica gel column chromatography [eluant; water], so as to obtain 0.29 of triethyl ammonium salts of a solid, {(2R, 3S, 4R, 5R)-5-[3-(aminocarbonyl)-2-oxo-1(2H)-pyrazinyl]-3,4-dihydroxytetrahydro-2-furanyl} methyl phosphate.
$^1$H-NMR($D_2O$)δ: 1.28(9H,t,J=7.3Hz), 3.20(6H,q,J=7.3Hz), 4.15-4.20(1H,m), 4.28-4.40(4H,m), 6.11(1H,d,J=2.0Hz), 7.80(1H,d,J=4.2Hz), 8.34(1H,d,J=4.2Hz)

7 ml of an acetone solution containing 0.35 g of sodium perchlorate was added to 1.4 mL of a methanol suspension containing 0.28 g of the above triethyl ammonium salts of monophosphoric acid at room temperature, and the mixture was stirred at the same temperature for 1 hour. The deposit was collected by filtration, and it was then washed with acetone, so as to obtain 0.19 g of sodium salts of a white solid, {(2R, 3S, 4R, 5R)-5-[3-(aminocarbonyl)-2-oxo-1(2H)-pyrazinyl]-3,4-dihydroxytetrahydro-2-furanyl} methyl phosphate.
IR(KBr)$cm^{-1}$: 1662
$^1$H-NMR($D_2O$)δ: 4.15-4.19(1H,m), 4.29-4.38(4H,m), 6.12(1H,s), 7.80(1H,d,J=3.8Hz), 8.35(1H,d,J=3.8Hz)

Reference Example 31

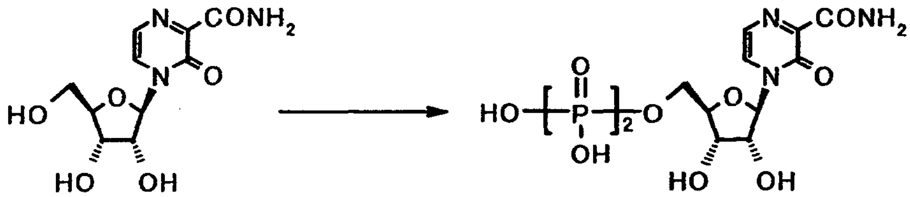

0.11 g of 4-[(2R, 3R, 4S, 5R)-3,4-dihydroxy-5-(hydroxymethyl)tetrahydro-2-furanyl]-3-oxo-3,4-dihydro-2-pyrazinecarboxamide was suspended in 2.0 mL of trimethyl phosphate, and thereafter, 0.11 mL of phosphorus oxychloride was added thereto under ice cooling, followed by stirring at the same temperature for 2 hours. 6.0 mL of a dimethylformamide solution containing 1.2 mL of tributylamine and 1.12 g of tributyl ammonium phosphate was added to the reaction mixture, and the obtained mixture was stirred at the same temperature for 1 hour. A 0.1 mol/L triethyl ammonium bicarbonate solution was added to the reaction mixture, and the obtained mixture was left at room temperature for 12 hours. The solvent was removed under reduced pressure. The obtained residue was purified by ion exchange column chromatography [eluant; 0.07 mol/L triethyl ammonium bicarbonate solution], to collect both fractions containing triethyl ammonium salts of {(2R, 3S, 4R, 5R)-5-[3-( aminocarbonyl)-2-oxo-1(2H)-pyrazinyl]-3,4-dihydroxytetrahydro-2-furanyl} methyl diphosphate, and fractions containing triethyl ammonium salts of {(2R, 3S, 4R, 5R)-5-[3-(aminocarbonyl)-2-oxo-1(2H)-pyrazinyl]-3,4-dihydroxytetrahydro-2-furanyl} methyl triphosphate, and as a result, 143 mg of a solid and 113 mg of another solid were obtained. 110 mg out of 143 mg of the obtained triethyl ammonium salts of {(2R, 3S, 4R, 5R)-5-[3-(aminocarbonyl)-2-oxo-1(2H)-pyrazinyl]-3,4-dihydroxytetrahydro-2-furanyl} methyl diphosphate was dissolved in 3.0 mL of methanol, and thereafter, 7.5 mL of an acetone solution containing 0.28 g of sodium perchlorate was added thereto. The solid was centrifuged and then washed with acetone, to obtain 64 mg of sodium salts of a white solid, {(2R, 3S, 4R, 5R)-5-[3-(aminocarbonyl)-2-oxo-1(2H)-pyrazinyl]-3,4-dihydroxytetrahydro-2-furanyl} methyl diphosphate.

IR(KBr)$cm^{-1}$: 3418, 1682, 1236, 983, 905

$^1$H-NMR($D_2O$)δ: 4.2-4.5(5H,m), 6.12(1H,s), 7.83(1H,d,J=3.7Hz), 8.35(1H,d,J=3.7Hz)

Example 1

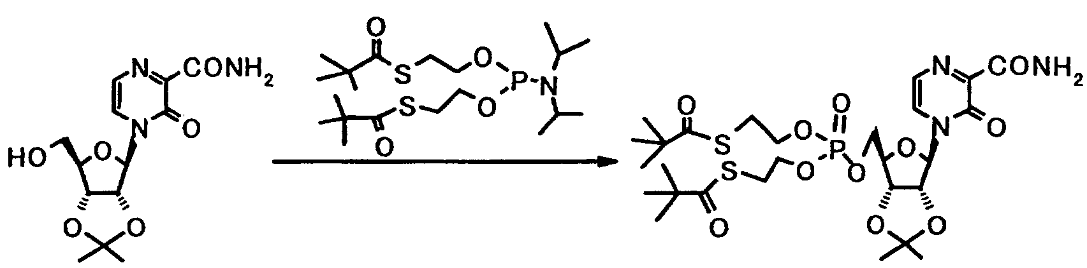

65 mg of 4-[(3aR, 4R, 6R, 6aR)-6-(hydroxymethyl)-2,2-dimethyltetrahydrofuro[3,4-d][1,3]dioxol-4-yl]-3-oxo-3,4-dihydro-2-pyrazinecarboxamide, 44 mg of 1H-tetrazole, and 10 mg of molecular sieves 4A were suspended in 2.0 mL of acetonitrile. 3.0 mL of an acetonitrile solution containing 0.16 g of bis(S-pivaloyl-2-thioethyl)-N,N-diisopropyl phosphoramidite, which had been separately prepared by the method described in Journal of Medicinal Chemistry (J. Med.Chem.) Vol. 38, No. 20, pp. 3941 to 3950 (1995), was added thereto in portions under ice cooling, and the obtained mixture was stirred at the same temperature for 40 minutes. 1.0 mL of an acetonitrile solution containing 78 mg of m-chloroperbenzoic acid was added to the reaction mixture, and the obtained mixture was further stirred at the same temperature for 10 minutes. 10 mL of ethyl acetate and 10 mL of water were added to the reaction mixture. The organic layers were separated, and the aqueous layer was extracted with 20 mL of ethyl acetate. All the organic layers were combined, and the combined layer was washed with a saturated sodium bicarbonate aqueous solution and then dried with anhydrous magnesium sulfate. The solvent was then removed under reduced pressure. The obtained residue

was purified by silica gel column chromatography [eluant; chloroform : methanol = 50 : 1], to obtain 68 mg of a yellow oil product, 2,2-dimethyl-thiopropionic acid S-(2-{[(3aR, 4R, 6R, 6aR)-6-(3-carbamoyl-2-oxo-2H-pyrazine-1-yl)-2,2-dimethyltetrahydrofuro[3,4-d][1,3]dioxol-4-ylmethoxy]-[2-(2,2-dimethylpropionylsulfanyl)-ethoxy]-phosphoryloxy}-ethyl)-ester.
IR(neat)$cm^{-1}$: 1684
$^1$H-NMR($CDCl_3$)δ: 1.22(9H,s), 1,23(9H,s), 1.40(3H,s), 1.65(3H,s), 3.08(2H,t,J=,6.9Hz), 3.10(2H,t,J=6.8Hz), 4.04-4.12 (4H,m), 4.29-4.36(1H,m), 4.39-4.45(1H,m), 4.59-4.64(1H,m), 4.86-4.88(2H,m), 6.02(1H,brs), 6.05(1H,d,J=1.5Hz), 7.84(1H,d,J=4.3Hz), 7.87(1H,d,J=4.3Hz), 9.17(1H,brs)

Example 2

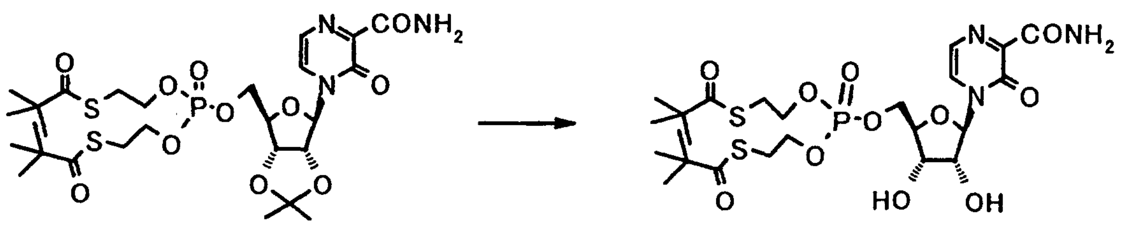

60 mg of 2,2-dimethyl-thiopropionic acid S-(2-{[(3aR, 4R, 6R, 6aR)-6-(3-carbamoyl-2-oxo-2H-pyrazine-1-yl)-2,2-dimethyl-tetrahydrofuro[3,4-d][1,3]dioxol-4-ylmethoxy]-[2-(2,2-dimethylpropionylsulfanyl)-ethoxy]-phosphoryloxy}-ethyl)-ester was dissolved in a mixed solvent of 2.4 mL of water and 2.4 mL of methanol, and thereafter, 1.2 g of a Dowex 50WX4-200 ion exchange resin (H+form) was added thereto in portions, and the mixture was stirred at room temperature for 3 hours. The reaction mixture was filtrated, the removed resin was washed with methanol, and the filtrate and the washings were combined. The organic solvent was then removed under reduced pressure. The deposit was collected by filtration, to obtain 36 mg of a white solid, 2,2-dimethyl-thiopropionic acid S-(2-[[(2R, 3S, 4R, 5R)-5-(3-carbamoyl-2-oxo-2H-pyrazine-1-yl)-3,4-dihydroxytetrahydro-furan-2-ylmethoxy]-[2-(2,2-dimethylpropionylsulfanyl)-ethoxy]-phosphoryloxy]-ethyl) ester.
IR(KBr)$cm^{-1}$: 1677, 1660
$^1$H-NMR($CDCl_3$)δ: 1.22(9H,s), 1,23(9H,s), 3.11(4H,t,J=7.0Hz), 3.67(1H,brs), 4.05-4.15(4H,m), 4.25-4.38(3H,m), 4.44-4.52(2H,m), 4.67(1H,brs), 6.04(1H,d,J=3.7Hz), 6.10(1H,brs), 7.90(1H,d,J=4.1Hz), 8.08(1H,d,J=4.1Hz), 8.95(1H, brs)

Example 3

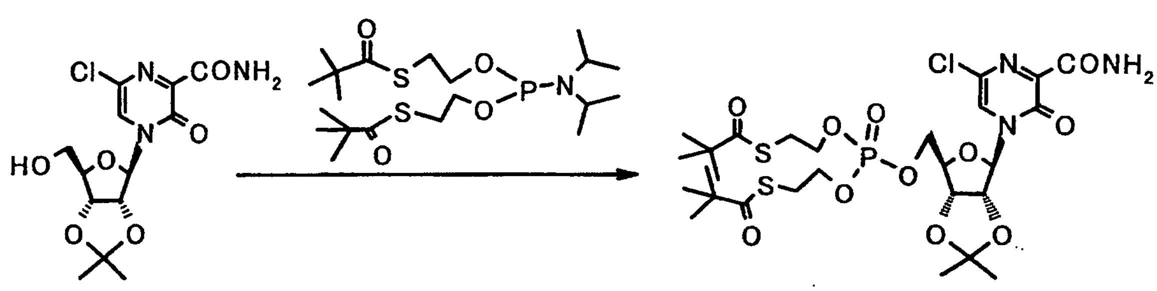

12 mg of a yellow oil product, 2,2-dimethyl-thiopropionic acid S-(2-{[(3aR, 4R, 6R, 6aR)-6-(3-carbamoyl-5-chloro-2-oxo-2H-pyrazine-1-yl)-2,2-dimethyl-tetrahydrofuro[3,4-d][1,3]dioxol-4-ylmethoxy]-[2-(2,2-dimethyl-propionylsulfanyl)-ethoxy]-phosphoryloxy}-ethyl) ester was obtained from 40 mg of 4-[(3aR,4R,6R,6aR)-6-(hydroxymethyl)-2,2-dimethyltetrahydrofuro[3,4-d][1,3]dioxol-4-yl]-6-chloro-3-oxo-3,4-dihydro-2-pyrazinecarboxamide in the same manner as in Example 1.
IR(neat)$cm^{-1}$: 1687

$^1H$-NMR($CDCl_3$)δ: 1.21(9H,s), 1,22(9H,s), 1.40(3H,s), 1.64(3H,s), 3.07(2H,dt,J=7.0,1.5Hz), 3.14(2H,t,J=6.8Hz), 4.06-4.16(4H,m), 4.34-4.39(1H,m), 4.42-4.47(1H,m), 4.63-4.65(1H,m), 4.85(1H,dd,J=6.4,2.2Hz), 4.89(1H,dd, J=6.2,2.8Hz), 6.04(1H,d,J=2.2Hz), 6.17(1H,brs), 7.98(1H,s), 9.07(1H,brs)

Example 4

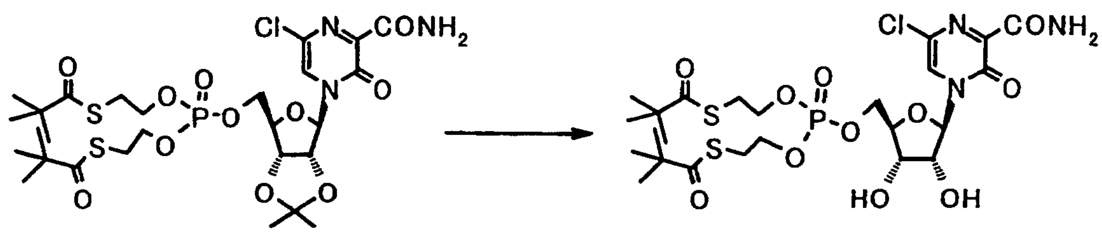

7 mg of a yellow oil product, 2,2-dimethyl-thiopropionic acid S-(2-{[(2R, 3S, 4R, 5R)-5-(3-carbamoyl-5-chloro-2-oxo-2H-pyrazine-1-yl)-3,4-dihydroxy-tetrahydro-furan-2-ylmethoxy]-[2-(2,2-dimethyl-propionylsulfanyl)-ethoxy]-phosphoryloxy}-ethyl) ester was obtained from 12 mg of 2,2-dimethyl-thiopropionic acid S-(2-{[(3aR, 4R, 6R, 6aR)-6-(3-carbamoyl-5-chloro-2-oxo-2H-pyrazine-1-yl)-2,2-dimethyl-tetrahydrofuro[3,4-d][1,3]dioxol-4-ylmethoxy] -[2-(2,2-dimethyl-propionylsulfanyl)-ethoxy]-phosphoryloxy}-ethyl) ester in the same manner as in Example 2.
IR(neat)$cm^{-1}$: 1686, 1654
$^1H$-NMR($CDCl_3$)δ: 1.21(9H,s), 1,23(9H,s), 3.00(1H,brs), 3.10-3.16(4H,m), 3.83(1H,brs), 4.05-4.20(4H,m), 4.25-4.55 (5H,m), 6.02(1H,d,J=2.4Hz), 6.40(1H,brs), 8.15(1H,s), 8.81(1H,brs)

Example 5

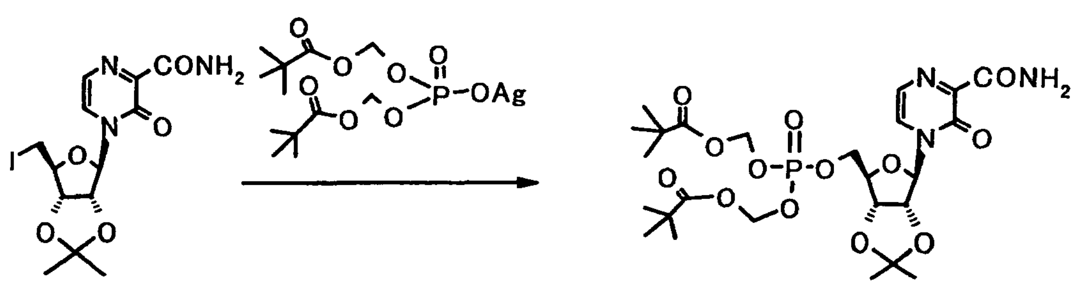

0.05 g of 4-[(3aR, 4R, 6S, 6aR)-6-(iodomethyl)-2,2-dimethyltetrahydrofuro[3,4-d][1,3]dioxol-4-yl]-3-oxo-3,4-dihydro-2-pyrazinecarboxamide was suspended in 2 mL of toluene. 70 mg of silver salts of bis(2,2-dimethylpropionyloxymethyl) phosphate, which had been separately prepared by the method described in Journal of Medicinal Chemistry (J. Med. Chem.) Vol. 37, pp. 3902 to 3909 (1994), was added thereto, and the mixture was stirred at 60°C for 2 hours. 0.15 g of 4-[(3aR, 4R, 6S, 6aR)-6-(iodomethyl)-2,2-dimethyltetrahydrofuro[3,4-d][1.3]dioxol-4-yl]-3-oxo-3,4-dihydro-2-pyrazinecarboxamide was treated in the same above manner. The obtained reaction mixtures were combined, and the solvent was removed under reduced pressure. The obtained residue was purified by silica gel column chromatography [eluant; chloroform : methanol = 10 : 1], to obtain 0.12 g of a yellow solid, 2,2-dimethyl-propionic acid [(3aR, 4R, 6R, 6aR)-6-(3-carbamoyl-2-oxo-2H-pyrazine-1-yl)-2,2-dimethyl-tetrahydrofuro[3,4-d][1,3]dioxol-4-ylmethoxy]-(2,2-dimethylpropionyloxymethoxy)-phosphoryloxymethyl ester.
IR(KBr)$cm^{-1}$: 1750, 1684, 1654
$^1H$-NMR(DMSO-$d_6$)δ: 1.14(18H,s), 1.29(3H,s), 1.51(3H,s), 3.5-3.7(2H,m), 4.34(1H,dd,J=4.0,7.2Hz), 4.75(1H,dd, J=2.8,6.0Hz), 4.86(1H,dd,J=2.0,6.0Hz), 5.23(1H,t,J=4.8Hz), 5.31(2H,s), 5.34(2H,s), 5.97(1H,d,J=2.0Hz), 7.56(1H,d, J=4.4Hz), 7.83(1H,brs), 8.06(1H,d,J=4.4Hz), 8.43(1H,brs)

Example 6

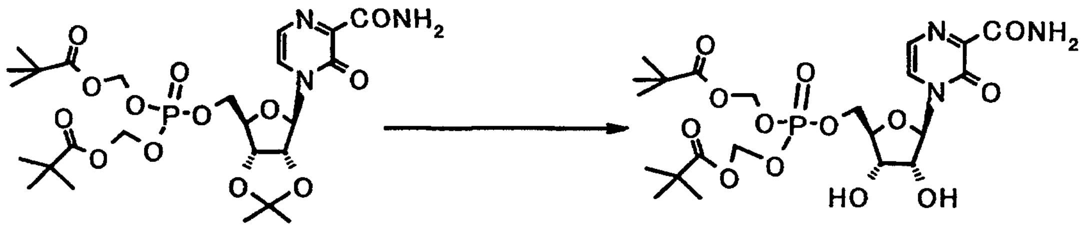

0.1 g of 2,2-dimethyl-propionic acid[(3aR, 4R, 6R, 6aR)-6-(3-carbamoyl-2-oxo-2H-pyrazine-1-yl)-2,2-dimethyl-tetrahydrofuro[3,4-d][1,3]dioxol-4-ylmethoxy]-(2,2-dimethyl-propionyloxymethoxy)-phosphoryloxymethyl ester was dissolved in a mixed solution of 1.5 mL of methanol and 1.5 mL of water, and thereafter, 5 mL of a Dowex 50WX4-200 ion exchange resin (H+form) was added thereto, followed by stirring at room temperature for 1.5 hours. Thereafter, the resin was removed by filtration and then washed with a mixed solution of 2.5 mL of acetonitrile and 2.5 mL of water. The obtained washings and the filtrate were combined, and the organic solvent was removed under reduced pressure. The obtained residue was lyophilized, to obtain 0.07 g of a light yellow solid, 2,2-dimethyl-propionic acid[(2R, 3S, 4R, 5R)-5-(3-carbamoyl-2-oxo-2H-pyrazine-1-yl)-3,4-dihydroxytetrahydro-furan-2-ylmethoxy]-(2,2-dimethylpropionyloxymethoxy)-phosphoryloxymethyl ester.
IR(KBr)$cm^{-1}$: 1751, 1670
$^1$H-NMR(DMSO-$d_6$)δ: 1.16(18H,s), 3.64(1H,dd,J=2.0,12.4Hz), 3.81(1H,dd,J=2.0,12.4Hz), 3.9-4.0(3H,m), 5.46(2H,s), 5.49(2H,s), 5.92(1H,d,J=2.4Hz), 7.54(1H,d,J=4.4Hz), 7.75(1H,brs), 8.29(1H,d,J=4.4Hz), 8.35(1H,brs)

Example 7

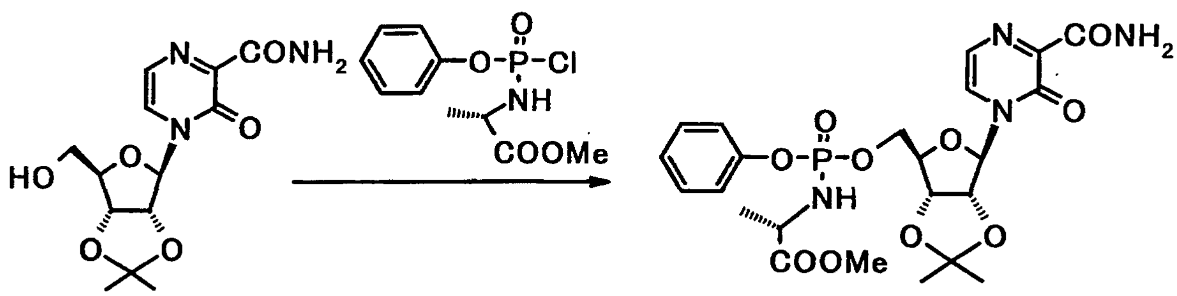

0.50 g of 4-[(3aR, 4R, 6R, 6aR)-6-(hydroxymethyl)-2,2-dimethyltetrahydrofuro[3,4-d][1,3]dioxol-4-yl]-3-oxo-3,4-dihydro-2-pyrazinecarboxamide was suspended in 50 mL of pyridine. Thereafter, 43 mL of a tetrahydrofuran solution containing 2.2g of methyl chloro phenylphosphoryl P→N-L-alaninate, which had been separately prepared by the method described in Antiviral Research, Vol. 43, pp. 37 to 53 (1999), and 1.3 mL of N-methylimidazole were successively added to the suspension, and the mixture was stirred at the same temperature for 3 hours. The solvent was removed from the reaction mixture under reduced pressure. The obtained residue was purified by silica gel column chromatography [eluant; chloroform : methanol = 40 : 1], to obtain 0.43 g of a light yellow solid, (2S)-[[(3aR, 4R, 6R, 6aR)-6-(3-carbamoyl-2-oxo-2H-pyrazine-1-yl)-2,2-dimethyl-tetrahydrofuro[3,4-d][1,3]dioxol-4-ylmethoxy]-phenoxy-phosphorylamino]-propionic acid methyl ester.
IR(KBr)$cm^{-1}$: 1749,1684
$^1$H-NMR(DMSO-$d_6$)δ: 1.22, 1.23(3H,d,J=7.1Hz), 1.28, 1.30(3H,s), 1.50, 1.51(3H,s), 3.58,3.60(3H,s), 3.80-3.88(1H,m), 4.15-4.34(2H,m), 4.42-4.45, 4.50-4.54(1H,m), 4.74, 4.81(1H,dd,J=2.0,6.3Hz), 4.67, 4.93(1H,dd,J=3.0,6.1Hz), 5.94-5.97(1H,m), 6.09-6.15(1H,m), 7.08-7.20(3H,m), 7.32-7.39(2H,m), 7.46, 7.51(1H,d,J=4.0Hz), 7.75-7.80(1H,brs), 7.84-7.87(1H,m), 8.26-8.32(1H,m)

Example 8

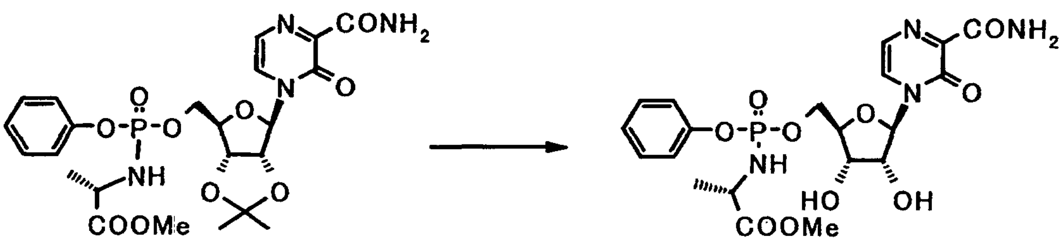

49 mg of a white solid, (2S)-{[(2R, 3S, 4R, 5R)-5-(3-carbamoyl-2-oxo-2H-pyrazine-1-yl)-3,4-dihydroxy-tetrahydro-furan-2-ylmethoxy]-phenoxyphosphorylamino}-propionic acid methyl ester was obtained from 190 mg of (2S)-{[(3aR, 4R, 6R, 6aR)-6-(3-carbamoyl-2-oxo-2H-pyrazine-1-yl)-2,2-dimethyltetrahydrofuro[3,4-d][1,3]dioxol-4-ylmethoxy]-phenoxyphosphorylamino}-propionic acid methyl ester in the same manner as in Example 2.
IR(KBr)$cm^{-1}$: 1735, 1676, 1661
$^1$H-NMR(DMSO-$d_6$)δ: 1.21, 1,23(3H,d,J=7.2Hz), 3.57,3.58(3H,s), 3.81-3.98(2H,m), 4.02-4.04(1H,m), 4.12-4.41(3H, m), 5.60(2H,brs), 5.91-5.94(1H,m), 6.10-6.20(1H,m), 7.15-7.24(3H,m), 7.34-7.45(3H,m), 7.73(1H,brs), 7.81,7.86(1H, d,J=4.2Hz), 8.29(1H,brs)

Example 9

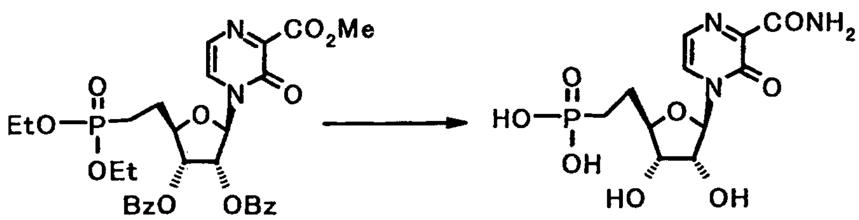

0.20 g of methyl 4-[(2R, 3R, 4R, 5R)-3,4-bis(benzoyloxy)-5-[2-(diethoxyphosphoryl)ethyl]tetrahydro-2-furanyl]-3-oxo-3,4-dihydro-2-pyrazinecarboxylate was dissolved in 2.0 mL of acetonitrile, and thereafter, 0.33 mL of bromotrimethyl silane was added thereto under ice cooling, followed by stirring at 0°C for 30 minutes, and then at room temperature for 1 hour. The solvent was removed from the reaction mixture under reduced pressure. 2.0 mL of methanol was added to the obtained solid and dissolved, and thereafter, ammonia gas was blown therein under ice cooling for saturation, followed by stirring at room temperature for 3 hours. The solvent was removed from the reaction mixture under reduced pressure, 10 mL of water was added, and the mixture was washed with chloroform. After insoluble products were filtrated, the solvent was removed under reduced pressure. The obtained residue was purified by reverse phase silica gel column chromatography [eluant; water], to obtain 28 mg of a light yellow solid, 2-[(2R, 3S, 4R, 5R)-5-[3-(aminocarbonyl)-2-oxo-1(2H)-pyrazinyl]-3,4-dihydroxytetrahydro-2-furanyl] ethyl phosphoric acid.
IR(KBr)$cm^{-1}$: 1676
$^1$H-NMR($D_2O$)δ: 1.7-1.95(2H,m), 2.0-2.2(2H,m), 3.95-4.0(1H,m), 4.2-4.25(1H,m), 4.33(1H,d,J=4.6Hz), 6.06(1H,s), 7.79(1H,d,J=4.0Hz), 8.03(1H,d,J=4.0Hz)

Example 10

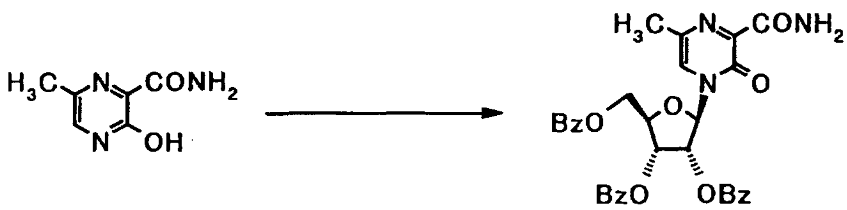

1.2 g of a yellow oil product, [(2R, 3R, 4R, 5R)-5-[3-(aminocarbonyl)-5-methyl-2-oxo-1(2H)-pyrazinyl]-3,4-bis (benzoyloxy)tetrahydro-2-furanyl] methyl benzoate was obtained from 0.43 g of 3-hydroxy-6-methyl-2-pyrazinecarboxamide in the same manner as in Reference Example 6.
IR(KBr)$cm^{-1}$: 1727, 1686, 1654
$^1$H-NMR($CDCl_3$)δ: 2.15(3H,s), 4.67(1H,dd,J=3.4, 12.7Hz), 4.85-4.88(1H,m), 4.99(1H,dd,J=2.4,12.7Hz), 5.88-5.95(2H, m), 6.03(1H,d,J=3.2Hz), 6.47(1H,d,J=3.9Hz), 7.36-7.65(10H,m), 7.93-8.00(4H,m), 8.10-8.13(2H,m), 9.12(1H,d, J=3.9Hz)

Example 11

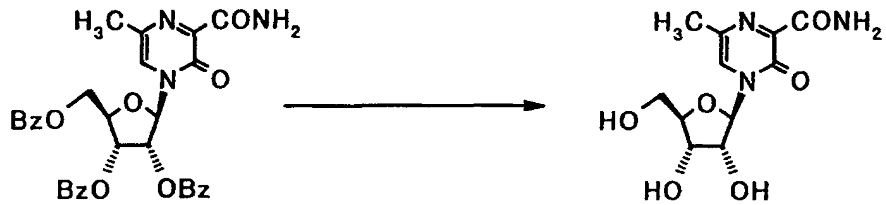

0.4 g of a light yellow solid, 4-[(2R, 3R, 4S, 5R)-3,4-dihydroxy-5-(hydroxymethyl)tetrahydro-2-furanyl]-6-methyl-3-oxo-3,4-dihydro-2-pyrazinecarboxamide was obtained from 1.05 g of [(2R, 3R, 4R, 5R)-5-[3-(aminocarbonyl)-5-methyl-2-oxo-1(2H)-pyrazinyl]-3,4-bis(benzoyloxy)tetrahydro-2-furanyl] methyl benzoate in the same manner as in Reference Example 7.
IR(KBr)$cm^{-1}$: 1698, 1654
$^1$H-NMR(DMSO-$d_6$)δ: 2.24(3H,s), 3.62-3.67(1H,m), 3.80-3.85(1H,m), 3.94-4.01(3H,m), 5.10(1H,d,J=5.1Hz), 5.32(1H, t,J=4.8Hz), 5.62(1H,d,J=3.2Hz), 5.92(1H,d,J=1.7Hz), 7.76(1H,brs), 8.16(1H,s), 8.48(1H,brs)

Example 12

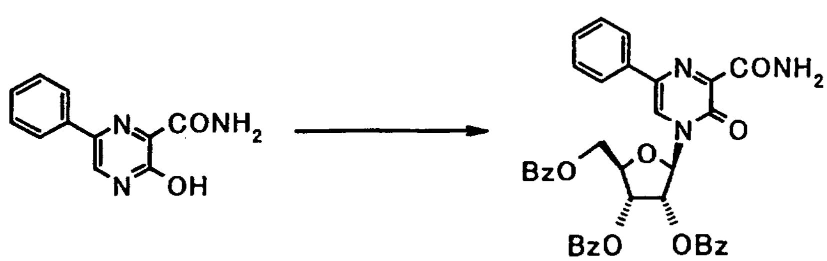

0.5 g of a yellow oil product, [(2R, 3R, 4R, 5R)-5-[3-(aminocarbonyl)-2-oxo-5-phenyl-1(2H)-pyrazinyl]-3,4-bis(benzoyloxy)tetrahydro-2-furanyl] methyl benzoate was obtained from 0.35 g of 3-hydroxy-6-phenyl-2-pyrazinecarboxamide in the same manner as in Reference Example 6.
IR(KBr)cm$^{-1}$: 1720, 1717, 1684, 1654
$^1$H-NMR($CDCl_3$)δ: 4.77(1H,dd,J=3.6, 12.4Hz), 4.90-4.97(2H,m), 5.93-5.99(2H,m), 6.03(1H,d,J=3.7Hz), 6.58(1H,d, J=4.2Hz), 7.32-7.68(14H,m), 7.95-8.05(6H,m), 8.11(1H,s), 8.93(1H,d,J=3.4Hz)

Example 13

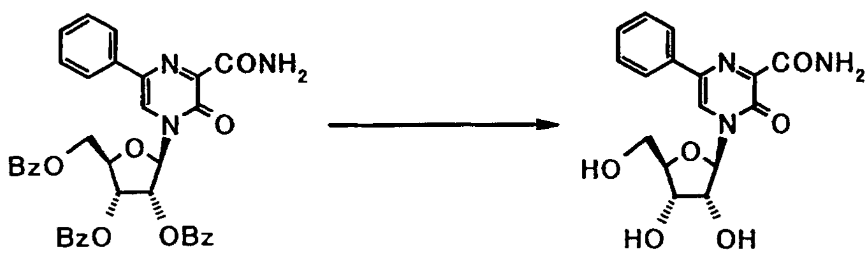

0.12 g of a yellow solid, 4-[(2R, 3R, 4S, 5R)-3,4-dihydroxy-5-(hydroxymethyl)tetrahydro-2-furanyl]-3-oxo-6-phenyl-3,4-dihydro-2-pyrazinecarboxamide was obtained from 0.48 g of [(2R, 3R, 4R, 5R)-5-[3-(aminocarbonyl)-2-oxo-5-phenyl-1(2H)-pyrazinyl]-3,4-bis(benzoyloxy)tetrahydro-2-furanyl] methyl benzoate in the same manner as in Reference Example 7.
IR(KBr)cm$^{-1}$: 1685, 1670, 1654
$^1$H-NMR(DMSO-$d_6$)δ: 3.71-3.75(1H,m), 3.93-3.97(1H,m), 4.02-4.15(3H,m), 5.10(1H,d,J=6.6Hz), 5.67(1H,t,J=4.0Hz), 5.73(1H,d,J=4.6Hz), 5.96(1H,s), 7.32-7.46(3H,m), 7.82(1H,brs), 7.88(2H,d,J=7.8Hz), 8.43(1H,brs), 9.07(1H,s)

Example 14

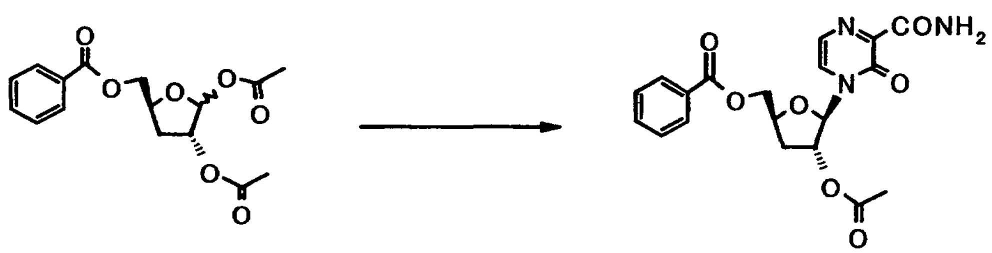

0.24 g of 3-hydroxy-2-pyrazinecarboxamide was suspended in 5 mL of 1,1,1-3,3,3-hexamethyldisilazane, and the suspension was under reflux for 2 hours. After cooling, the solvent was removed under reduced pressure. 5 mL of xylene was added, and the solvent was removed under reduced pressure. To the obtained residue, 12.5 mL of acetonitrile, 0.50 g of [(2S,4R)-4,5-bis(acetyloxy)tetrahydro-2-furanyl] methyl benzoate, and 0.29 mL of tin(IV) chloride were successively added, and the mixture was then stirred at room temperature for 30 minutes. Water was added to the reaction solution, and the organic solvent was removed under reduced pressure. Thereafter, a saturated sodium bicarbonate aqueous solution and ethyl acetate were added to the residue, and insoluble products were removed by filtration. The organic layer was separated, and the aqueous layer was extracted with ethyl acetate. The obtained organic layer was dried with anhydrous magnesium sulfate, and the solvent was removed under reduced pressure. The obtained residue was purified by silica gel chromatography [eluant; chloroform : methanol = 16 : 1], to obtain 0.61 g of a light yellow solid, {(2S, 4R, 5R)-4-(acetyloxy)-5-[3-(aminocarbonyl)-2-oxo-1(2H)-pyrazinyl]tetrahydro-2-furanyl} methyl benzoate.
IR(KBr)cm$^{-1}$: 1743, 1706, 1667
$^1$H-NMR($CDCl_3$)δ: 2.13-2.23(5H,m), 4.67(1H,dd,J=4.0, 12.8Hz), 4.78-4.85(2H,m), 5.48(1H,d,J=4.0Hz), 6.05(1H,s),

6.61(1H,brs), 7.47-7.51(2H,m), 7.58(1H,d,J=4.0Hz), 7.62-7.66(1H,m), 7.96(1H,d,J=4.0Hz), 8.02-8.04(2H,m), 9.06(1H, brs)

Example 15

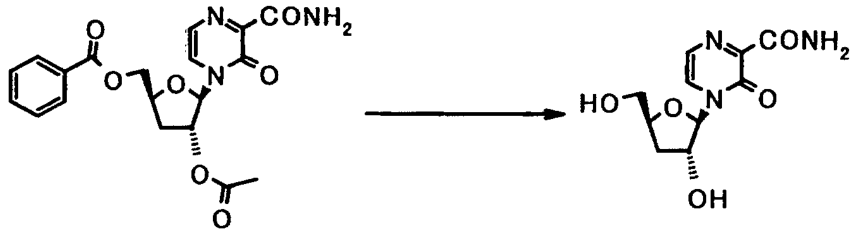

1.21 g of {(2S, 4R, 5R)-4-(acetyloxy)-5-[3-(aminocarbonyl)-2-oxo-1(2H)-pyrazinyl]tetrahydro-2-furanyl} methyl benzoate was dissolved in 36 mL of methanol, and thereafter, 1.28 g of a 28% sodium methoxide methanol solution was added thereto under ice cooling, followed by stirring at the same temperature for 1 hour. The reaction solution was adjusted to pH 5 by adding 1 mol/L hydrochloric acid, and the solvent was then removed under reduced pressure. The obtained residue was purified by silica gel chromatography [eluant; n-hexane : ethyl acetate = 4 : 1], and a mixed solvent of 2-propanol and ethyl acetate was added thereto. The precipitate was collected by filtration, to obtain 0.47 g of a white solid, 4-[(2R, 3R, 5S)-3-hydroxy-5-(hydroxymethyl)tetrahydro-2-furanyl]-3-oxo-3,4-dihydro-2-pyrazinecarboxamide.
IR(KBr)$cm^{-1}$: 1682, 1654
$^1$H-NMR(DMSO-$d_6$)δ: 1.72(1H,dd,J=5.2,13.2Hz), 1.86-1.93(1H,m), 3.61-3.66(1H,m), 3.88-3.93(1H,m), 4.25(1H,t, J=4.0Hz), 4.43-4.48(1H,m), 5.28(1H,t,J=5.0Hz), 5.77(1H,d,J=4.0Hz), 5.82(1H,s), 7.56(1H,d,J=4.4Hz), 7.76(1H,brs), 8.37(1H,d,J=4.4Hz), 8.42(1H,brs)

Example 16

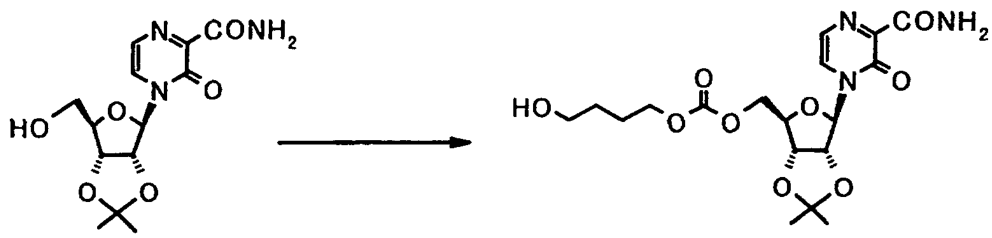

0.5 g of 4-[(3aR, 4R, 6R, 6aR)-6-(hydroxymethyl)-2,2-dimethyltetrahydrofuro[3,4-d][1,3]dioxol-4-yl]-3-oxo-3,4-dihydro-2-pyrazinecarboxamide was dissolved in 5 mL of N,N-dimethylformamide, and thereafter, 0.29 g of 1,1'-carbonyldiimidazole was added thereto, followed by stirring at room temperature for 1 hour. Thereafter, 0.21 mL of 1,4-butanediol was added thereto, and the mixture was stirred at room temperature for 1 hour, and then at 60°C for 1 hour. 0.29 mL of 1,4-butanediol was further added thereto, and the mixture was stirred at 60°C for 3 hours. The reaction solution was concentrated under reduced pressure, and then, 30 mL of ethyl acetate, 30 mL of water, and 5 mL of a saturated saline solution were added to the concentrate. The organic layers were separated, and the aqueous layer was then extracted with 20 mL of ethyl acetate. The organic layers were combined and then dried with anhydrous magnesium sulfate, and the solvent was removed under reduced pressure. The obtained residue was purified by silica gel chromatography [eluant; ethyl acetate : methanol = 10 : 1], to obtain 0.31 g of a light yellow oil product, {(3aR, 4R, 6R, 6aR)-6-[3-(aminocarbonyl)-2-oxo-1(2H)-pyrazinyl]-2,2-dimethyltetrahydrofuro[3,4-d][1,3]dioxol-4-yl} methyl 4-hydroxybutyl carbonate.

IR(KBr)$cm^{-1}$: 1750, 1684, 1654
$^1$H-NMR($CDCl_3$)δ: 1.41(3H,s), 1.47-1.54(2H,m), 1.62-1.68(5H,m), 3.60-3.66(2H,m), 4.04-4.16(2H,m), 4.32(1H,dd, J=3.2, 12.4Hz), 4.48(1H,dd,J=2.2, 12.2Hz), 4.73-4.76(1H,m), 4.79(1H,dd,J=2.4, 6.0Hz), 4.92(1H,dd,J=1.8, 6.2Hz), 5.98(1H,d,J=2.0Hz), 6.31(1H,d,J=23.6Hz), 7.77(1H,s), 7.75(1H,s), 8.78(1H,br), 9.25(1H,brs)

Example 17

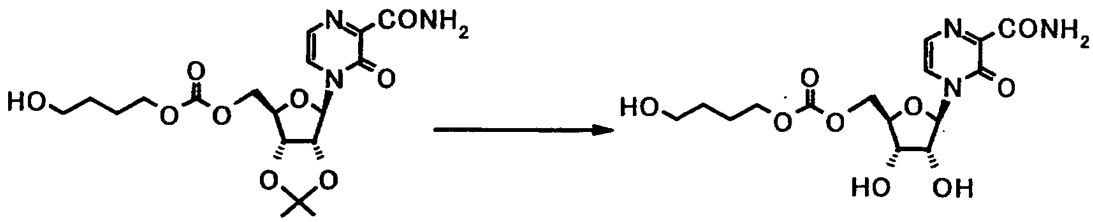

1.2 g of {(3aR, 4R, 6R, 6aR)-6-[3-(aminocarbonyl)-2-oxo-1(2H)-pyrazinyl]-2,2-dimethyltetrahydrofuro[3,4-d][1,3]dioxol-4-yl} methyl 4-hydroxybutyl carbonate was dissolved in a mixed solvent of 8 mL of methanol and 60 mL of water, and thereafter, 19 g of a Dowex 50WX4-200 ion exchange resin (H+form) was added thereto, followed by stirring at room temperature for 3 hours. Thereafter, the resin was removed by filtration, and it was washed with methanol and then with water. The obtained filtrate and the washings were combined, and the solvent was removed under reduced pressure. The residue was dissolved in water, and the solution was then lyophilized. The obtained solid was washed with acetonitrile and then with chloroform, to obtain 0.16 g of a colorless solid, {(2R, 3S, 4R, 5R)-5-[3-(aminocarbonyl)-2-oxo-1(2H)-pyrazinyl]-3,4-dihydroxytetrahydro-2-furanyl} methyl 4-hydroxybutyl carbonate.
IR(KBr)$cm^{-1}$: 1745, 1664
$^1$H-NMR(DMSO-$d_6$)δ: 1.45-1.51(2H,m), 1.64-1.67(2H,m), 3.40-3.42(2H,m), 3.90(1H,d,J=4.8Hz), 4.08-4.15(4H,m), 4.36-4.47(3H,m), 5.38(1H,d,J=5.2Hz), 5.74(1H,brs), 5.92(1H,s), 7.50(1H,d,J=3.6Hz), 7.75(1H,brs), 7.81(1H,d, J=3.6Hz), 8.30(1H,brs)

Example 18

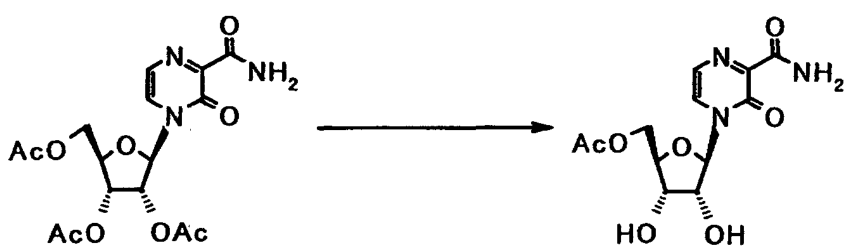

200 mg of (2R, 3R, 4R, 5R)-4-(acetyloxy)-2-[(acetyloxy)methyl]-5-[3-(aminocarbonyl)-2-oxo-1(2H)-pyrazinyl] tetrahydro-3-furanyl acetate was dissolved in 2 mL of pyridine, and thereafter, 0.1 mL of hydrazine monohydrate was added thereto, followed by stirring at room temperature for 1 hour. Thereafter, acetone was added thereto, and the solvent was then removed at reduced pressure. The obtained residue was purified by silica gel chromatography [eluant; ethyl acetate : methanol = 10 : 1], to obtain 42 mg of a white solid, {(2R, 3S, 4R, 5R)-5-[3-(aminocarbonyl)-2-oxo-1(2H)-pyrazinyl]-3,4-dihydroxytetrahydro-2-furanyl} methyl acetate.
$^1$H-NMR(DMSO-$d_6$)δ: 3.20(3H,s), 3.90(1H,dd,J=6.8,11.5), 4.06-4.09(1H,m), 4.12-4.17(1H,m), 4.31(1H,dd, J=5.6,12.7), 4.35(1H,dd,J=3.4,12.7), 5.33(1H,d,J=6.6), 5.73,(1H,d,J=5.1), 5.90(1H,d,J=2.0), 7.57(1H,d,J=4.4), 7.75 (1H,brs), 7.85(1H,d,J=4.4), 8.31(1H,brs)

Example 19

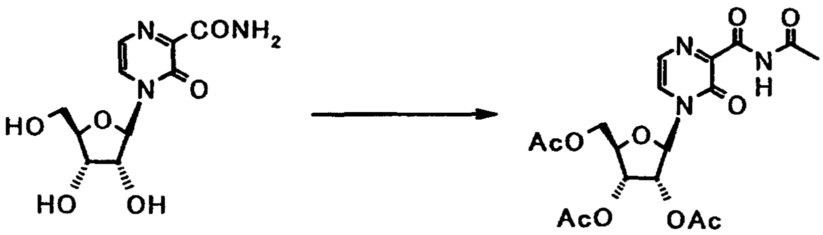

5 g of 4-[(2R, 3R, 4S, 5R)-3,4-dihydroxy-5-(hydroxymethyl)tetrahydro-2-furanyl]-3-oxo-3,4-dihydro-2-pyrazinecarboxamide was suspended in 25 mL of acetic anhydride and 12.5 mL of pyridine, and the suspension was stirred at 50°C for 1 hour and then at 70°C for 1 hour. After cooling the suspension to room temperature, insoluble products were removed by filtration. The residue was concentrated under reduced pressure, and the concentrate was then purified by silica gel column chromatography [eluant; ethyl acetate], to obtain 1.26 g of a light yellow solid, (2R, 3R, 4R, 5R)-2-[3-[(acetylamino)carbonyl]-2-oxo-1(2H)-pyrazinyl]-4-(acetyloxy)-5-[(acetyloxy)methyl] tetrahydro-3-furanyl acetate.

IR(KBr)cm$^{-1}$: 1750, 1709

$^1$H-NMR(DMSO-$d_6$)δ: 2.06(6H,s), 2.09(3H,s), 2.17(3H,s), 4.26-4.42(3H,m), 5.35(1H,t,J=6.1Hz), 5.50(1H,dd, J=3.7,6.1Hz), 6.06(1H,d,J=3.4Hz), 7.50(1H,d,J=4.4Hz), 7.83(1H,d,J=4.4Hz), 11.44(1H,s)

Example 20

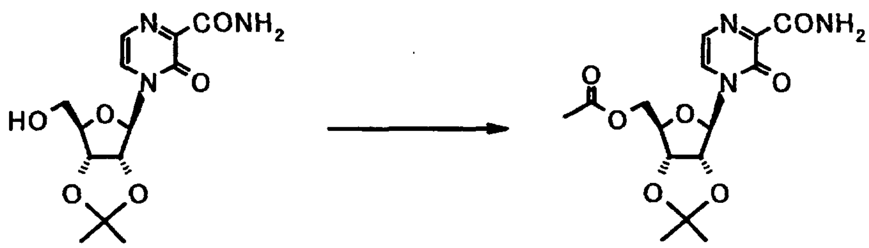

0.2 g of 4-[(3aR, 4R, 6R, 6aR)-6-(hydroxymethyl)-2,2-dimethyltetrahydrofuro[3,4-d][1,3]dioxol-4-yl]-3-oxo-3,4-dihydro-2-pyrazinecarboxamide was suspended in 1 mL of acetic anhydride and 4 mL of pyridine, and the suspension was stirred at room temperature for 1 hour. The reaction solution was concentrated under reduced pressure, 5 mL of water was added to the residue, and the mixture was extracted with 5 mL of ethyl acetate 10 times. The organic layers were combined, 1 mL of water was added thereto, and the obtained solution was adjusted to pH 3 with 2 mol/ L hydrochloric acid. The organic layer was separated, washed with a saturated sodium chloride aqueous solution, and then dried with anhydrous magnesium sulfate. Then, the solvent was removed under reduced pressure. The obtained residue was purified by silica gel column chromatography [eluant; chloroform : methanol = 10 : 1], to obtain 0.15 g of a light yellow oil product, {(3aR, 4R, 6R, 6aR)-6-[3-(aminocarbonyl)-2-oxo-1(2H)-pyrazinyl]-2,2-dimethyltetrahydrofuro [3,4-d][1,3]dioxol-4-yl} methyl acetate.

IR(KBr)cm$^{-1}$: 1743, 1685

$^1$H-NMR($CDCl_3$)δ: 1.41(3H,s), 1.65(3H,s), 1.94(3H,s), 4.32(1H,dd,J=4.4,12.5Hz), 4.40(1H,dd,J=2.9,12.5Hz), 4.68-4.71(1H,m), 4.76(1H,dd,J=2.9,6.1Hz), 4.87(1H,dd,J=2.1,6.2Hz), 5.95(1H,d,J=2.0Hz), 6.17(1H,brs), 7.70(1H,d, J=4.2Hz), 7.80(1H,d,J=4.2Hz), 9.13(1H,brs)

Example 21

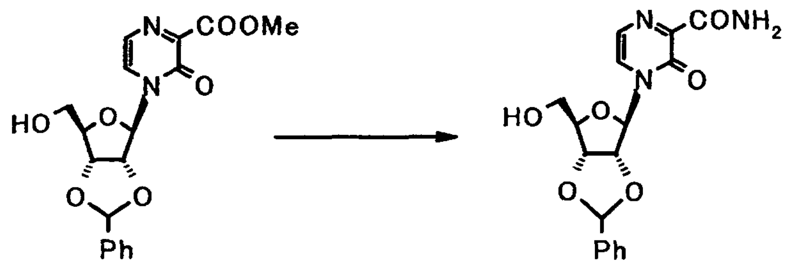

0.5 g of methyl 4-[(3aR, 4R, 6R, 6aR)-6-(hydroxymethyl)-2-phenyltetrahydrofuro[3,4-d][1,3]dioxol-4-yl]-3-oxo-3,4-dihydro-2-pyrazinecarboxylate was suspended in 15 mL of a methanol solution containing approximately 5 mol/L dry ammonia, and the suspension was stirred at room temperature for 3 hours. The deposit was collected by filtration and then washed with methanol, to obtain 0.34 g of a colorless solid, 4-[(3aR, 4R, 6R, 6aR)-6-(hydroxymethyl)-2-phenyltetrahydrofuro[3,4-d][1,3]dioxol-4-yl]-3-oxo-3,4-dihydro-2-pyrazinecarboxamide.
IR(KBr)$cm^{-1}$: 1684
$^1$H-NMR(DMSO-$d_6$)δ: 3.61-3.75(2H,m), 4.52-4.54(1H,m), 4.87(1H,dd,J=2.4,6.3Hz), 5.00(1H,dd,J=2.2,6.6Hz), 5.25 (1H,t,J=4.9Hz), 5.96(1H,s), 6.10(1H,d,J=2.0Hz), 7.45-7.48(3H,m), 7.54-7.57(3H,m), 7.76(1H,brs), 8.06(1H,d, J=4.4Hz), 8.34(1H,brs)

Example 22

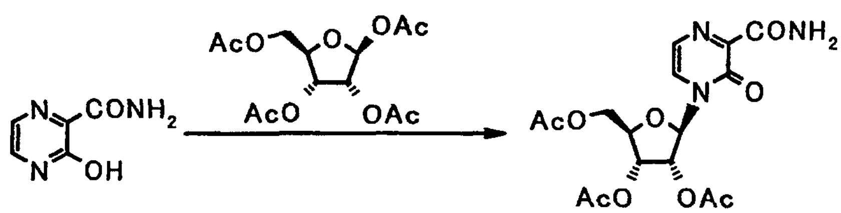

1.39 g of 3-hydroxy-2-pyrazinecarboxamide was suspended in 10 mL of 1,1,1,3,3,3-hexamethyldisilazane, and the suspension was under reflux for 1 hour. After cooling, the solvent was removed under reduced pressure. Then, 5 mL of toluene was added, and the solvent was removed under reduced pressure. 15 mL of acetonitrile, 4.14 g of β-D-ribofuranose 1,2,3,5-tetraacetate, and 1.99 mL of trimethylsilyl trifluoromethane sulfonate were successively added to the obtained residue, and the obtained mixture was stirred at room temperature for 2 hours. Thereafter, 0.95 g of β-D-ribofuranose 1,2,3,5-tetraacetate was added thereto, and the mixture was further stirred at room temperature for 1 hour. The solvent was removed under reduced pressure, and chloroform and water were added. The organic layer was separated, and it was washed with a saturated sodium bicarbonate aqueous solution and then dried with anhydrous magnesium sulfate. The solvent was removed under reduced pressure. Ethyl acetate was added to the residue, and the precipitate was collected by filtration, to obtain 1.73 g of (2R, 3R, 4R, 5R)-4-(acetyloxy)-2-[(acetyloxy) methyl]-5-[3-(aminocarbonyl)-2-oxo-1(2H)-pyrazinyl] tetrahydro-3-furanyl acetate.
$^1$H-NMR($CDCl_3$)δ: 2.10(3H,s), 2.16(3H,s), 2.17(3H,s), 4.40-4.52(3H,m), 5.29(1H,t,J=6.2Hz), 5.48(1H,dd,J=3.2,5.1Hz), 6.05(1H,brs), 6.16(1H,d,J=3.2Hz), 7.79(1H,d,J=4.2Hz), 7.81(1H,d,J=4.2Hz), 8.98(1H,brs),

Example 23

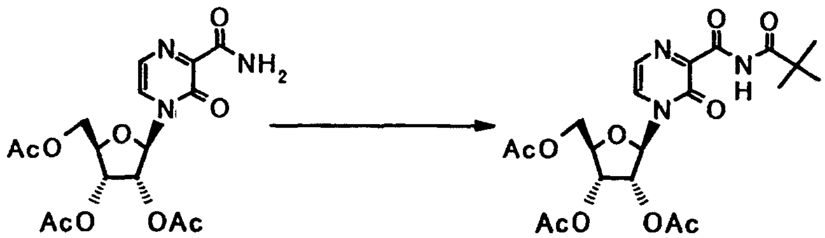

0.40 g of (2R, 3R, 4R, 5R)-4-(acetyloxy)-2-[(acetyloxy)methyl]-5-[3-(aminocarbonyl)-2-oxo-1(2H)-pyrazinyl] tetrahydro-3-furanyl acetate was suspended in tetrahydrofuran, and thereafter, 0.06 g of sodium hydride (60% mineral oil suspension) and 0.14 mL of pivaloyl chloride were successively added to the suspension under ice cooling. The mixture was stirred at room temperature for 2 hour. Thereafter, 0.06 g of sodium hydride (60% mineral oil suspension) and 0.14 mL of pivaloyl chloride were further added thereto, and the mixture was stirred at room temperature for 1 hour. Further, 0.06 g of sodium hydride (60% mineral oil suspension) and 0.14 mL of pivaloyl chloride were further added thereto, and the mixture was stirred at room temperature for 1 hour. Ethyl acetate and water were added to the reaction solution. The organic layer was separated, washed with water, and dried with anhydrous magnesium sulfate. The solvent was removed under reduced pressure, and the obtained residue was purified by silica gel chromatography [eluant; n-hexane : ethyl acetate = 3 : 1], to obtain 0.14 g of a yellow solid, (2R, 3R, 4R, 5R)-4-(acetyloxy)-2-[(acetyloxy) methyl]-5-[3-{[(2,2-dimethylpropanoyl)amina]carbonyl}-2-oxo-1(2H)-pyrazinyl] tetrahydro-3-furanyl acetate.
$^1$H-NMR($CDCl_3$) δ: 1.30(9H,s), 2.13(3H,s), 2.16(6H,s), 4.39-4.49(2H,m), 4.52-4.55(1H,m), 5.29(1H,t,J=5.9Hz), 5.48 (1H,dd,J=3.7,5.6Hz), 6.22(1H,d,J=3.4Hz), 7.84(1H,d,J=4.2Hz), 7.86(1H,d,J=4.2Hz), 11.82(1H,brs)

Example 24

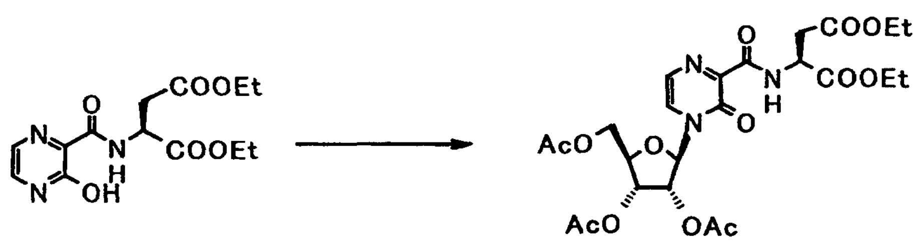

0.16 g of diethyl (2S)-2-{[(3-hydroxy-2-pyrazinyl)carbonyl]amino} butanedioate was suspended in 2 mL of 1,1,1,3,3,3-hexamethyldisilazane, and the suspension was under reflux for 1 hour. After cooling, the solvent was removed under reduced pressure. 1 mL of toluene was added, and the solvent was removed under reduced pressure. 2 mL of acetonitrile, 0.24 g of β-D-ribofuranose 1,2,3,5-tetraacetate, and 0.11 mL of trimethylsilyl trifluoromethane sulfonate were successively added to the obtained residue, and the obtained mixture was stirred at room temperature for 1 hour. Chloroform and water were added thereto, and the organic layer was separated. The obtained organic layer was washed with a saturated sodium bicarbonate aqueous solution, and then dried with anhydrous magnesium sulfate. The solvent was removed under reduced pressure. The obtained residue was purified by silica gel chromatography [eluant; chloroform : methanol = 40 : 1], to obtain 0.25 g of a light yellow solid, diethyl (2S)-2-{[(4-{(2R, 3R, 4R, 5R)-3,4-bis(acetyloxy)-5-[(acetyloxy)methyl]tetrahydro-2-furanyl}-3-oxo-3,4-dihydro-2-pyrazinyl)carbonyl]amino} butanedioate.
$^1$H-NMR($CDCl_3$)δ: 1.25(3H,t,J=7.2Hz), 1.28(3H,t,J=7.2Hz), 2.10(3H,s), 2.15(3H,s), 2.17(3H,s), 2.98(1H,dd, J=4.8,17.2Hz), 3.11(1H,dd,J=4.8Hz,17.2Hz), 4.13-4.18(2H,m), 4.21-4.28(2H,m), 4.40-4.47(2H,m), 4.49-4.51(1H,m), 5.09-5.12(1H,m), 5.26-5.28(1H,m), 5.43-5.44(1H,m), 6.27(1H,d,J=3.3Hz), 7.77(1H,d,J=4.0Hz), 7.79(1H,d,J=4.0Hz), 10.13(1H,d,J=8.1Hz)

Example 25

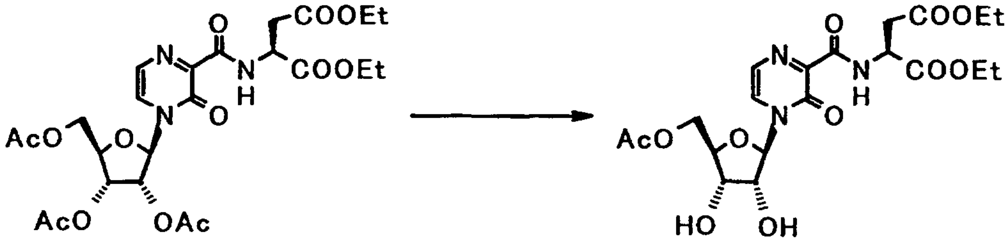

0.21 g of diethyl (2S)-2-{[(4-{(2R, 3R, 4R, 5R)-3,4-bis(acetyloxy)-5-[(acetyloxy)methyl]tetrahydro-2-furanyl}-3-oxo-3,4-dihydro-2-pyrazinyl)carbonyl]amino} butanedioate was dissolved in ethanol, and thereafter, 75 mg of sodium ethoxide was added thereto under ice cooling, followed by stirring at room temperature for 1 hour. Thereafter, the stirring was terminated, and the reaction mixture was left overnight at room temperature. 0.13 mL of acetic acid and 1 mL of water were successively added thereto, and the solvent was removed under reduced pressure. The obtained residue was purified by silica gel chromatography [eluant; chloroform : methanol = 40 : 1]. Ethyl acetate and n-hexane were added to the obtained residue, and the precipitate was collected by filtration, to obtain 39 mg of a yellow solid, diethyl (2S)-2-{[(4-{(2R, 3R, 4S, 5R)-5-[(acetyloxy)methyl]-3,4-dihydroxytetrahydro-2-furanyl}-3-oxo-3,4-dihydro-2-pyrazinyl)carbonyl]amino} butanedioate.
IR(KBr)$cm^{-1}$: 1739, 1670
$^1$H-NMR(DMSO-$d_6$)δ: 1.18(6H,t,J=6.8Hz), 2.10(3H,s), 2.89(2H,brs), 3.90(1H,brs), 4.07-4.15(6H,m), 4.30-4.38(2H,m), 4.87-4.92(1H,m), 5.30-5.34(1H,m), 5.77-5.79(1H,m), 5.91(1H,s), 7.70(1H,d,J=3.4Hz), 7.96(1H,d,J=3.4Hz), 9.83(1H, d,J=7.6Hz)

Example 26

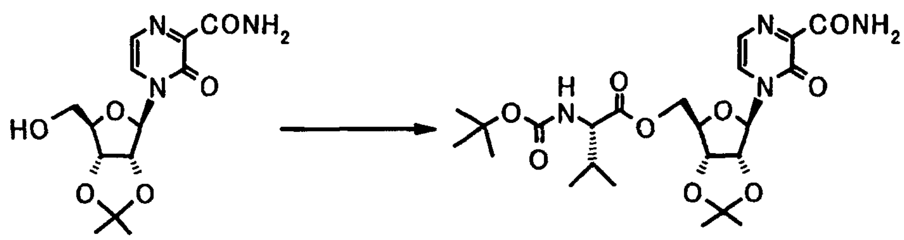

0.31 g of 4-[(3aR, 4R, 6R, 6aR)-6-(hydroxymethyl)-2,2-dimethyltetrahydrofuro[3,4-d][1,3]dioxol-4-yl]-3-oxo-3,4-dihydro-2-pyrazinecarboxamide was dissolved in 2 mL of N,N-dimethylformamide, and thereafter, 0.33 g of N-(t-butoxycarbonyl)-L-valine, 12 mg of 4-dimethylaminopyridine, and 0.41 g of 1,3-dicyclohexylcarbodiimide were successively added thereto, followed by stirring at room temperature for 2 hours. 0.5 mL of acetic acid was added to the mixture, and it was stirred at room temperature for 30 minutes. Thereafter, ethyl acetate and water were added thereto, and insoluble products were removed by filtration. The organic layer was separated, washed with 1 mol/L hydrochloric acid, and dried with anhydrous magnesium sulfate. The solvent was then removed under reduced pressure. The obtained residue was purified by silica gel chromatography [eluant; ethyl acetate], to obtain 0.40 g of a yellow solid, {(3aR, 4R, 6R, 6aR)-6-[3-(aminocarbonyl)-2-oxo-1(2H)-pyrazinyl]-2,2-dimethyltetrahydrofuro[3,4-d][1,3]dioxol-4-yl} methyl (2S)-2-[(tert-butoxycarbonyl)amino]-3-methyl butanoate.
$^1$H-NMR(DMSO-$d_6$)δ: 0.83-0.86(6H,m), 1.30(3H,s), 1.38(9H,s), 1.52(3H,s), 1.86-1.95(1H,m), 3.79(1H,t,J=7.2Hz), 4.27-4.34(2H,m), 4.46(1H,brs), 4.78-4.80(1H,m), 5.03(1H,d,J=6.1Hz), 6.00(1H,s), 7.24(1H,d,J=7.2Hz), 7.54(1H,d, J=4.3Hz), 7.77(1H,brs), 7.86(1H,d,J=4.3Hz), 8.28(1H,brs)

Example 27

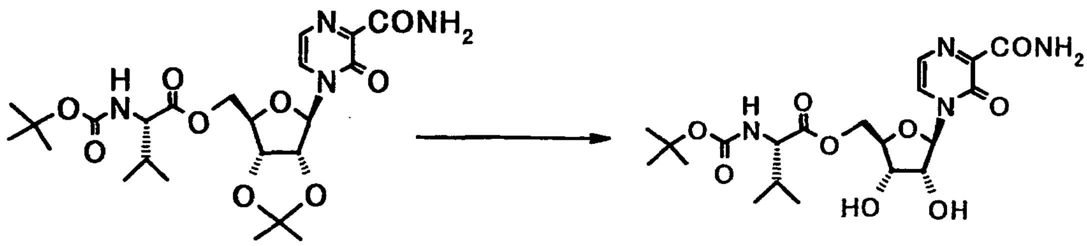

0.26 g of {(3aR, 4R, 6R, 6aR)-6-[3-(aminocarbonyl)-2-oxo-1(2H)-pyrazinyl]-2,2-dimethyltetrahydrofuro[3,4-d][1,3]dioxol-4-yl} methyl (2S)-2-[(tert-butoxycarbonyl)amino]-3-methyl butanoate was dissolved in a mixed solvent of 3 mL of methanol and 3 mL of water, and thereafter, 2.6 g of a Dowex 50WX4-200 ion exchange resin (H+form) was added thereto, followed by stirring at room temperature for 3 hours. Thereafter, the resin was removed by filtration, and it was then washed with methanol and then with water. The obtained filtrate and the washings were combined, and the solvent was removed under reduced pressure. 2 mL of toluene was added, and the solvent was removed under reduced pressure, to obtain 70 mg of a light yellow solid, {(2R, 3S, 4R, 5R)-5-[3-(aminocarbonyl)-2-oxo-1(2H)-pyrazinyl]-3,4-dihydroxytetrahydro-2-furanyl}methyl (2S)-2-[(tert-butoxycarbonyl)amino]-3-methyl butanoate.
$^1$H-NMR(DMSO-$d_6$)δ: 0.87-0.91(6H,m), 1.38(9H,s), 1.97-2.05(1H,m), 3.53(2H,br), 3.85-3.93(2H,m), 4.07(1H,brs), 4.19(1H,brs), 4.31-4.35(1H,m), 4.39-4.42(1H,m), 5.92(1H,s), 7.32(1H,d,J=7.6Hz), 7.60(1H,d,J=4.1Hz), 7.76(1H,brs), 7.87(1H,d,=4.1Hz), 8.32(1H,brs)

Example 28

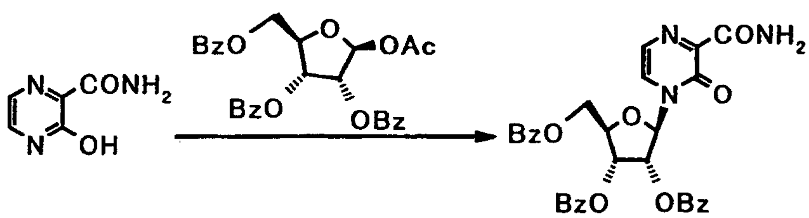

14.1 g of 3-hydroxy-2-pyrazinecarboxamide was suspended in 150 mL of toluene, and the suspension was stirred under reflux for 1 hour, followed by azeotropic dehydration. After cooling it to room temperature, the solvent was removed under reduced pressure. 42.3 mL of 1,1,1,3,3,3-hexamethyldisilazane was added to the residue, and the obtained mixture was heated under reflux for 2 hours. The reaction mixture was concentrated under reduced pressure, 45 mL of toluene was added thereto, and the solvent was removed under reduced pressure. Using 45 mL of toluene, the same above operation was repeated twice. The residue was dissolved in 40 mL of acetonitrile, and it was then added to 100 mL of an acetonitrile solution containing 48.4 g of β-D-ribofuranose 1-acetate 2,3,5-tribenzoate. 25 g of tin(IV) chloride was dropped into the mixture at room temperature. The obtained solution was stirred at 50°C for 4.5 hours. After cooling it to room temperature, it was added to a mixed solution consisting of 100 g of sodium bicarbonate, 500 mL of water and 280 mL of methylene chloride. Insoluble products were filtrated, and the organic layer was separated. The obtained organic layer was washed with 50 mL of water and then with 50 mL of a saturated sodium chloride aqueous solution, and the solvent was removed under reduced pressure. 280 mL of ethyl acetate and 30 mL of water were added to the obtained residue, and the mixture was heated to 50°C, and it was then cooled to 5°C. The deposit was collected by filtration, to obtain 40.9 g of a grayish-white solid, [(2R, 3R, 4R, 5R)-5-[3-(aminocarbonyl)-2-oxo-1(2H)-pyrazinyl]-3,4-bis(benzoyloxy)tetrahydro-2-furanyl] methyl benzoate.
IR(KBr)cm$^{-1}$: 1729, 1683
$^1$H-NMR(DMSO-$d_6$)δ: 4.69-4.78(2H,m), 4.88-4.92(1H,m), 5.97-6.05(2H,m), 6.34(1H,d,J=2.4Hz), 7.40-7.54(7H,m),

7.62-7.71(3H,m), 7.80(1H,brs), 7.84-7.86(2H,m), 7.95-8.03(5H,m), 8.22(1H,m)

Example 29

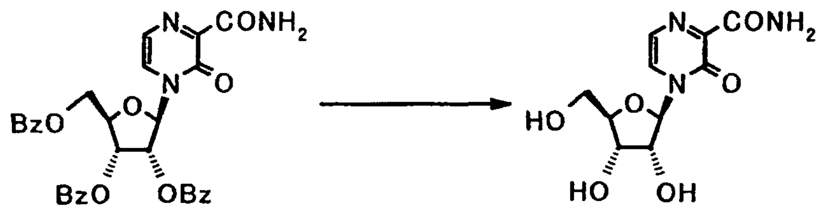

17.5 mL of water containing 2.2 g of sodium hydroxide was added to 330 mL of a methanol suspension containing 35 g of [(2R, 3R, 4R, 5R)-5-[3-(aminocarbonyl)-2-oxo-1(2H)-pyrazinyl]-3,4-bis(benzoyloxy)tetrahydro-2-furanyl] methyl benzoate at room temperature. The mixture was stirred at the same temperature for 2 hours, and then cooled to 5°C. The obtained deposit was collected by filtration, to obtain 12.5 g of a light yellow solid, 4-[(2R, 3R, 4S, 5R)-3,4-dihydroxy-5-(hydroxymethyl)tetrahydro-2-furanyl]-3-oxo-3,4-dihydro-2-pyrazinecarboxamide (water content: 0.4%).
IR(KBr)cm$^{-1}$: 3406, 1654
$^1$H-NMR(DMSO-$d_6$) δ: 3.65(1H,ddd,J=2.6,5.1,12.5Hz), 3.81(1H,ddd,J=2.2,4.8,12.1Hz), 3.96-4.00(2H,m), 4.01-4.03 (1H,m), 5.10(1H,d,J=5.9Hz), 5.28(1H,t,J=4.9Hz), 5.64(1H,d,J=4.8Hz), 5.93(1H,d,J=2.6Hz), 7.54(1H,d,J=4.0Hz), 7.74 (1H,s), 8.29(1H,d,J=4.0Hz), 8.36(1H,s)

Example 29(2)

11 g of the 4-[(2R, 3R, 4S, 5R)-3,4-dihydroxy-5-(hydroxymethyl)tetrahydro-2-furanyl]-3-oxo-3,4-dihydro-2-pyrazinecarboxamide obtained in Example 29 was dissolved in 300 mL of water (45°C), and then 1.1 g of activated carbon was added thereto, followed by stirring for 10 minutes. The activated carbon was filtrated and washed with 20 mL of water twice. The filtrate and the washings were combined, and 1.1 g of activated carbon was added thereto, followed by stirring for 10 minutes. The activated carbon was filtrated, washed with 20 mL of water twice, and then concentrated under reduced pressure. 90 mL of water was added to the concentrate, and thereafter the mixture was filtrated, to obtain 9.35 g of a colorless crystal, 4-[(2R, 3R, 4S, 5R)-3,4-dihydroxy-5-(hydroxymethyl)tetrahydro-2-furanyl]-3-oxo-3,4-dihydro-2-pyrazinecarboxamide monohydrate (water content: 6.8%).
IR(KBr)cm$^{-1}$: 3578, 3399, 3091, 2929, 1674
$^1$H-NMR(DMSO-$d_6$) δ: 3.65(1H,ddd,J=2.6,5.1,12.5Hz), 3.81(1H,ddd,J=2.2,4.8,12.1Hz), 3.96-4.00(2H,m), 4.01-4.03 (1H,m), 5.10(1H,d,J=5.9Hz), 5.28(1H,t,J=4.9Hz), 5.64(1H,d,J=4.8Hz), 5.93(1H,d,J=2.6Hz), 7.54(1H,d,J=4.0Hz), 7.74 (1H,s), 8.29(1H,d,J=4.0Hz), 8.36(1H,s)

Example 30

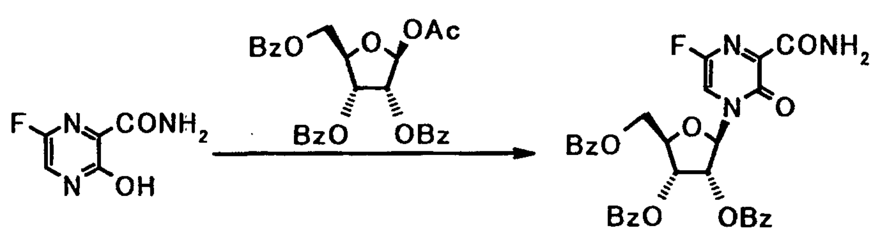

0.47 g of a light yellow solid, [(2R, 3R, 4R, 5R)-5-[3-(aminocarbonyl)-5-fluoro-2-oxo-1(2H)-pyrazinyl]-3,4-bis(benzoyloxy)tetrahydro-2-furanyl] methyl benzoate was obtained from 0.23 g of 6-fluoro-3-hydroxy-2-pyrazinecarboxamide in the same manner as in Reference Example 6.

IR(KBr)$cm^{-1}$: 1726, 1690
$^1$H-NMR(DMSO-$d_6$)δ: 4.6-5.0(3H,m), 5.9-6.1(2H,m), 6.33(1H,s), 7.3-8.2(17H,m), 8.53(1H,brs)

Example 31

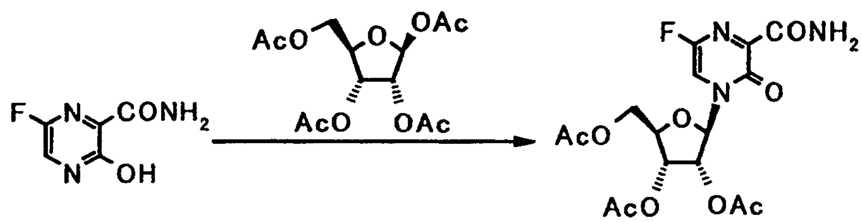

7.37 g of a light yellow solid, (2R, 3R, 4R, 5R)-4-(acetyloxy)-2-[(acetyloxy)methyl]-5-[3-(aminocarbonyl)-5-fluoro-2-oxo-1(2H)-pyrazinyl] tetrahydro-3-furanyl acetate was obtained from 4.0 g of 6-fluoro-3-hydroxy-2-pyrazinecarboxamide in the same manner as in Reference Example 6.
IR(KBr)$cm^{-1}$: 1748, 1715, 1662
$^1$H-NMR($CDCl_3$)δ: 2.04(3H,s), 2.08(3H,s), 2.18(3H,s) 4.47-4.58(3H,m), 5.20-5.34(1H,m), 5.51(1H,dd,J=2.3,5.0Hz), 6.16(1H,d,J=2.2Hz), 6.41(1H,brs), 7.95(1H,d,J=5.6Hz), 8.94(1H,brs)

Example 32

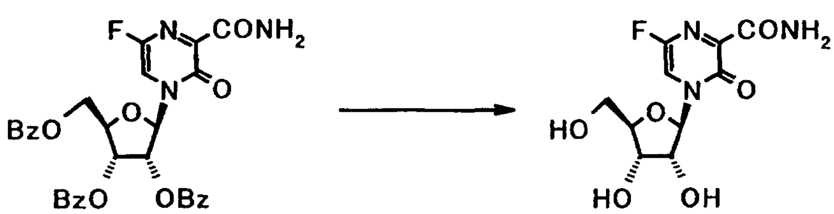

0.15 g of [(2R, 3R, 4R, 5R)-5-[3-(aminocarbonyl)-5-fluoro-2-oxo-1(2H)-pyrazinyl)-3,4-bis(benzoyloxy)tetrahydro-2-furanyl] methyl benzoate was dissolved in 2.0 mL of methanol, and thereafter, 0.14 g of a 28% sodium methoxide methanol solution was added thereto under ice cooling. The obtained mixture was stirred at the same temperature for 20 minutes and then at room temperature for 30 minutes. 0.75 mL of 1 mol/L hydrochloric acid was added to the reaction mixture, and the solvent was removed under reduced pressure. The obtained residue was purified by column chromatography [eluant; chloroform : methanol = 5 : 1], and thereafter, isopropanol and diethyl ether were added thereto. The mixture was then filtrated, to obtain 40 mg of 4-[(2R, 3R, 4S, 5R)-3,4-dihydroxy-5-(hydroxymethyl)tetrahydro-2-furanyl]-6-fluoro-3-oxo-3,4-dihydro-2-pyrazinecarboxamide.
IR(KBr)$cm^{-1}$: 1686

Example 33

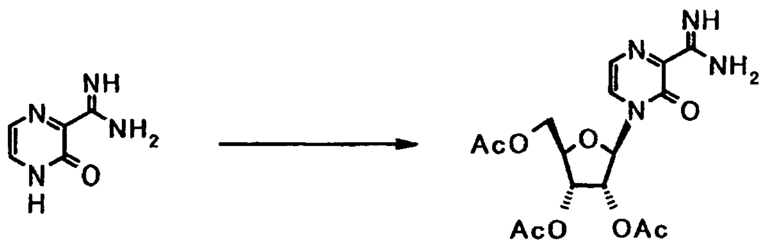

0.20 g of 3-oxo-3,4-dihydro-2-pyrazinecarboximidamide and 10 mg of ammonium sulfate were suspended in 2.0 mL of 1,1,1,3,3,3-hexamethyldisilazane, and the suspension was heated under reflux for 10 minutes under nitrogen current. 9.0 mg of ammonium sulfate was added thereto, and the mixture was further heated under reflux for 2 hours. The reaction mixture was cooled, and the solvent was then removed under reduced pressure. The obtained residue was dissolved in 4.0 mL of acetonitrile, and thereafter, 0.46 g of β-D-ribofuranose-1,2,3,5-tetraacetate and 0.34 mL of tin(IV) chloride were successively added thereto, followed by stirring at room temperature for 3 hours. 10 μL of trifluoroacetic acid and 1.0 mL of water were added to the reaction mixture, and the solvent was then removed under reduced pressure. Using 0.05 g of 3-oxo-3,4-dihydro-2-pyrazinecarboximidamide, the same above reaction was repeated to obtain a reaction mixture. The obtained reaction mixtures were combined and then purified by reverse phase silica gel column chromatography [eluant; acetonitrile : water = 1 : 4], to obtain 0.34 g of a light yellow solid, (2R, 3R, 4R, 5R)-4-(acetyloxy)-2-[(acetyloxy)methyl]-5-[3-[amino(imino)methyl]-2-oxo-1(2H)-pyrazinyl] tetrahydro-3-furanyl acetate.
IR(KBr)cm$^{-1}$: 3392, 1750, 1685
$^1$H-NMR($CDCl_3$)δ: 2.11(3H,s), 2.16(6H,s), 4.4-4.7(3H,m), 5.31(1H,t,J=5.4Hz), 5.5-5.6(1H,m), 6.22(1H,d,J=3.0Hz), 7.8-8.0(1H,m), 8.1-8.3(1H,m), 8.67(1H,brs), 10.45(2H,brs)

Example 34

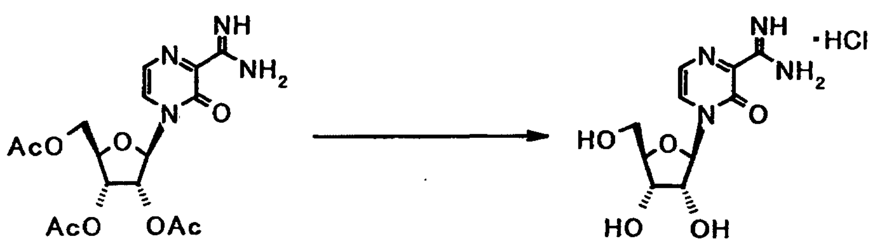

0.10 g of (2R, 3R, 4R, 5R)-4-(acetyloxy)-2-[(acetyloxy)methyl]-5-[3-[amino(imino)methyl]-2-oxo-1(2H)-pyrazinyl] tetrahydro-3-furanyl acetate was added to 5.0 mL of 25% ammonia water under ice cooling, and the mixture was stirred at the same temperature for 2 hours. 4.9 mL of acetic acid was added to the reaction mixture, and the solvent was removed under reduced pressure. Using 20 mg of (2R, 3R, 4R, 5R)-4-(acetyloxy)-2-[(acetyloxy)methyl]-5-[3-[amino(imino)methyl]-2-oxo-1(2H)-pyrazinyl] tetrahydro-3-furanyl acetate, the same above reaction was carried out. The obtained reaction mixtures were combined and then purified by reverse phase silica gel column chromatography [eluant; water]. 5.0 mL of 1 mol/L hydrochloric acid was added to the obtained solid, and the solvent was removed under reduced pressure. Moreover, 5.0 mL of 1 mol/L hydrochloric acid was further added thereto, and the solvent was removed under reduced pressure. Ethanol was added to the obtained residue, and the solid was collected by filtration, to obtain 30 mg of hydrochloride of a light yellow solid, 4-[(2R, 3R, 4S, 5R)-3,4-dihydroxy-5-(hydroxymethyl)tetrahydro-2-furanyl]-3-oxo-3,4-dihydro-2-pyrazinecarboximidamide.
IR(KBr)cm$^{-1}$: 3374, 3281, 1690
$^1$H-NMR(DMSO-$d_6$) δ: 3.7-3.9(2H,m), 3.9-4.2(3H,m), 5.1-5.3(1H,m), 5.3-5.6(1H,m), 5.6-5.8(1H,m), 5.90(1H,s), 7.86 (1H,d,J=4.0Hz), 8.76(1H,d,J=4.0Hz), 9.44(4H,brs)

Example 35

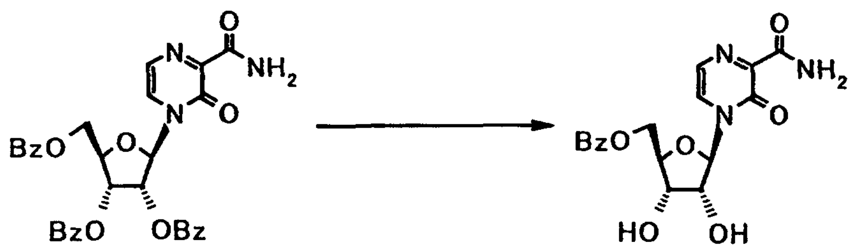

37 mg of {(2R, 3S, 4R, 5R)-5-[3-(aminocarbonyl)-2-oxo-1(2H)-pyrazinyl]-3,4-dihydroxytetrahydro-2-furanyl} methyl benzoate was obtained from 200 mg of {(2R, 3R, 4R, 5R)-5-[3-(aminocarbonyl)-2-oxo-1(2H)-pyrazinyl]-3,4-bis (benzoyloxy)tetrahydro-2-furanyl} methyl benzoate in the same manner as in Example 18.
$^1$H-NMR(DMSO-$d_6$)δ: 4.06(1H,dd,J= 6.6,12.7), 4.12-4.15(1H,m), 4.28-4.32(1H,m), 4.59(1H,dd,J=5.1,12.2), 4.67(1H, dd,J=2.7,12.5), 5.40(1H,d,J=6.3), 5.76(1H,d,J=4.9), 5.91(1H,d,J=2.0), 7.37(1H,d,J=4.4), 7.57(2H,t,J=7.8), 7.71(1H, dd,J=7.1,7.6), 7.75(1H,brs), 7.85(1H,d,J=4.2), 8.01(2H,dd,J=1.0,7.4), 8.30(1H,brs)

Example 36

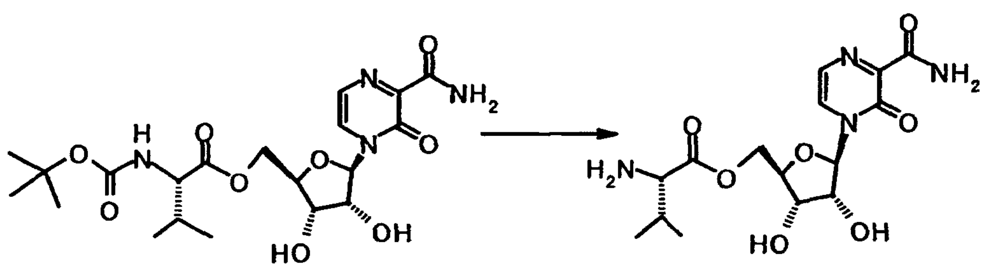

100 mg of {(2R, 3S, 4R, 5R)-5-[3-(aminocarbonyl)-2-oxo-1(2H)-pyrazinyl]-3,4-dihydroxytetrahydro-2-furanyl} methyl (2S)-2-[(tert-butoxycarbonyl)amino]-3-methyl butanoate was dissolved in a mixed solvent of 1 mL of trifluoroacetic acid and 0.1 mL of water, and the mixture was stirred at room temperature for 1 hour. Trifluoroacetic acid was removed under reduced pressure, and water was added. The mixture was adjusted to pH 8 with a saturated sodium bicarbonate aqueous solution, and it was then purified by reverse phase silica gel chromatography [eluant; 10% acetonitrile aqueous solution], followed by azeotropy with ethanol, to obtain 59 mg of a white solid, {(2R, 3S, 4R, 5R)-5-[3-(aminocarbonyl)-2-oxo-1(2H)-pyrazinyl]-3,4-dihydroxytetrahydro-2-furanyl} methyl (2S)-2-amino-3-methyl butanoate.
IR(KBr)$cm^{-1}$: 1724, 1670, 1653
$^1$H-NMR(DMSO-$d_6$, $D_2O$)δ: 0.84(3H,d,J=6.6Hz), 0.89(3H,d,J=6.6Hz), 1.82-1.90(1H,m), 3.22(1H,d,J=5.2Hz), 3.88-3.94(1H,m), 4.09(1H,s), 4.17-4.20(1H,m), 4.36-4.38(2H,m), 5.91(1H,s), 7.58(1H,d,J=4.0Hz), 7.90(1H,d,J=4.0)

Example 37

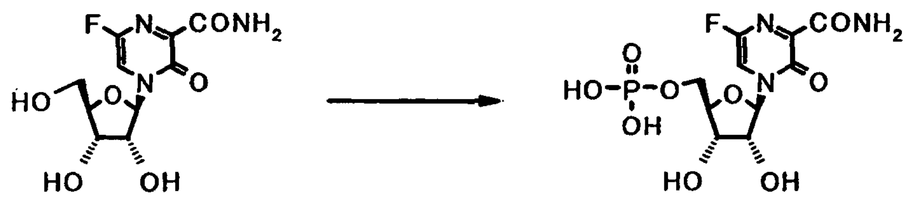

58 mg of 4-[(2R, 3R, 4S, 5R)]-3,4-dihydroxy-5-(hydroxymethyl)tetrahydro-2-furanyl]-6-fluoro-3-oxo-3,4-dihydro-2-pyrazinecarboxamide was suspended in 1 mL of trimethyl phosphate, and thereafter, 37 μL of pyridine and 49 μL of phosphorus oxychloride were successively added thereto under ice cooling, followed by stirring at the same temperature for 1 hour. 3 mL of a 1 mol/L triethyl ammonium bicarbonate aqueous solution was added thereto, and the mixture was stirred for 15 minutes. The solvent was removed under reduced pressure, and the residue was purified by ion exchange column chromatography [eluant; 0.1 mol/L triethyl ammonium bicarbonate aqueous solution]. Subsequently, it was purified by reverse phase silica gel column chromatography [eluant; water], to obtain 39 mg of triethyl ammonium salts of a solid, [(2R, 3S, 4R, 5R)-5-[3-(aminocarbonyl)-5-fluoro-2-oxo-1(2H)-pyrazinyl]-3,4-dihydroxytetrahydro-2-furanyl] methyl phosphate.

3 mL of an acetone solution containing 0.14 g of sodium perchlorate was added to 0.5 mL of a methanol suspension containing 36 mg of the above obtained triethyl ammonium salts of monophosphoric acid at room temperature, and the mixture was stirred for 30 minutes. The deposit was collected by filtration and washed with acetone, to obtain 27 mg of sodium salts of a light yellow solid, [(2R, 3S, 4R, 5R)-5-[3-(aminocarbonyl)-5-fluoro-2-oxo-1(2H)-pyrazinyl)-3,4-dihydroxytetrahydro-2-furanyl] methyl phosphate.
$^{1}$H-NMR($D_2O$)δ: 4.13-4.18(1H,m), 4.29-4.40(4H,m), 6.10(1H,s), 8.46(1H,d,J=4.9Hz)

INDUSTRIAL APPLICABILITY

From the above test examples, it was found that a pyrazine nucleotide triphosphate analog has an activity to specifically inhibit virus polymerase in the virus polymerase inhibition test, that the pyrazine carboxamide nucleotide modified with a substituent given in the present invention moves into a cell and has an antiviral activity therein, although it has been generally known that nucleotide cannot move into a cell through a cell membrane, and that pyrazine nucleoside is converted into a pyrazine nucleotide triphosphate analog in the body of an animal administered with the pyrazine nucleoside. Accordingly, the virus growth inhibition and/or virucidal method of the present invention, which is characterized by the use of a pyrazine nucleotide or pyrazine nucleoside analog generated by the effect of kinase such as nucleotide kinase or a salt thereof, is useful as a method for treating virus infection. Moreover, the novel pyrazine nucleotide or pyrazine nucleoside analog or a salt thereof of the present invention is useful as an agent for preventing and/or treating virus infection.

## Claims

1. A virus growth inhibition and/or virucidal method, **characterized by** using a pyrazine nucleotide analog represented by the following general formula [1] or a salt thereof:

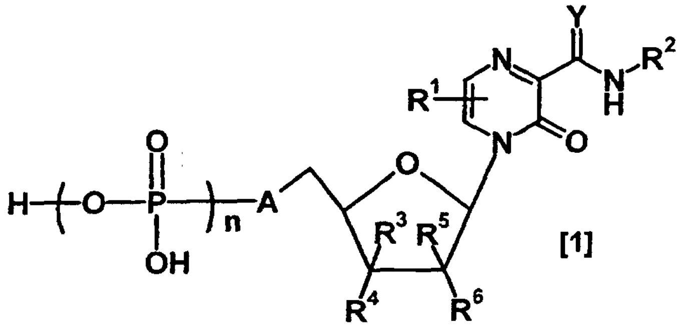

[1]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^4$, $R^5$ and $R^6$, which may be the same or different, represents a hydrogen atom, or a hydroxyl group that may be substituted or protected; A represents an oxygen atom or a methylene group; Y represents an oxygen atom or an imino group; and n represents an integer of 0 to 3.

2. The method according to claim 1, **characterized by** using the pyrazine nucleotide analog represented by general formula [1] or a salt thereof wherein Y is an oxygen atom.

3. The method according to claim 1 or 2, **characterized by** using the pyrazine nucleotide analog represented by general formula [1] or a salt thereof wherein n is an integer of 1 to 3.

4. The method according to any one of claims 1 to 3, **characterized by** using the pyrazine nucleotide analog represented by general formula [1] or a salt thereof wherein the substituent of a pyrazine ring is one or more groups selected from the group consisting of a halogen atom; an alkyl group that may be substituted with a hydroxyl, alkoxy, alkylthio, aryl, amino or alkylamino group; an alkyl or alkenyl group that may be substituted with a halogen atom; a cycloalkyl group; a hydroxyl group; an alkoxy group; a cycloalkyloxy group; alkoxycarbonyl group; a mercapto group; an alkylthio group that may be substituted with an aryl group; an aryl group; an aryloxy group; an arylthio group; an arylamino group; a cyano group; a nitro group; an amino group that may be substituted with an acyl group; alkylamino group; a cycloalkylamino group; an acyl group; a carboxyl group; a carbamoyl group; a thiocarbamoyl group; an alkylcarbamoyl group; and a heterocyclic group.

5. The method according to any one of claims 1 to 4, **characterized by** using the pyrazine nucleotide analog represented by general formula [1] or a salt thereof wherein n is 3.

6. The method according to any one of claims 1 to 5, **characterized by** using the pyrazine nucleotide analog represented by general formula [1] or a salt thereof generated by a kinase.

7. The method according to any one of claims 1 to 5, **characterized by** using the pyrazine nucleotide analog represented by general formula [1] or a salt thereof generated by a nucleotide kinase.

8. The method according to any one of claims 1 to 7, wherein the pyrazine nucleotide analog represented by general formula [1] or a salt thereof is derived from a pyrazine nucleotide analog represented by the following general formula [2] or a salt thereof:

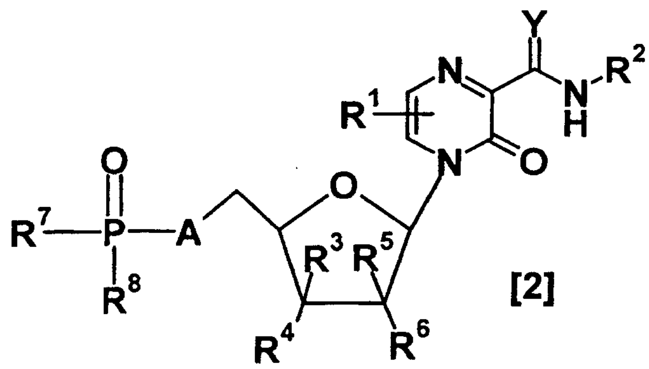

[2]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^4$, $R^5$ and $R^6$, which may be the same or different, represents a hydrogen atom, or a hydroxyl group that may be substituted or protected; each of $R^7$ and $R^8$ in phosphoric acid or phosphonic acid independently represents a protected or unprotected, substituted or unsubstituted hydroxyl group to be decomposed under physiological conditions; A represents an oxygen atom or a methylene group; and Y represents an oxygen atom or an imino group.

**9.** The method according to any one of claims 1 to 8, wherein the pyrazine nucleotide analog represented by general formula [1] or a salt thereof is derived from a pyrazine nucleotide analog represented by the following general formula [3] or a salt thereof:

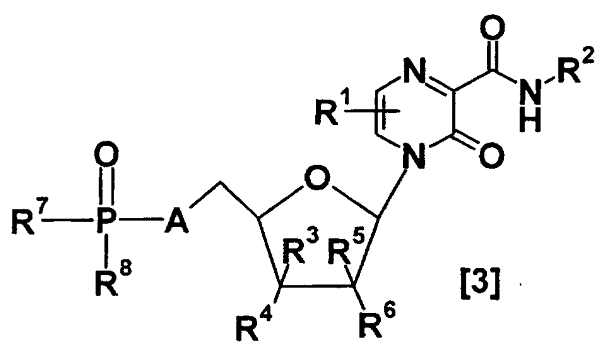

[3]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^4$, $R^5$ and $R^6$, which may be the same or different, represents a hydrogen atom, or a hydroxyl group that may be substituted or protected; each of $R^7$ and $R^8$ in phosphoric acid or phosphonic acid independently represents a protected or unprotected, substituted or unsubstituted hydroxyl group to be decomposed under physiological conditions; and A represents an oxygen atom or a methylene group.

**10.** A virus growth inhibition and/or virucidal method, **characterized by** using a pyrazine nucleoside analog represented by the following general formula [3z] or a salt thereof:

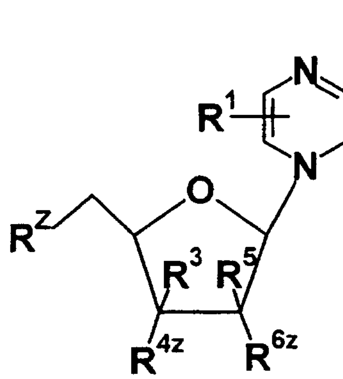

[3z]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^{4Z}$, $R^5$ and $R^{6Z}$, which may be the same or different, represents a hydrogen atom or a hydroxyl group that may be substituted or protected, or $R^{4Z}$ and $R^{6Z}$ together represent a group represented as -O-alkylene-O- that may be substituted; $R^Z$ represents a protected or unprotected, substituted or unsubstituted hydroxyl group to be decomposed under physiological conditions; and Y represents an oxygen atom or an imino group.

**11.** The method according to claim 10, wherein, in the pyrazine nucleotide analog represented by general formula [3z] or a salt thereof, Y is an imino group.

**12.** The method according to any one of claims 1 to 11, **characterized by** using a polymerase inhibitory effect.

**13.** The method according to any one of claims 1 to 12, wherein the virus is influenza virus, RS virus, AIDS virus, papilloma virus, adenovirus, hepatitis A virus, hepatitis B virus, hepatitis C virus, hepatitis E virus, poliovirus, echovirus, Coxsackie virus, enterovirus, rhinovirus, rotavirus, Newcastle disease virus, mumps virus, vesicular stomatitis virus, rabies virus, Lassa fever virus, measles virus, Filovirus, Japanese encephalitis virus, yellow fever virus, dengue fever virus or West Nile virus.

**14.** The method according to any one of claims 1 to 12, wherein the virus is influenza virus or hepatitis C virus.

**15.** A pyrazine nucleotide analog represented by the following general formula [1z] or a salt thereof:

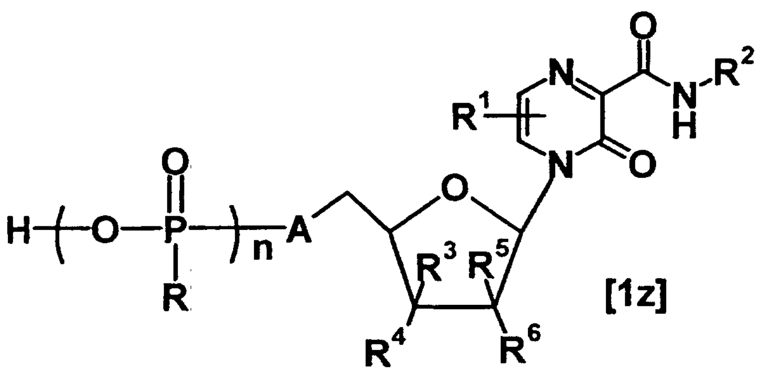

[1z]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^4$, $R^5$ and $R^6$, which may be the same or different, represents a hydrogen atom, or a hydroxyl group that may be substituted or protected; R represents a hydroxyl group that may be protected or substituted with a group decomposed under physiological conditions; A represents an oxygen atom or a methylene group; and n represents an integer of 1 to 3.

**16.** A pyrazine nucleotide analog represented by the following general formula [1z] or a salt thereof:

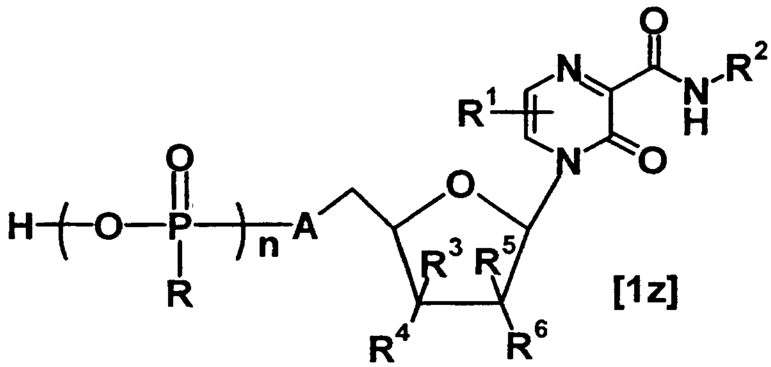

[1z]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^4$, $R^5$ and $R^6$, which may be the same or different, represents a hydrogen atom, or a hydroxyl group that may be substituted or protected; R represents a hydroxyl group that may be protected or substituted with a group decomposed under physiological conditions; A represents an oxygen atom or a methylene group; and n represents an integer of 1 to 3; provided that a case where R is a hydroxyl group, A is an oxygen atom, and $R^1$ is a hydrogen atom or halogen atom is excluded.

**17.** A pyrazine nucleotide analog represented by the following general formula [2] or a salt thereof:

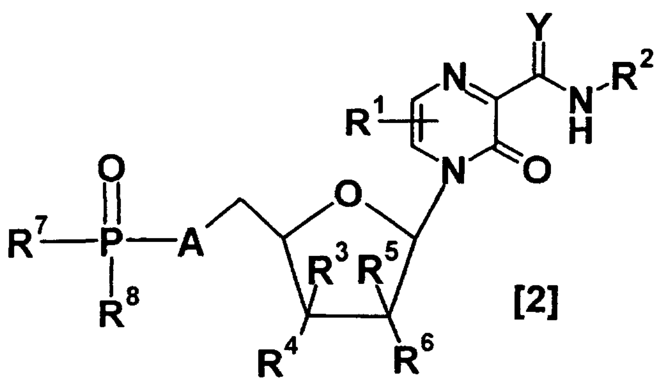

[2]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^4$, $R^5$ and $R^6$, which may be the same or different, represents a hydrogen atom, or a hydroxyl group that may be substituted or protected; each of $R^7$ and $R^8$ in phosphoric acid or phosphonic acid independently represents a protected or unprotected, substituted or unsubstituted hydroxyl group to be decomposed under physiological conditions; A represents an oxygen atom or a methylene group; and Y represents an oxygen atom or an imino group.

**18.** The pyrazine nucleotide analog or a salt thereof according to claim 17, wherein Y is an oxygen atom.

**19.** A pyrazine nucleoside analog represented by the following general formula [3z] or a salt thereof:

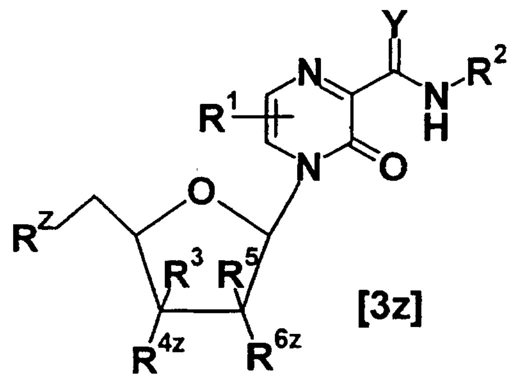

[3z]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^{4Z}$, $R^5$ and $R^{6Z}$, which may be the same or different, represents a hydrogen atom or a hydroxyl group that may be substituted or protected, or $R^{4Z}$ and $R^{6Z}$ together represent a group represented as -O-alkylene-O- that may be substituted; $R^Z$ represents a protected or unprotected, substituted or unsubstituted hydroxyl group to be decomposed under physiological conditions; and Y represents an oxygen atom or an imino group.

**20.** The pyrazine nucleoside analog or a salt thereof according to claim 19, wherein, in the pyrazine nucleotide analog represented by general formula [3z] or a salt thereof, Y is an imino group.

**21.** The pyrazine nucleoside analog represented by general formula [3z] or a salt thereof according to claim 19, represented by general formula [3z']:

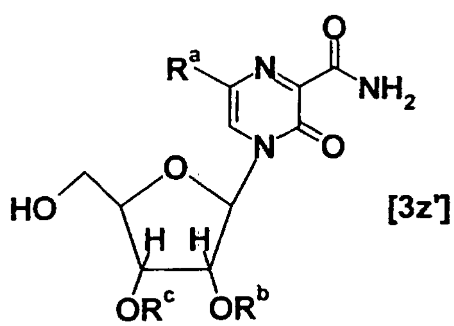

[3z']

wherein $R^a$ represents a hydrogen or halogen atom; and each of $R^b$ and $R^c$, which may be the same or different, represents a hydrogen atom or a hydroxyl protecting group, or $R^b$ and $R^c$ together represent an alkylene group that may be substituted.

**22.** An RNA polymerase inhibitor precursor having a pyrazine nucleotide analog structure represented by general formula [1x]:

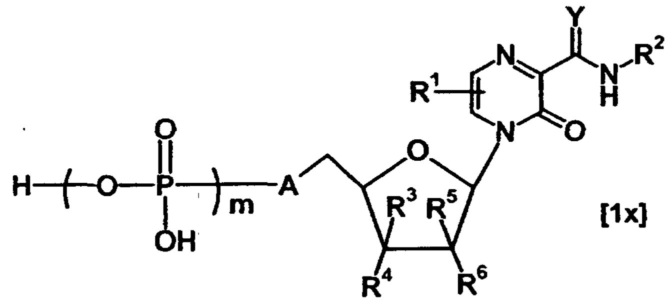

[1x]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^4$, $R^5$ and $R^6$, which may be the same or different, represents a hydrogen atom, or a hydroxyl group that may be substituted or protected; A represents an oxygen atom or a methylene group; Y represents an oxygen atom or an imino group; and m represents an integer of 0 to 2,
said RNA polymerase inhibitor precursor being converted in vivo into a pyrazine triphosphate nucleotide analog represented by general formula [1y]:

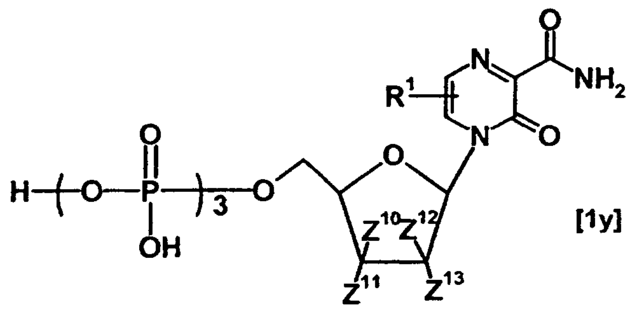

[1y]

wherein $R^1$ represents a hydrogen atom, or a substituent of a pyrazine ring; each of $Z^{10}$, $Z^{11}$, $Z^{12}$ and $Z^{13}$, which may be the same or different, represents a hydrogen atom or a hydroxyl group, and thereby exhibiting an RNA polymerase inhibitory effect.

**23.** The RNA polymerase inhibitor precursor according to claim 22 wherein, in the pyrazine nucleotide analog structure represented by general formula [1x], Y is an oxygen atom.

**24.** The RNA polymerase inhibitor precursor according to claim 22 or 23 wherein, in the pyrazine nucleotide analog structure represented by general formula [1x], the substituent of a pyrazine ring is one or more groups selected from the group consisting of: a halogen atom; an alkyl group that may be substituted with a hydroxyl, alkoxy, alkylthio, aryl, amino or alkylamino group; an alkyl or alkenyl group that may be substituted with a halogen atom; a cycloalkyl group; a hydroxyl group; an alkoxy group; a cycloalkyloxy group; an alkoxycarbonyl group; a mercapto group; an alkylthio group that may be substituted with an aryl group; an aryl group; an aryloxy group; an arylthio group; an arylamino group; a cyano group; a nitro group; an amino group that may be substituted with an acyl group; an alkylamino group; a cycloalkylamino group; an acyl group; a carboxyl group; a carbamoyl group; a thiocarbamoyl group; an alkylcarbamoyl group; and a heterocyclic group.

**25.** The RNA polymerase inhibitor precursor according to any one of claims 22 to 24, wherein each of $R^1$, $R^3$ and $R^5$ represents a hydrogen atom; and each of $R^4$ and $R^6$ represents a hydroxyl group, in the pyrazine nucleotide analog structure represented by general formula [1x] that is converted in vivo into the pyrazine triphosphate nucleotide analog represented by general formula [1y] wherein each of $R^1$, $Z^{10}$ and $Z^{12}$ represents a hydrogen atom; and each of $Z^{11}$ and $Z^{13}$ represents a hydroxyl group.

**26.** The RNA polymerase inhibitor precursor according to any one of claims 22 to 25, wherein the pyrazine triphosphate

nucleotide analog represented by general formula [1y] is generated by a kinase.

**27.** The RNA polymerase inhibitor precursor according to any one of claims 22 to 26, wherein the pyrazine triphosphate nucleotide analog represented by general formula [1y] is generated by a nucleotide kinase.

**28.** The RNA polymerase inhibitor precursor according to any one of claims 22 to 27, **characterized in that** it inhibits virus-derived RNA polymerase with selectivity 200 times or more higher than that for host-derived RNA polymerase.

**29.** The RNA polymerase inhibitor precursor according to any one of claims 22 to 27, which is a pyrazine nucleoside or pyrazine mononucleotide analog structure represented by general formula [1w]:

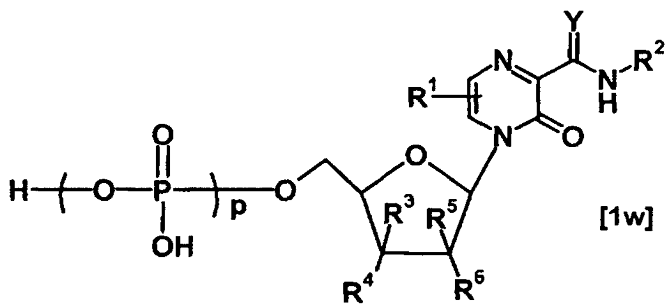

[1w]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^4$, $R^5$ and $R^6$, which may be the same or different, represents a hydrogen atom, or a hydroxyl group that may be substituted or protected; Y represents an oxygen atom or an imino group; and p represents 0 or 1,
**characterized in that** the ratio of an inhibitory effect of the RNA polymerase inhibitor precursor on the virus-derived RNA polymerase to an inhibitory effect of the RNA polymerase inhibitor precursor on host cell-derived inosine monophosphate dehydrogenase is 900 : 1 or more.

**30.** The RNA polymerase inhibitor precursor according to claim 29 wherein, in the pyrazine nucleoside or pyrazine mononucleotide analog represented by general formula [1w], Y is an oxygen atom.

**31.** The RNA polymerase inhibitor precursor according to claim 29 or 30 wherein, in the pyrazine nucleoside or pyrazine mononucleotide analog structure represented by general formula [1w], the substituent of a pyrazine ring is one or more groups selected from the group consisting of: a halogen atom; an alkyl group that may be substituted with a hydroxyl, alkoxy, alkylthio, aryl, amino or alkylamino group; an alkyl or alkenyl group that may be substituted with a halogen atom; a cycloalkyl group; a hydroxyl group; an alkoxy group; a cycloalkyloxy group; an alkoxycarbonyl group; a mercapto group; an alkylthio group that may be substituted with an aryl group; an aryl group; an aryloxy group; an arylthio group; an arylamino group; a cyano group; a nitro group; an amino group that may be substituted with an acyl group; an alkylamino group; a cycloalkylamino group; an acyl group; a carboxyl group; a carbamoyl group; a thiocarbamoyl group; an alkylcarbamoyl group; and a heterocyclic group.

**32.** The RNA polymerase inhibitor precursor according to any one of claims 29 to 31, which is the pyrazine nucleoside or pyrazine mononucleotide analog structure represented by general formula [1w], wherein each of $R^1$, $R^3$ and $R^5$ represents a hydrogen atom; and each of $R^4$ and $R^6$ represents a hydroxyl group.

**33.** The RNA polymerase inhibitor precursor according to any one of claims 29 to 32, wherein the virus-derived RNA polymerase is derived from influenza virus, RS virus, hepatitis A virus, hepatitis C virus, hepatitis E virus, poliovirus, echovirus, Coxsackie virus, enterovirus, rhinovirus, rotavirus, Newcastle disease virus, mumps virus, vesicular stomatitis virus, rabies virus, Lassa fever virus, measles virus, Filovirus, Japanese encephalitis virus, yellow fever virus, dengue fever virus or West Nile virus.

**34.** The RNA polymerase inhibitor precursor according to claim 33, wherein the virus-derived RNA polymerase is derived from influenza virus or hepatitis C virus.

**35.** The RNA polymerase inhibitor precursor according to any one of claims 22 to 34 wherein, in the pyrazine nucleoside

or pyrazine mononucleotide analog structure represented by general formula [1w], m is 0.

**36.** An RNA polymerase inhibitor, which exhibits an RNA polymerase inhibitory effect and has a pyrazine triphosphate nucleotide analog structure represented by general formula [1y]:

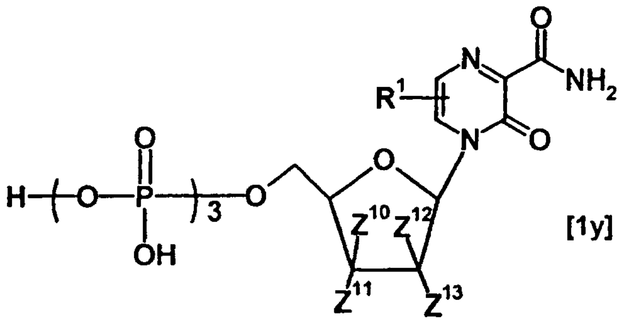

[1y]

wherein $R^1$ represents a hydrogen atom, or a substituent of a pyrazine ring; and each of $Z^{10}$, $Z^{11}$, $Z^{12}$ and $Z^{13}$, which may be the same or different, represents a hydrogen atom or hydroxyl group.

**37.** The RNA polymerase inhibitor having a pyrazine triphosphate nucleotide analog structure according to claim 36, **characterized in that** its inhibitory effect on virus-derived RNA polymerase is 200 times or more selective than the inhibitory effect on host-derived RNA polymerase.

**38.** The RNA polymerase inhibitor having a pyrazine triphosphate nucleotide analog structure according to claim 37, wherein the virus-derived RNA polymerase is derived from influenza virus, RS virus, hepatitis A virus, hepatitis C virus, hepatitis E virus, poliovirus, echovirus, Coxsackie virus, enterovirus, rhinovirus, rotavirus, Newcastle disease virus, mumps virus, vesicular stomatitis virus, rabies virus, Lassa fever virus, measles virus, Filovirus, Japanese encephalitis virus, yellow fever virus, dengue fever virus or West Nile virus.

**39.** The RNA polymerase inhibitor having a pyrazine triphosphate nucleotide analog structure according to claim 38, wherein the virus-derived RNA polymerase is influenza virus-derived RNA polymerase or hepatitis C virus-derived RNA polymerase.

**40.** A method for treating patients infected with virus, comprising the step of administering to a patient infected with virus a pyrazine nucleoside analog represented by the following general formula [3z] or a salt thereof:

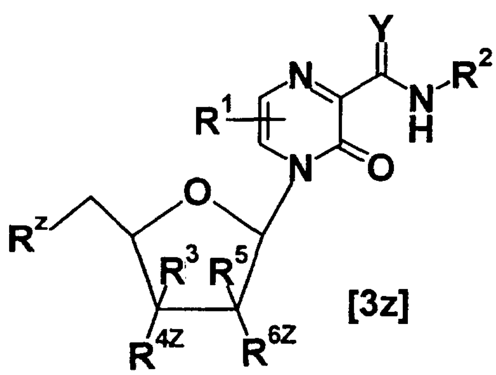

[3z]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^{4Z}$, $R^5$ and $R^{6Z}$, which may be the same or different, represents a hydrogen atom or a hydroxyl group that may be substituted or protected, or $R^{4Z}$ and $R^{6Z}$ together represent a group represented as -O-alkylene-O- that may be substituted; $R^Z$ represents a protected or unprotected, substituted or unsubstituted hydroxyl group to be decomposed under physiological

conditions; and Y represents an oxygen atom or an imino group.

**41.** The method according to claim 40, further comprising the step of converting the general formula [3z] into a pyrazine triphosphate nucleotide analog represented by general formula [1y]:

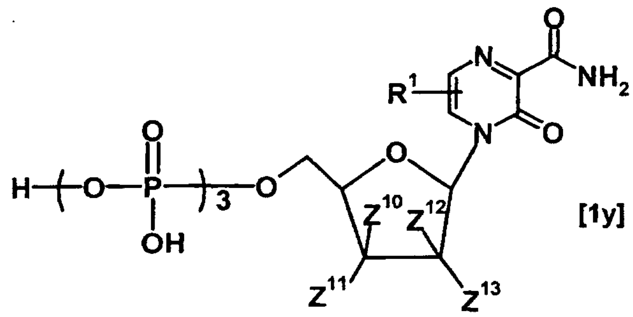

[1y]

wherein $R^1$ represents a hydrogen atom, or a substituent of a pyrazine ring; and each of $Z^{10}$, $Z^{11}$, $Z^{12}$ and $Z^{13}$, which may be the same or different, represents a hydrogen atom or hydroxyl group, in the body of a patient infected with virus.

**42.** The method according to claim 41,
**characterized in that**, in the body of a patient infected with virus, the general formula [3z] is converted into the pyrazine triphosphate nucleotide analog represented by general formula [1y] through a pyrazine nucleotide analog represented by general formula [1v]:

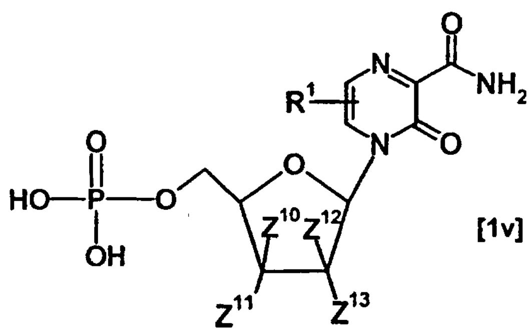

[1v]

wherein $R^1$ represents a hydrogen atom, or a substituent of a pyrazine ring; and each of $Z^{10}$, $Z^{11}$, $Z^{12}$ and $Z^{13}$, which may be the same or different, represents a hydrogen atom or hydroxyl group.

**43.** The method according of claim 42, **characterized in that** the pyrazine nucleotide analog represented by general formula [1v] does not substantially inhibit inosine monophosphate dehydrogenase derived from a host cell.

**44.** The method according to any one of claims 41 to 43, **characterized in that** the pyrazine triphosphate nucleotide analog represented by general formula [1y] inhibits virus-derived RNA polymerase more selectively than host-derived RNA polymerase.

**45.** The method according to claim 44, wherein the virus-derived RNA polymerase is derived from influenza virus, RS virus, hepatitis A virus, hepatitis C virus, hepatitis E virus, poliovirus, echovirus, Coxsackie virus, enterovirus, rhinovirus, rotavirus, Newcastle disease virus, mumps virus, vesicular stomatitis virus, rabies virus, Lassa fever virus, measles virus, Filovirus, Japanese encephalitis virus, yellow fever virus, dengue fever virus or West Nile virus.

**46.** The method for treating a patient infected with virus according to claim 40, wherein $R^1$ represents a hydrogen atom, $R^2$ represents a hydrogen atom, each of $R^3$ and $R^5$ represents a hydrogen atom, each of $R^{4Z}$, $R^{6Z}$ and $R^Z$

represents a hydroxyl group, and Y represents an oxygen atom.

**47.** Use of a pyrazine nucleotide analog represented by general formula [1] or a salt thereof for the manufacture of an antiviral agent:

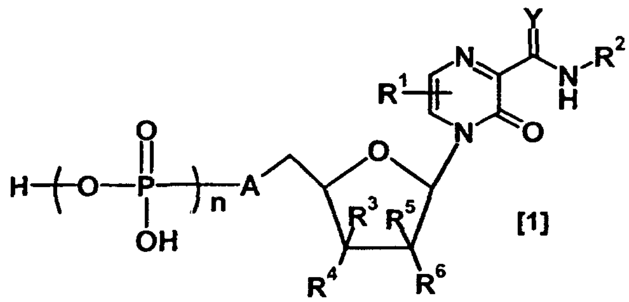

[1]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^4$, $R^5$ and $R^6$, which may be the same or different, represents a hydrogen atom, or a hydroxyl group that may be substituted or protected; A represents an oxygen atom or a methylene group; Y represents an oxygen atom or an imino group; and n represents an integer of 0 to 3.

**48.** The use according to claim 47, wherein $R^1$ represents a hydrogen atom, $R^2$ represents a hydrogen atom, each of $R^3$ and $R^5$ represents a hydrogen atom, each of $R^4$ and $R^6$ represents a hydroxyl group, A represents an oxygen atom, Y represents an oxygen atom, and n is 0.

**49.** Use of a pyrazine nucleoside analog represented by general formula [3z] or a salt thereof for the manufacture of an antiviral agent:

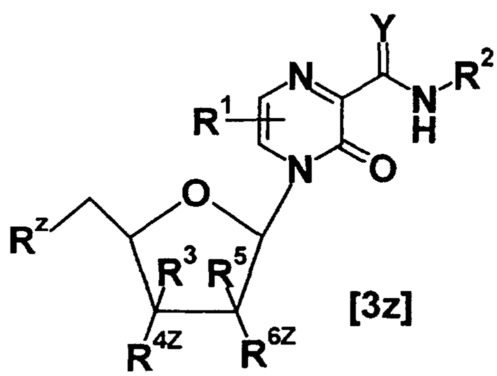

[3z]

wherein $R^1$ represents a hydrogen atom, or a substituent of a pyrazine ring; $R^2$ represents a hydrogen atom, an acyl group, or a carbamoylalkyl or carboxyalkyl group that may be substituted; each of $R^3$, $R^{4Z}$, $R^5$ and $R^{6Z}$, which may be the same or different, represents a hydrogen atom or a hydroxyl group that may be substituted or protected, or $R^{4Z}$ and $R^{6Z}$ together represent a group represented as -O-alkylene-O- that may be substituted; $R^Z$ represents a protected or unprotected, substituted or unsubstituted hydroxyl group to be decomposed under physiological conditions; and Y represents an oxygen atom or an imino group.

**50.** The use according to any one of claims 47 to 49, wherein the general formula [3z] is converted into a pyrazine triphosphate nucleotide analog represented by general formula [1y]:

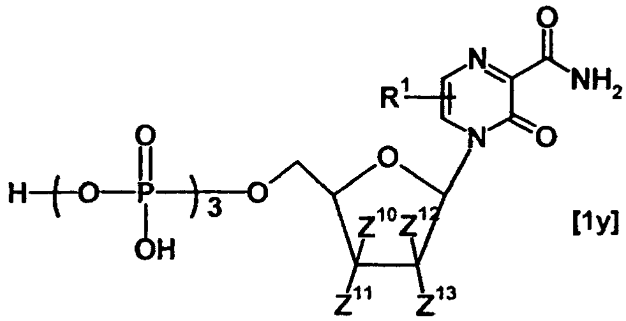

[1y]

wherein $R^1$ represents a hydrogen atom, or a substituent of a pyrazine ring; and each of $Z^{10}$, $Z^{11}$, $Z^{12}$ and $Z^{13}$, which may be the same or different, represents a hydrogen atom or hydroxyl group, in the body of a patient infected with virus.

**51.** The use according to claim 49, **characterized in that**, in the body of a patient infected with virus, the general formula [3z] is converted into the pyrazine triphosphate nucleotide analog represented by general formula [1y] through a pyrazine nucleotide analog represented by general formula [1v]:

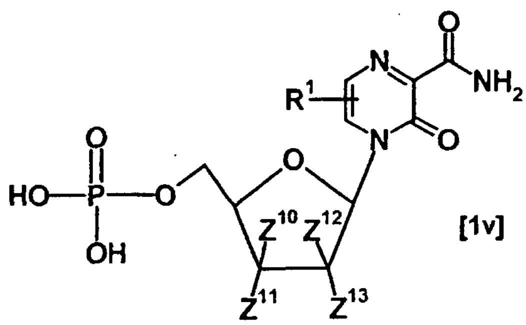

[1v]

wherein $R^1$ represents a hydrogen atom, or a substituent of a pyrazine ring; and each of $Z^{10}$, $Z^{11}$, $Z^{12}$ and $Z^{13}$, which may be the same or different, represents a hydrogen atom or hydroxyl group.

**52.** The use according to claim 51, **characterized in that** the pyrazine nucleotide analog represented by general formula [1v] does not substantially inhibit inosine monophosphate dehydrogenase derived from a host cell.

**53.** The use according to any one of claims 50 to 52, **characterized in that** the pyrazine triphosphate nucleotide analog represented by general formula [1y] inhibits virus-derived RNA polymerase than more selectively host-derived RNA polymerase.

**54.** The method according to any one of claims 50 to 53, wherein the virus-derived RNA polymerase is derived from influenza virus, RS virus, hepatitis A virus, hepatitis C virus, hepatitis E virus, poliovirus, echovirus, Coxsackie virus, enterovirus, rhinovirus, rotavirus, Newcastle disease virus, mumps virus, vesicular stomatitis virus, rabies virus, Lassa fever virus, measles virus, Filovirus, Japanese encephalitis virus, yellow fever virus, dengue fever virus or West Nile virus.

## INTERNATIONAL SEARCH REPORT

International application No.
PCT/JP02/08250

**A. CLASSIFICATION OF SUBJECT MATTER**
Int.Cl$^7$ A61K31/706, 45/00, A61P31/12, C07H19/04, C12N9/99

According to International Patent Classification (IPC) or to both national classification and IPC

**B. FIELDS SEARCHED**

Minimum documentation searched (classification system followed by classification symbols)
Int.Cl$^7$ A61K31/706-31/7072, C07H19/04, 19/06-19/11

Documentation searched other than minimum documentation to the extent that such documents are included in the fields searched

Electronic data base consulted during the international search (name of data base and, where practicable, search terms used)
REGISTRY(STN), CAplus(STN), MEDLINE(STN), WPI(DIALOG), BIOSIS(DIALOG), JICST FILE(JOIS)

**C. DOCUMENTS CONSIDERED TO BE RELEVANT**

| Category* | Citation of document, with indication, where appropriate, of the relevant passages | Relevant to claim No. |
|---|---|---|
| P,X | WO 01/60834 A1 (Toyama Chemical Co., Ltd.), 23 August, 2001 (23.08.01), & AU 200132287 A | 15-27,35 |
| A | DAVIS, Jean et al., Synthesis and antiviral evaluation of pyrazinones substituted with acyclic chains., Nucleosides & Nucleotides, May, 1998, Vol.17, No.5, pages 875 to 893 | 15-39 |
| A | BENJAHAD, Abdellah et al., Synthesis of 3-alkyl piperazin-2-one nucleosides with potential antiretroviral activity, Nucleosides & Nucleotides, 1996, Vol.15, Nos. 11 & 12, pages 1849 to 1861 | 15-39 |
| A | FR 2688003 A1 (Universite de Limoges), 03 September, 1993 (03.09.93), (Family: none) | 15-39 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 November, 2002 (21.11.02) | 10 December, 2002 (10.12.02) |
| Name and mailing address of the ISA/ Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP02/08250

C (Continuation). DOCUMENTS CONSIDERED TO BE RELEVANT

| Category* | Citation of document, with indication, where appropriate, of the relevant passages | Relevant to claim No. |
|---|---|---|
| A | KOBAYASHI, Nobuo et al., Eimeria tenella, E. necatrix, E. acervulina, E. maxima, and E. brunetti: Potent Anticoccidial Activity of an Uridine Analog, 1-(β-D-Ribofuranosyl)-2(1H)-pyrazinone 4-Oxide, Experimental Parasitology, February, 1986, Vol.61, No.1, pages 42 to 47 | 15-39 |

Form PCT/ISA/210 (continuation of second sheet) (July 1998)

INTERNATIONAL SEARCH REPORT

International application No.
PCT/JP02/08250

**Box I Observations where certain claims were found unsearchable (Continuation of item 1 of first sheet)**

This international search report has not been established in respect of certain claims under Article 17(2)(a) for the following reasons:

1. [x] Claims Nos.: 1 to 14 and 40 to 54
because they relate to subject matter not required to be searched by this Authority, namely:
(1) The subject matter of claims 1 to 14 is a method for the inhibition of virus proliferation and/or killing of a virus, and the embodiments thereof include an in vivo method practiced in the human body. Consequently, this (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.

[ ] No protest accompanied the payment of additional search fees.

Form PCT/ISA/210 (continuation of first sheet (1)) (July 1998)

INTERNATIONAL SEARCH REPORT

International application No.
PCT/JP02/08250

Continuation of Box No.I-1 of continuation of first sheet(1)

subject matter falls under the category of methods for treatment of the human body by therapy.

(2) The subject matter of claims 40 to 46 is a therapeutic method practiced on virus-infected patients and hence falls under the category of methods for treatment of the human body by therapy.

(3) Claims 50 and 51 disclose a use of a pyradine nucleotide (nucleoside) analogue or a salt thereof for antiviral agent production, which comprises converting the compound represented by the general formula [3z] into a pyradine triphosphate nucleotide analogue or the like in the body of a virus-infected patient. This use is no more than a therapeutic method practiced on virus-infected patients because this use produces an antiviral agent in the body of a virus-infected patient by converting a chemical substance into another chemical substance. Consequently, it can be said that not only the subject matter of claims 50 and 51 and that of the claims depending thereon but the subject matter of claims 47 to 49, which involve the subject matter of claims 50 and 51 as an embodiment, are therapeutic methods practiced on virus-infected patients. Therefore, the subject matters of claims 47 to 54 fall under the category of methods for treatment of the human body by therapy.

Form PCT/ISA/210 (extra sheet) (July 1998)